# EUROPÄISCHE PATENTSCHRIFT

(11) **EP 3 130 015 B1**
(45) Veröffentlichungstag und Bekanntmachung des Hinweises auf die Patenterteilung: **16.09.2026**
(21) Anmeldenummer: 15709617.3
(22) Anmeldetag: 12.03.2015
(51) Int. Cl.: C07D 209/86, C07D 401/14, C07D 403/04, C07D 403/10, C07D 403/14, C07D 405/04, C07D 405/10, C07D 491/048, C07D 495/04, C07D 498/06, C07D 209/82, C07D 403/02, C09B 19/00, C09B 21/00, C09B 57/00, C09B 57/10, C09K 11/02, C09K 11/06, H10K 85/60

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX DESTINÉS À DES DISPOSITIFS ÉLECTROLUMINESCENTS ORGANIQUES

(30) Priorität: 11.04.2014 EP 14001336
(43) Veröffentlichungstag der Anmeldung: 15.02.2017
(73) Patentinhaber: Merck Patent GmbH, 64293 Darmstadt (DE)
(72) Erfinder: PARHAM, Amir Hossain, 60486 Frankfurt am Main (DE); PFLUMM, Christof, 64291 Darmstadt (DE); EBERLE, Thomas, 76829 Landau (DE); JATSCH, Anja, 60489 Frankfurt am Main (DE); KROEBER, Jonas Valentin, 60311 Frankfurt am Main (DE); GROSSMANN, Tobias, 64297 Darmstadt (DE)
(74) Vertreter: Merck Patent Association
(86) Internationale Anmeldenummer: PCT/EP2015/000546
(87) Internationale Veröffentlichungsnummer: WO 2015/154843

(56) Entgegenhaltungen:
- EP-A1- 2 479 234
- EP-A2- 2 818 468
- EP-A2- 2 849 240
- WO-A1-2009/069442
- WO-A1-2014/021572
- WO-A2-2011/049325
- JP-A- 2005 093 159

## Beschreibung

Die vorliegende Erfindung beschreibt Carbazol-Derivate, insbesondere zur Verwendung als Triplettmatrixmaterialien in organischen Elektrolumineszenzvorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer.

Die Eigenschaften von phosphoreszierenden OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik werden unter anderem Indolocarbazolderivate (z. B. gemäß WO 2007/063754 A1 oder WO 2008/056746 A1) oder Indenocarbazolderivate (z. B. gemäß WO 2010/136109 A1 oder WO 2011/000455 A1), insbesondere solche, die mit elektronenarmen Heteroaromaten wie Triazin substituiert sind, als Matrixmaterialien für phosphoreszierende Emitter verwendet. Weiterhin werden beispielsweise Bisdibenzofuranderivate (z. B. gemäß EP 2301926) als Matrixmaterialien für phosphoreszierende Emitter verwendet. Allerdings besteht bei Verwendung dieser Matrixmaterialien noch Verbesserungsbedarf, insbesondere in Bezug auf die Effizienz, die Lebensdauer und die Betriebsspannung der Vorrichtung.

EP 2479234 A1 offenbart verschiedene Carbazol-, Dibenzofuran- und Dibenzothiophenderivate für die Verwendung in OLEDs. Neben einer Vielzahl anderer Verbindungen wird auch ein Carbazolderivat offenbart (Verbindung 104), welches in 1-, 3- und 6-Position mit Phenylenpyrimidin-Gruppen substituiert ist und welches am Stickstoffatom mit einer Phenylen-CH₂-Phenylen-N-Carbazolyl-Gruppe substituiert ist.

WO 2009/069442 A1 offenbart verschiedene Carbazol-, Dibenzofuran- und Dibenzothiophenderivate für die Verwendung in OLEDs, welche mit Heteroarylgruppen, wie Pyridin, Pyrimidin oder Triazin substituiert sind.

WO 2011/049325 A2 beschreibt Carbazolderivate, welche mit zwei N-Carbazolylgruppen substituiert sind, für die Verwendung in OLEDs.

Aufgabe der vorliegenden Erfindung ist daher die Bereitstellung von Verbin-dungen, welche sich für den Einsatz in einer fluoreszierenden oder phos-phoreszierenden OLED, insbesondere einer phosphoreszierenden OLED, eignen, beispielsweise als Matrixmaterial oder als Ladungstransportmaterial, insbesondere Lochtransport- bzw. Elektronenblockiermaterial. Insbesondere ist es die Aufgabe der vorliegenden Erfindung, Matrixmaterialien bereitzustellen, welche sich auch für grün und blau phosphoreszierende OLEDs eignen, sowie neue Ladungstransportmaterialien bereitzustellen.

Es wurde überraschend gefunden, dass Elektrolumineszenzvorrichtungen, die Verbindungen gemäß der folgenden Formel 4 enthalten, Verbesserungen gegenüber dem Stand der Technik aufweisen, insbesondere beim Einsatz als Matrixmaterialien für phosphoreszierende Dotanden. 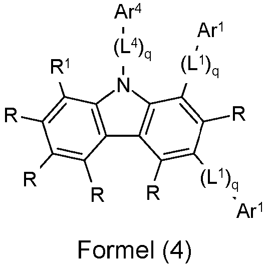 wobei für die verwendeten Symbole und Indizes gilt:
L¹, L⁴ sind bei jedem Auftreten gleich oder verschieden ausgewählt aus den folgenden Formeln (Ar2-1) bis (Ar2-12): 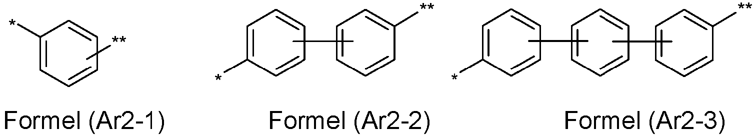 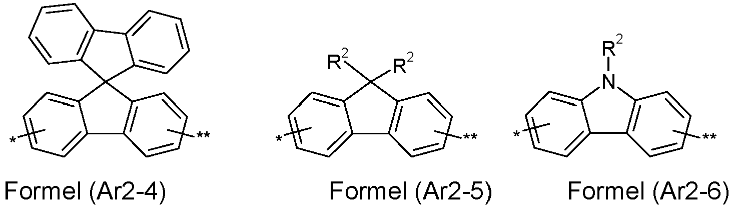 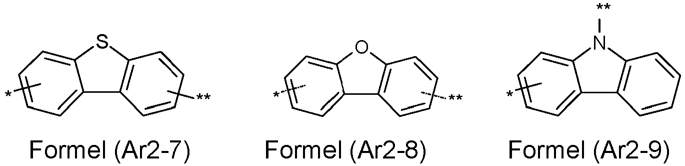 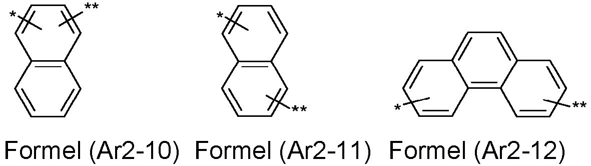 wobei die mit * gekennzeichnete Bindung die Bindung zum Grundgerüst und die mit ** gekennzeichnete Bindung die Bindung zum zugehörigen Rest Ar¹ oder Ar⁴ anzeigt; und wobei die Gruppen (Ar2-1) bis (Ar2-12) an den freien Positionen mit R² substituiert sein können, bevorzugt aber unsubstituiert sind;
   - Ar¹: ist bei jedem Auftreten ein aromatisches Ringsystem mit 6 bis 60 aromatischen Ringatomen, das jeweils auch mit einem oder mehreren Reste R² substituiert sein kann;
   - Ar⁴: ist ausgewählt aus einer der folgenden Formeln (Ar-1), (Ar-5), (Ar-6), (Ar-8) oder (Ar-10), 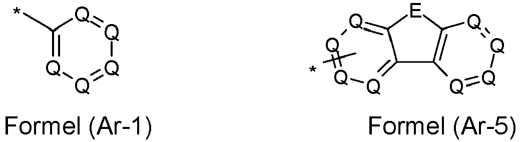 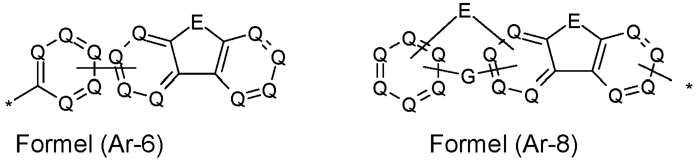 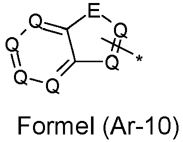
   wobei gilt:
   - Q: ist bei jedem Auftreten gleich oder verschieden CR² oder N, wobei maximal 3 Symbole Q pro Cyclus für N stehen;
   - E: ist bei jedem Auftreten gleich oder verschieden (CR²)₂, NR², O, S oder C=O;
   - G: ist bei jedem Auftreten eine Einfachbindung, (CR²)₂, NR², O, S oder C=O;
   - *: die Bindung zu dem Grundgerüst darstellt;
   - R: ist bei jedem Auftreten gleich oder verschieden H, D, F oder ein aliphatischer Kohlenwasserstoffrest mit 1 bis 20 C-Atomen, wobei ein oder mehrere H-Atome durch D oder F, ersetzt sein können,
   - R¹: ist bei jedem Auftreten H, D, F oder ein aliphatischer Kohlenwasserstoffrest mit 1 bis 20 C-Atomen, wobei ein oder mehrere H-Atome durch D oder F, ersetzt sein können;
   - R²: ist bei jedem Auftreten gleich oder verschieden H, D, F, CN, eine geradkettige Alkyl-gruppe mit 1 bis 10 C-Atomen oder eine verzweigte oder cyclische Alkyl-gruppe mit 3 bis 10 C-Atomen, die jeweils mit einem oder mehreren Resten R³ substituiert sein kann, wobei ein oder mehrere H-Atome durch D oder F ersetzt sein können; oder ein aromatisches Ringsystem mit 5 bis 60 aromatischen Ringatomen, das durch einen oder mehrere Substituenten R³ substituiert sein kanndabei können zwei oder mehrere Substituenten R² zusammen mit den Atomen, an die sie gebunden sind auch miteinander ein mono- oder polycyclisches, aliphatisches oder aromatisches Ringsystem bilden;
   - R³: ist bei jedem Auftreten gleich oder verschieden H, D, F, eine geradkettige Alkyl-gruppe mit 1 bis 40 C-Atomen oder eine verzweigte oder cyclische Alkylgruppe mit 3 bis 40 C-Atomen, wobei ein oder mehrere H-Atome durch D oder F ersetzt sein können;
   - q: ist bei jedem Auftreten gleich oder verschieden 0 oder 1,
mit der Maßgabe, dass die folgenden Verbindungen von der Erfindung ausgenommen sind:

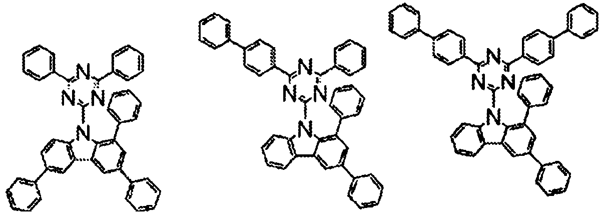

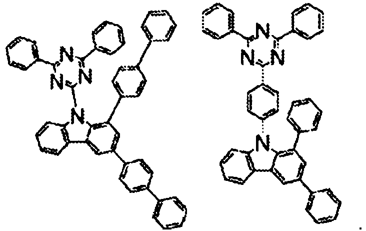

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Thiophen, etc., oder eine kondensierte (anellierte) Aryl- oder Heteroarylgruppe, beispielsweise Naphthalin, Anthracen, Phenanthren, Dibenzofuran, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im RingsystemUnter einem aromatischen Ringsystem im Sinne dieser Erfindung soll ein System verstanden werden, das nicht notwendigerweise nur Arylgruppen enthält, sondern in dem auch mehrere Arylgruppen durch eine nicht-aromatische Einheit (bevorzugt weniger als 10 % der von H verschiedenen Atome), wie ein C--Atom, verbunden sein können. So sollen beispielsweise auch Systeme wie Fluoren, 9,9'-Spirobifluoren, 9,9-Diarylfluoren, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Weiterhin werden miteinander durch Einfachbindung verknüpfte Aromaten, wie zum Beispiel Biphenyl, als aromatisches Ringsystem im Sinne dieser Anmeldung bezeichnet.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc.. Dabei können an diese Gruppen auch noch weitere 6-Ring-Aryl- oder 6-Ring-Heteroarylgruppen ankondensiert sein, wie beispielsweise in Benzimidazol oder Chinolin. Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkenyl-, Alkinyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen Ringsystem mit 6 - 60 aromatischen Ringatomen, welches noch jeweils mit den oben genannten Resten substituiert sein kann und welches über beliebige Positionen am Aromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, , Truxen, Isotruxen, Spirotruxen, Spiroisotruxen, oder Gruppen, die abgeleitet sind von Kombinationen dieser Systeme. Dabei können diese Gruppen jeweils durch die oben genannten Reste substituiert sein.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc. Dabei können an diese Gruppen auch noch weitere 6-Ring-Aryl- oder 6-Ring-Heteroarylgruppen ankondensiert sein, wie beispielsweise in Benzimidazol oder Chinolin. Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht: Ringbildung

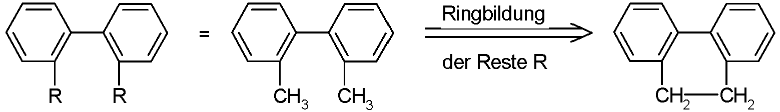

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

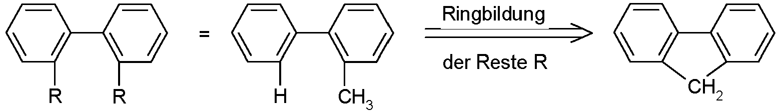

In einer bevorzugten Ausführungsform der Erfindung ist R bei jedem Auftreten gleich oder verschieden H, D oder F.

In einer bevorzugten Ausführungsform der Erfindung ist R¹ bei jedem Auftreten gleich oder verschieden H, D oder F, besonders bevorzugt H oder D.

In einer bevorzugten Ausführungsform der Erfindung ist die Gruppe Ar¹ bei jedem Auftreten ausgewählt aus den Gruppen mit den folgenden Formeln (Ar-1) bis (Ar-11): 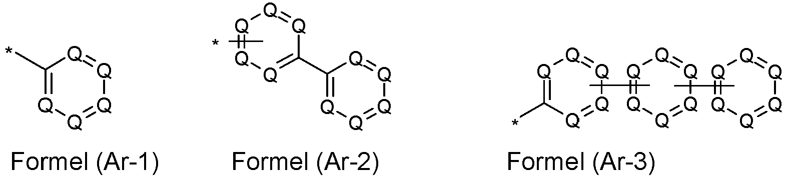 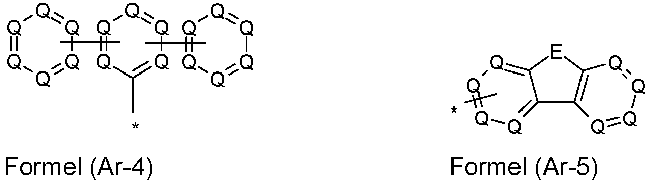 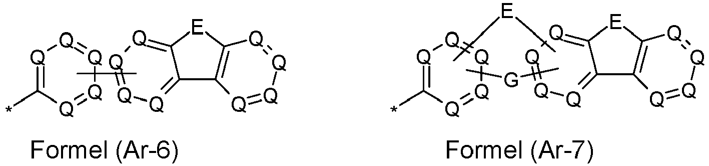 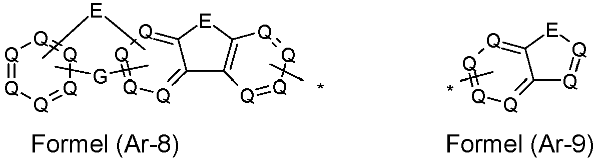 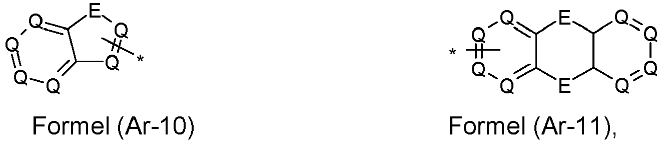 wobei die Symbole und Indizes den Symbolen und Indizes der Formel (4) entsprechen und zusätzlich gilt:
- Q: ist bei jedem Auftreten gleich oder verschieden CR² oder N, wobei maximal 3 Symbole Q pro Cyclus für N stehen, wobei für die Gruppe Ar¹ Q nicht für N stehen kann;
- E: ist bei jedem Auftreten gleich oder verschieden (CR²)₂ oder C=O, wobei für die Gruppe Ar¹ E nicht für NR², O oder S stehen kann;
- G: ist bei jedem Auftreten eine Einfachbindung, (CR²)₂, NR², O, S oder C=O, wobei für die Gruppe Ar¹ EG nicht für NR², O oder S stehen kann
und
* die Bindung zu L¹ oder zu dem Grundgerüst darstellt.

In einer weiteren bevorzugten Ausführungsform der Erfindung ist mindestens eine der Gruppen Ar¹ oder Ar⁴ ausgewählt aus einer der Formeln (Ar-5), (Ar-6), (Ar-8) oder (Ar-10), wobei für die Gruppe Ar¹ die obigen Einschränkungen gelten.

In einer weiteren besonders bevorzugten Ausführungsform der Erfindung umfasst die Gruppe Ar⁴ eine elektronenarme Heteroarylgruppe. Bevorzugt ist die Gruppe Ar⁴ ausgewählt aus einer der Formeln (Ar-1-2), (Ar-1-3), (Ar-1-4) oder (Ar-1-5), besonders bevorzugt aus einer der Formeln (Ar-1-2) oder (Ar-1-5).

In einer weiteren bevorzugten Ausführungsform stehen in der Formel (Ar-1) 0, 2 oder 3 Symbole Q für N.

Bevorzugte Ausführungsformen der Formel (Ar-8) zeigen die folgenden Formeln (Ar-8-1) bis (Ar-8-7): 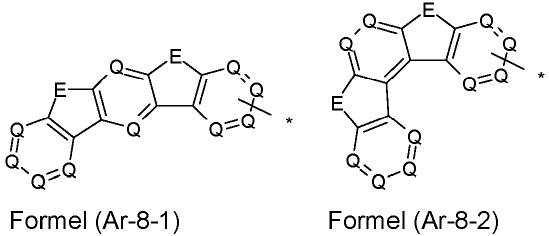 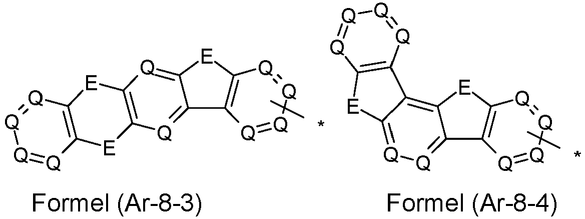 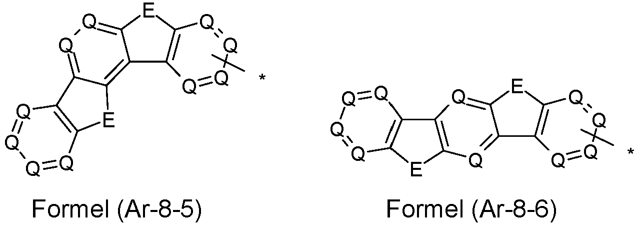 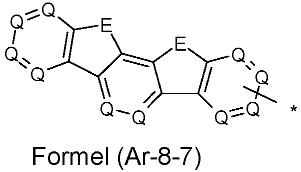 wobei die Symbole den Symbolen der Formel (Ar-8) entsprechen und wobei für die Gruppe Ar¹ die obigen Einschränkungen gelten. Besonders bevorzugt ist Q immer CR².

In einer weiteren bevorzugten Ausführungsform ist die Gruppe Ar¹ bei jedem Auftreten gleich oder verschieden ausgewählt aus den Gruppen mit den Strukturen der Formeln (Ar-1) bis (Ar-11), wobei die allgemeinen Formeln durch die besonders bevorzugten Ausführungsformen jeweils gemäß der folgenden Formeln (Ar-1-1) bis (Ar-11-1) ersetzt werden (z. B. wird Formel (Ar-1) durch eine der Formeln (Ar-1-1) und (Ar-1-6) bis (Ar-1-9) ersetzt): 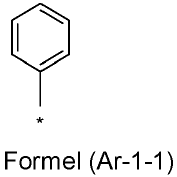 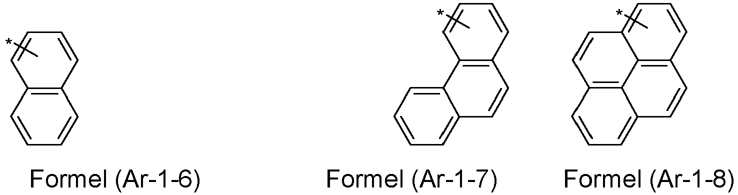 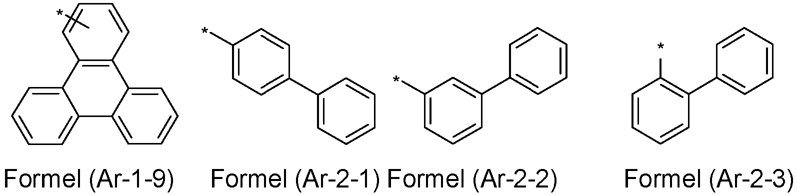 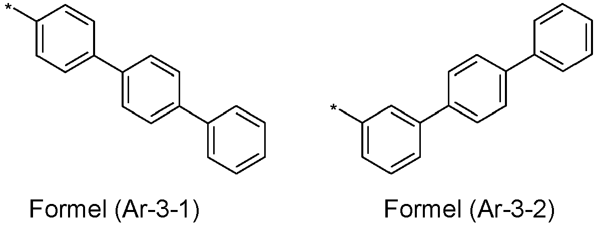 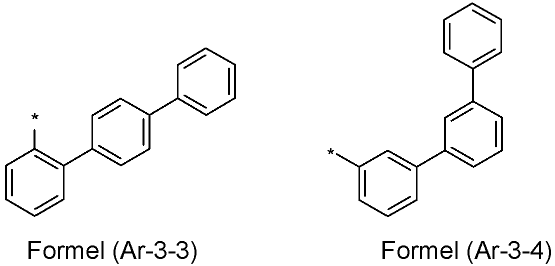 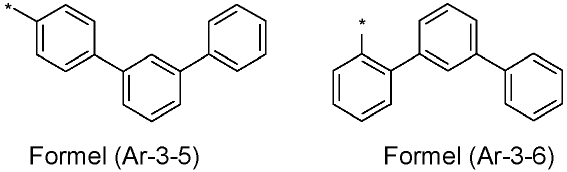 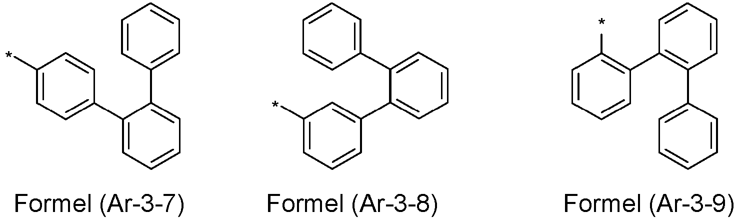 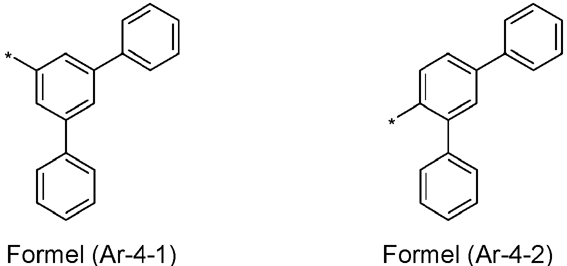 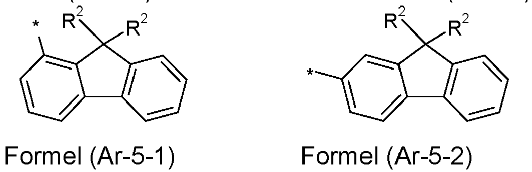 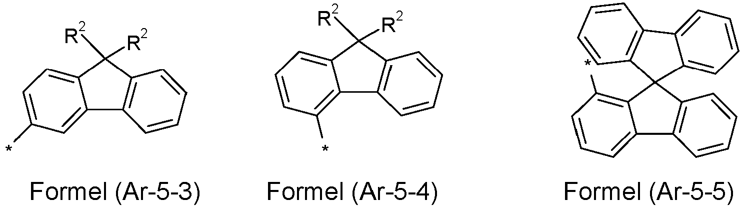 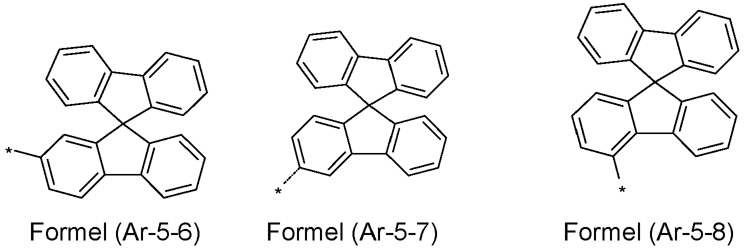 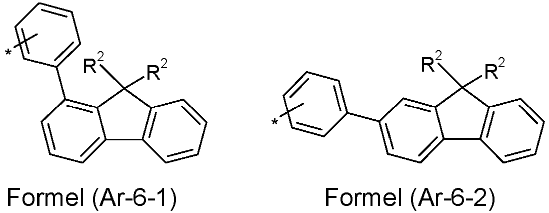 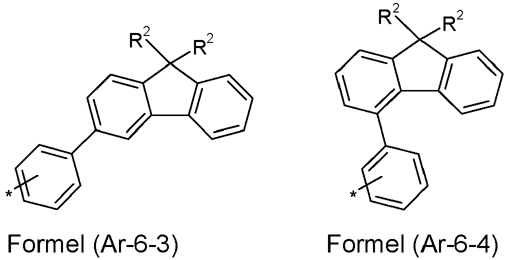 wobei die Symbole den Symbolen in Formel (4) entsprechen. Die Formeln können an den freien Positionen mit R² substituiert sein.

In einer weiteren Ausführungsform der Erfindung ist die Gruppe Ar⁴ bevorzugt ausgewählt aus den Formeln (Ar-1-1) bis (Ar-1-9), (Ar-5-1) bis (Ar-5-20), (Ar-6-1) bis (Ar-6-16), (Ar-8-1-1) bis (Ar-8-7-6) und (Ar-10-1): 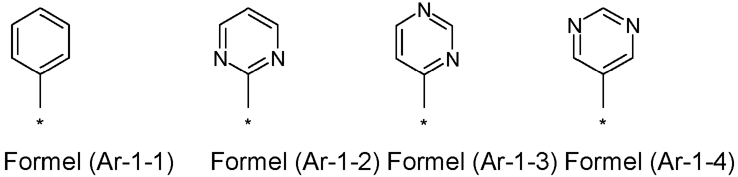 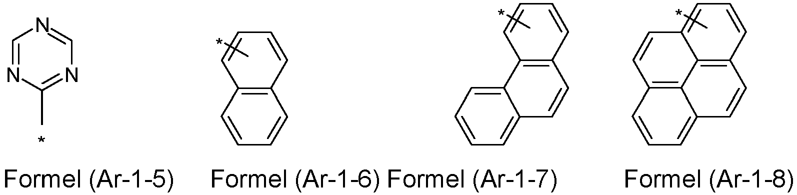 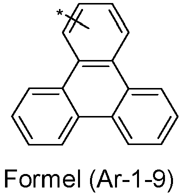 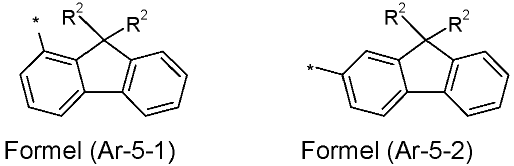 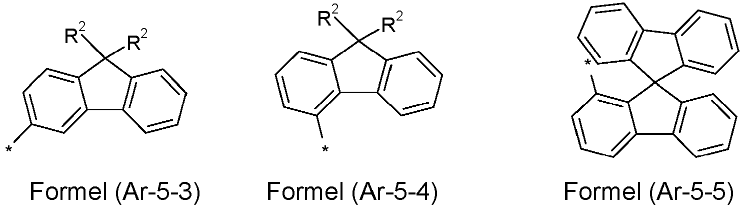 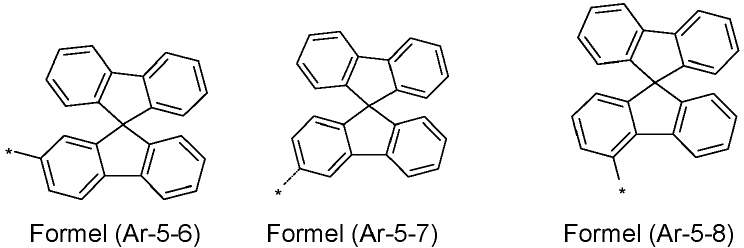 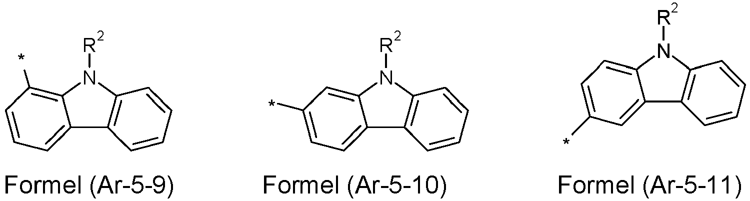 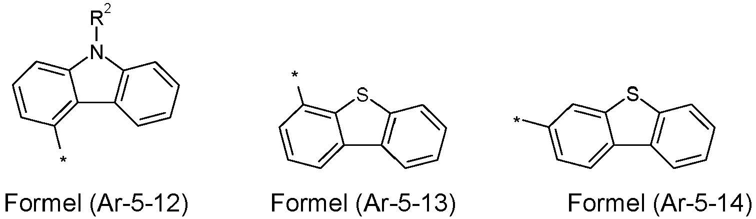 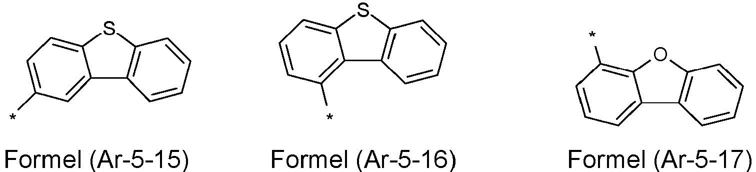 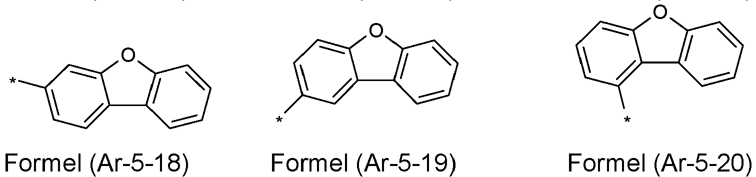 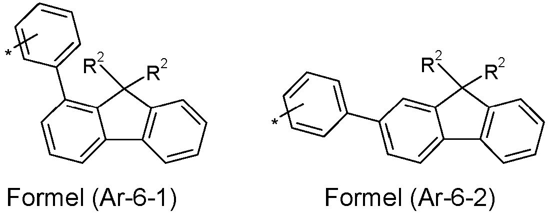 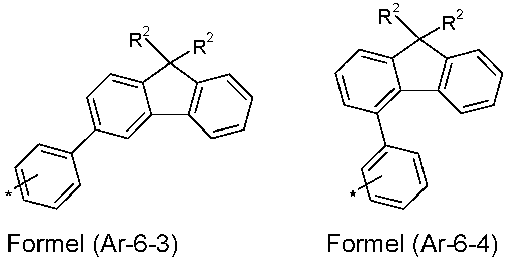 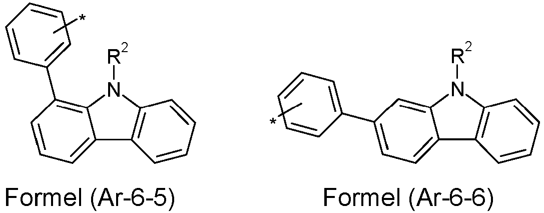 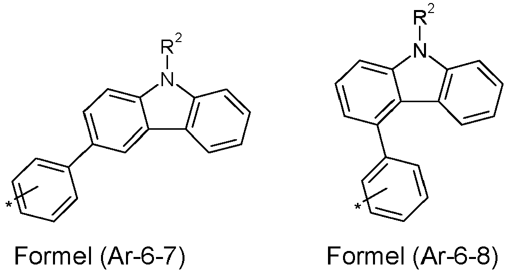 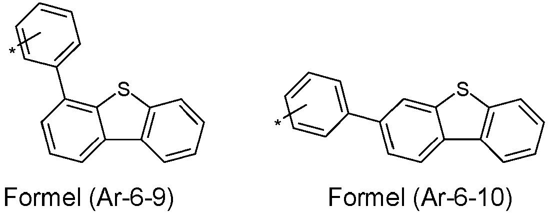 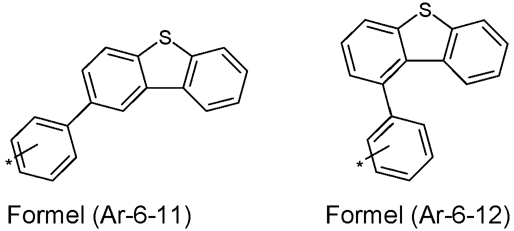 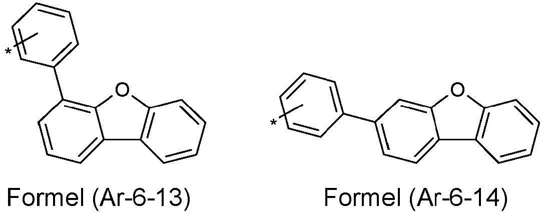 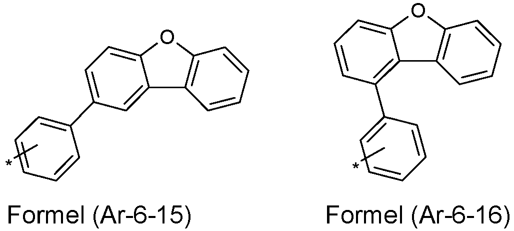 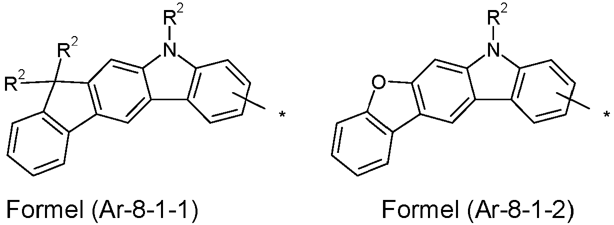 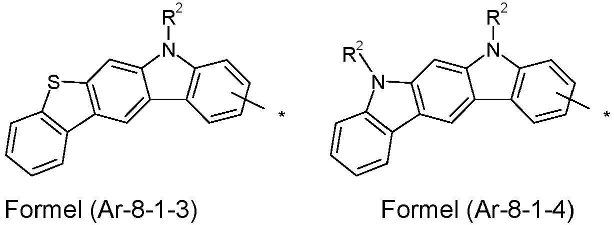 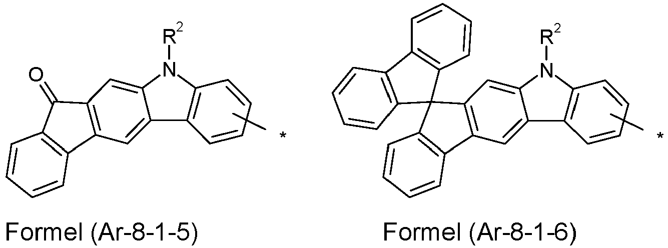 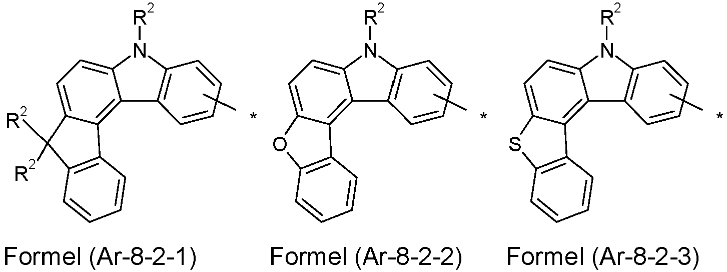 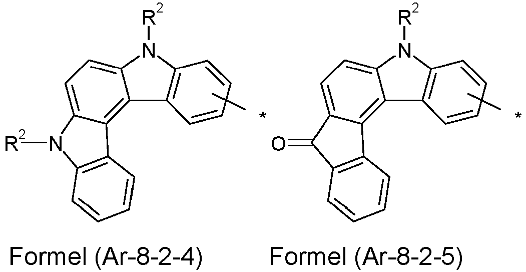 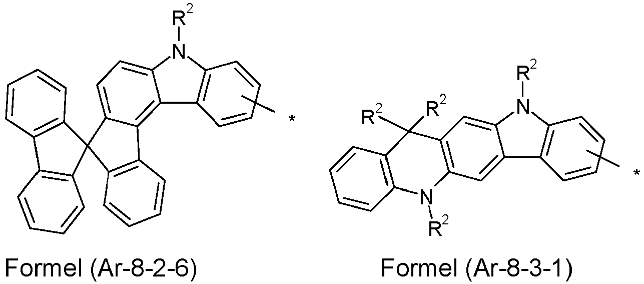 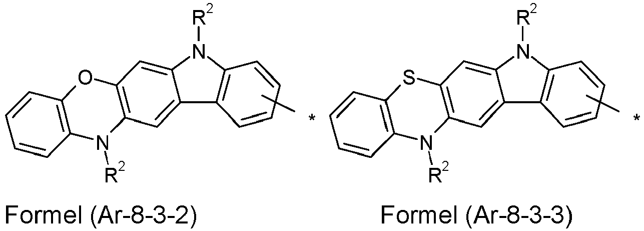 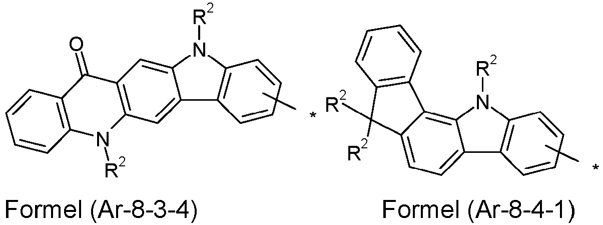 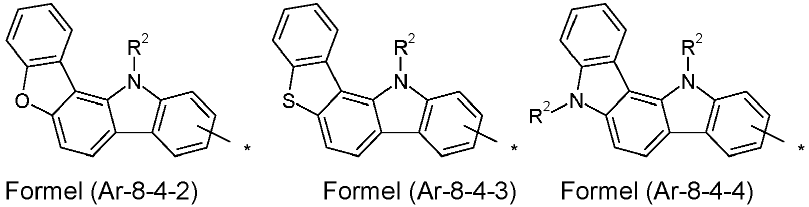 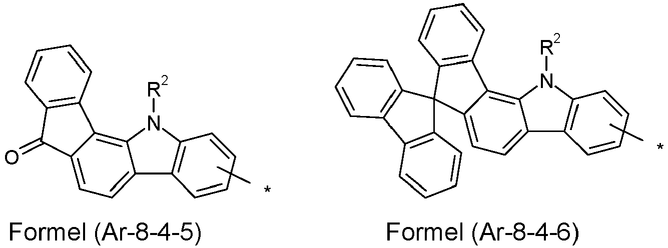 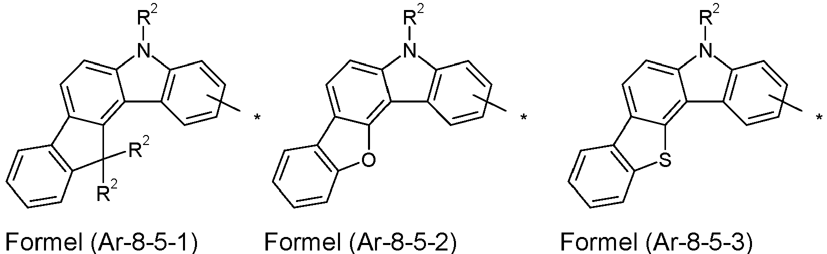 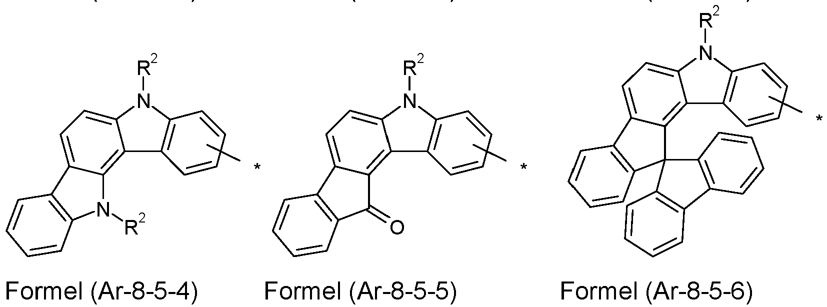 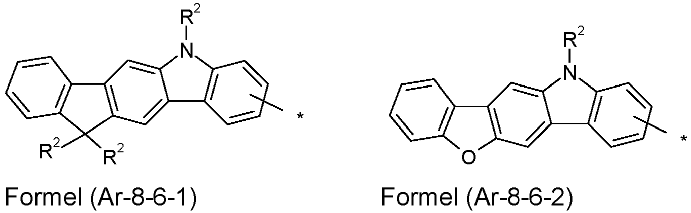 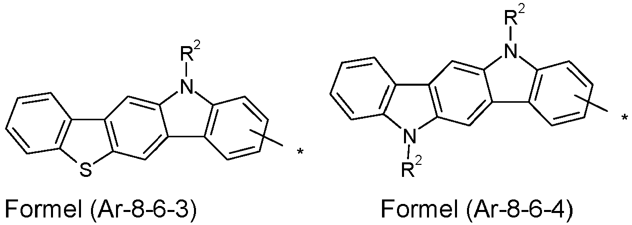 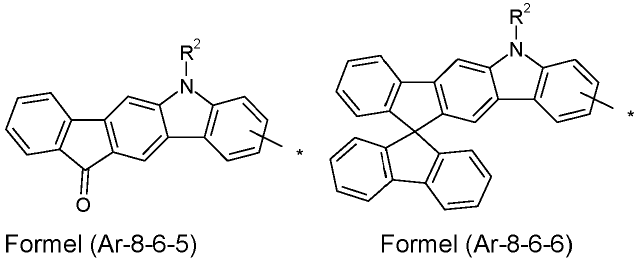 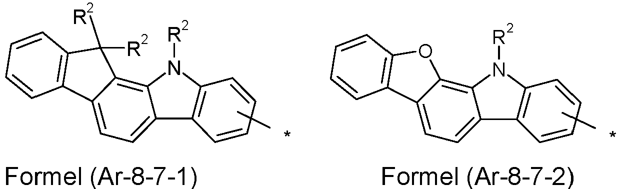 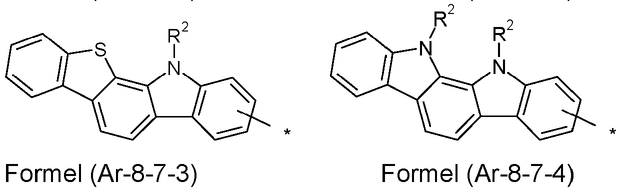 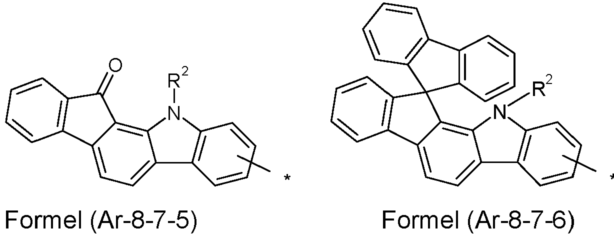 und

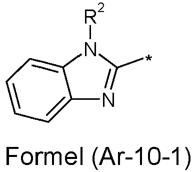

Die Formeln können an den freien Positionen mit R² substituiert sein.

In einer weiteren Ausführungsform der Erfindung sind die Gruppen gemäß Formel (Ar-8) bzw. deren bevorzugte Ausführungsformen für die Gruppe Ar⁴ ausgewählt aus den Gruppen gemäß eine der Formeln (Ar-8-1-1a) bis (Ar-8-7-6a) 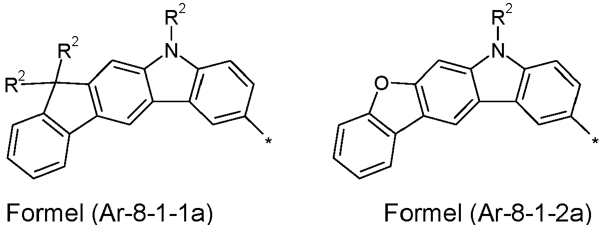 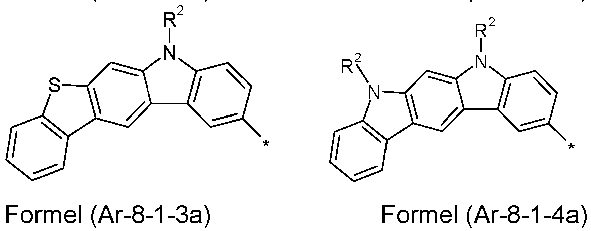 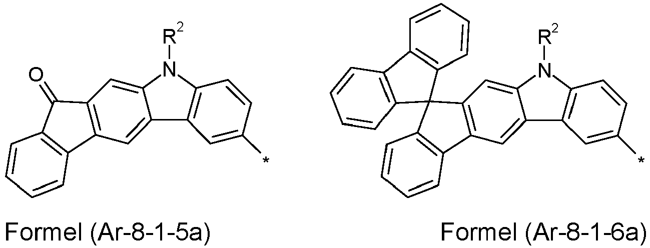 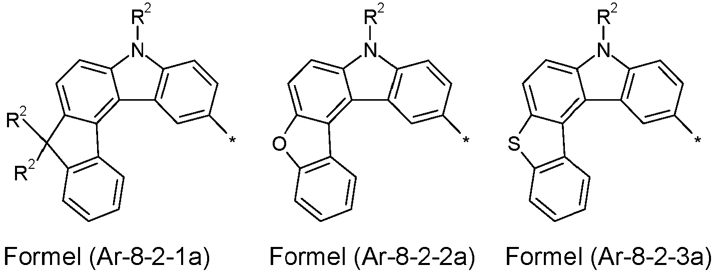 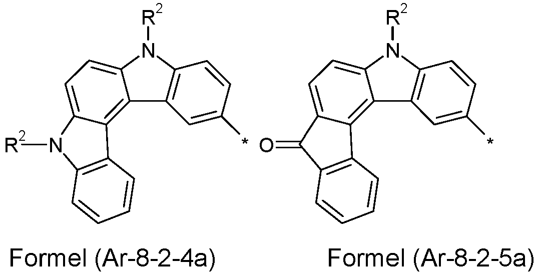 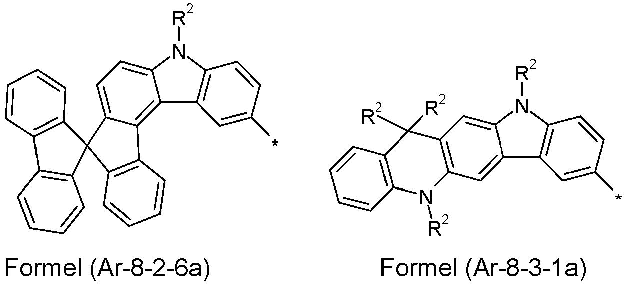 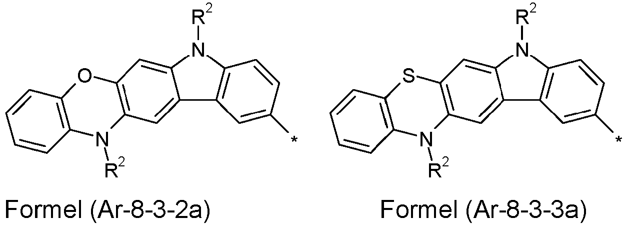 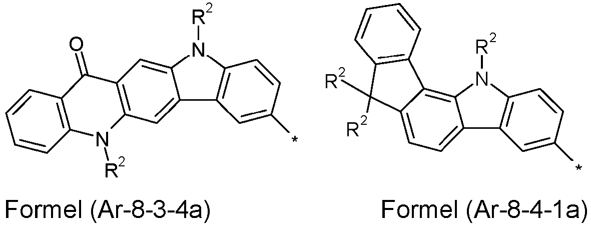 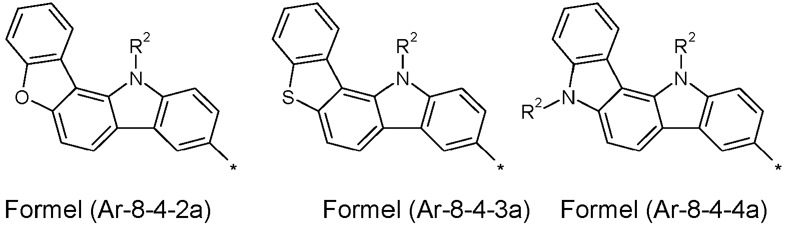 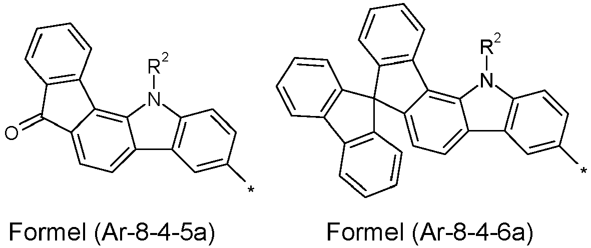 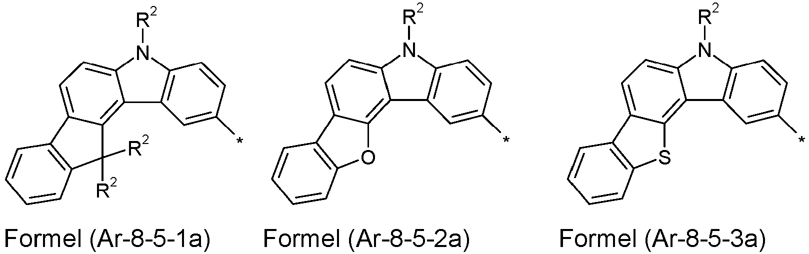 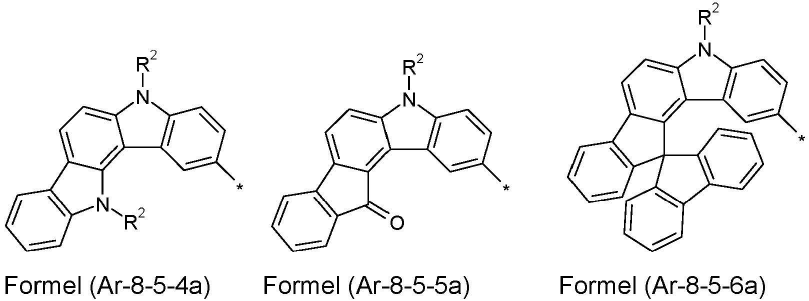 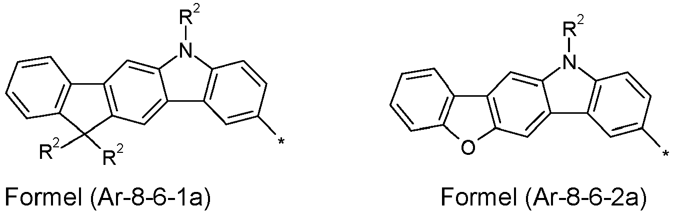 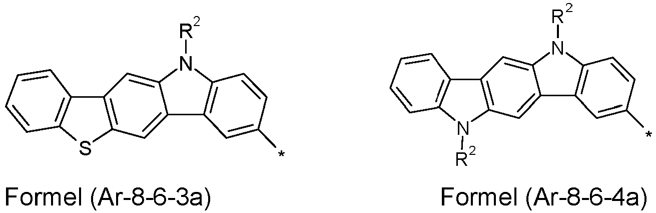 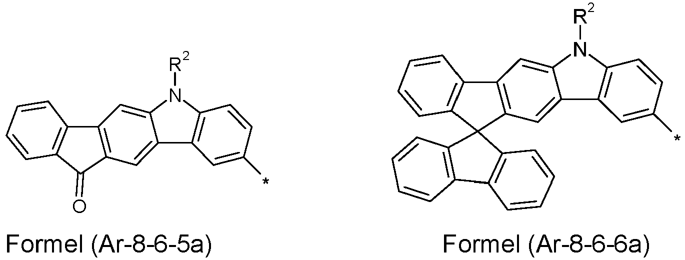 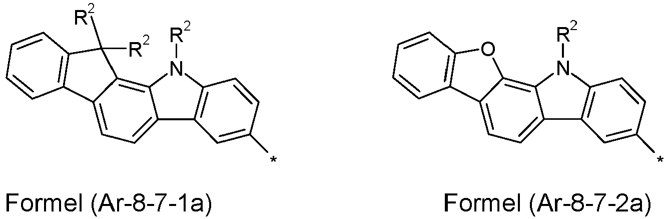 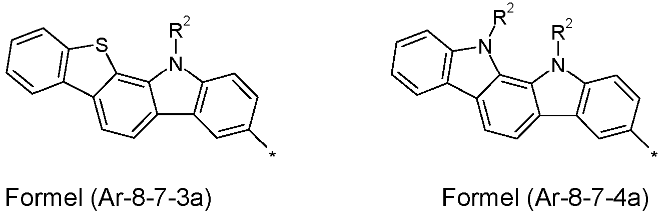 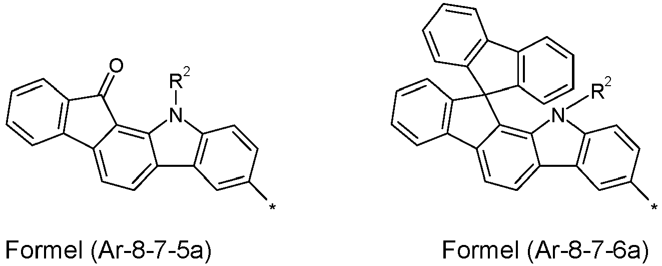 wobei die Symbole den Symbolen in Formel (4) entsprechen. Die Formeln können an den freien Positionen mit R² substituiert sein.

In einer weiteren besonders bevorzugten Ausführungsform der Erfindung ist die Gruppe Ar⁴ ausgewählt aus einer der Formeln (Ar-8-1-1) bis (Ar-8-7-6), bevorzugt aus einer der Formeln (Ar-8-1-1a) bis (Ar-8-7-6a).

In einer weiteren Ausführungsform der Erfindung umfasst die Verbindung der Formel (4) keine zwei Dibenzofuran-Derivate und/oder Dibenzothiophen-Derivate. Dies können unsubstituierte Dibenzofurane (z.B. Formeln (Ar-5-17), (Ar-5-18), (Ar-5-19), (Ar-5-20), (Ar2-8)) oder substituierte Dibenzofurane (z. B. Formeln (Ar-6-13), (Ar-6-14), (Ar-6-15), (Ar-6-16)) sein. Die Dibenzofuranstruktur kann aber auch Teil eines kondensierten heteroaromatischen Ringsystem sein (z.B. Formeln (Ar-8-1-2), (Ar-8-4-2), (Ar-8-5-2), (Ar-8-6-2), (Ar-8-7-2)). Dies können unsubstituierte Dibenzothiophene (z.B. Formel (Ar-5-13), (Ar-5-14), (Ar-5-15), (Ar-5-16)) oder substituierte Dibenzothiophene (z. B. Formel (Ar-6-9), (Ar-6-10), (Ar-6-11), (Ar-6-12), (Ar2-7)) sein. Die Dibenzothiophenstruktur kann aber auch Teil eines kondensierten heteroaromatischen Ringsystems sein (z.B. Formeln (Ar-8-1-3), (Ar-8-2-3), (Ar-8-4-3), (Ar-8-5-3), (Ar-8-6-3) oder (Ar-8-7-3)).

In einer weiteren Ausführungsform der Erfindung ist die Gruppe Ar¹ bei jedem Auftreten gleich oder verschieden ausgewählt aus den Formeln bevorzugt (Ar-1) bis (Ar-11) oder ihren bevorzugten Ausführungsformen, und ist die Gruppe Ar⁴ ausgewählt aus den Formeln (Ar-1), (Ar-5), (Ar-6), (Ar-8) und (Ar-10) oder ihren bevorzugten Ausführungsformen, und sind die Gruppen L¹ und/oder L⁴, soweit vorhanden, gleich oder verschieden ausgewählt aus den Formeln (Ar2-1) bis (Ar2-12).

In einer weiteren bevorzugten Ausführungsform ist R², welcher an eine Kohlenstoffbrücke in einem aromatischen oder heteroaromatischen Ringsystem bindet, wie beispielsweise in den Formeln (Ar-5-1), (Ar-5-2), (Ar-5-3), (Ar-5-4), (Ar-6-1), (Ar-6-2), (Ar-6-3), (Ar-6-4), (Ar-8-3-1), (Ar-11-1), (Ar-18-1) oder (Ar2-8b), gleich oder verschieden bei jedem Auftreten ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das wie oben definiert ist und das mit einem oder mehreren Resten R³ substituiert sein kann. Dabei können die beiden Gruppen R² auch miteinander ein Ringsystem bilden, welches aliphatisch oder zusätzlich zur oben gegebenen Definition von R² auch aromatisch sein kann. Durch Ringbildung wird ein Spiro-System aufgespannt.

In einer weiteren bevorzugten Ausführungsform ist R², welcher an ein Stickstoffatom bindet, ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, insbesondere ein aromatisches Ringsystem mit 6 bis 24 C-Atomen, das wie oben definiert ist, und das mit einem oder mehreren Resten R³ substituiert sein kann.

Die oben genannten Ausführungsformen können beliebig miteinander kombiniert werden. Insbesondere ist es bevorzugt, die oben aufgeführten bevorzugten Ausführungsformen miteinander zu kombinieren.

Beispiele für bevorzugte Verbindungen gemäß den oben aufgeführten Ausführungsformen bzw. Verbindungen, wie sie bevorzugt in organischen elektronischen Vorrichtungen eingesetzt werden können, sind die folgenden Verbindungen:

| | |
|---|---|
| 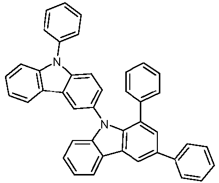 | 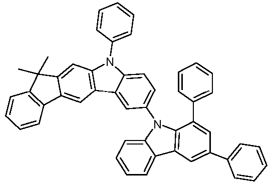 |
| (1) | (2) |
| 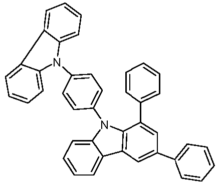 | 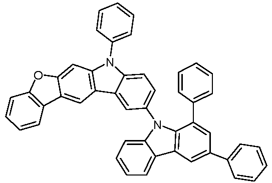 |
| (3) | (4) |
| 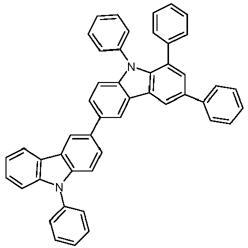 | 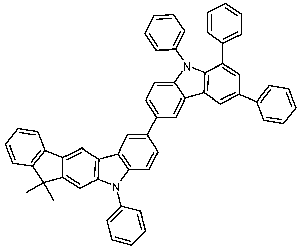 |
| (5)* | (6)* |
| 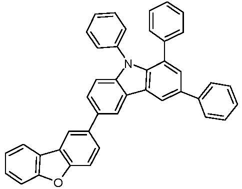 | 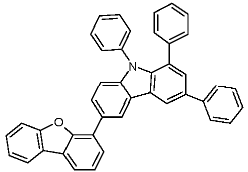 |
| (7)* | (8)* |
| 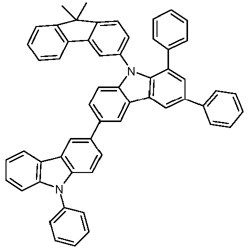 | 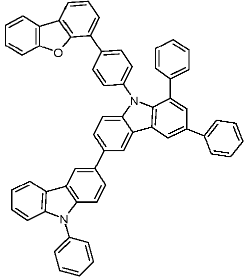 |
| (9)* | (10)* |
| 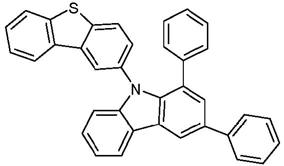 | 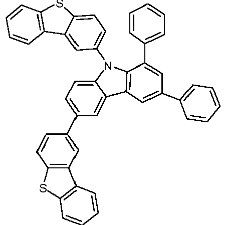 |
| (11) | (12)* |
| 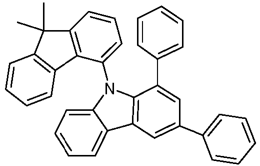 | 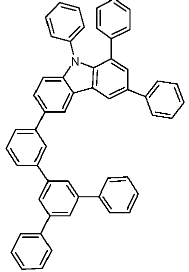 |
| (13) | (14)* |
| 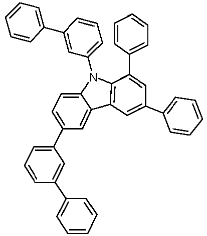 | 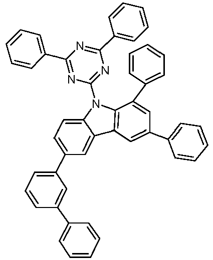 |
| (15)* | (16)* |
| 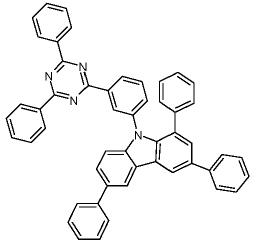 | 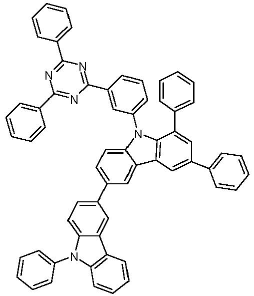 |
| (17)* | (18)* |
| 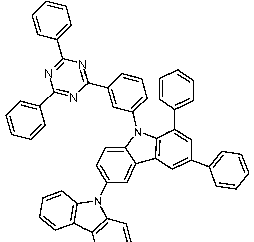 | 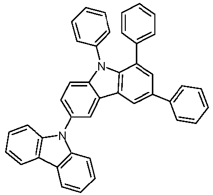 |
| (19)* | (20)* |
| 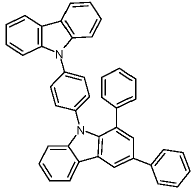 | 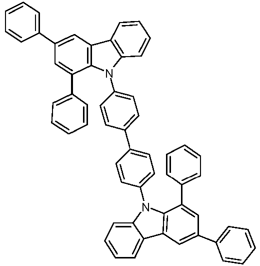 |
| (21) | (22) |
| 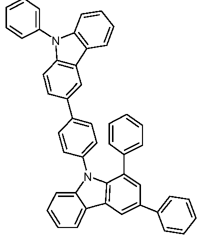 | 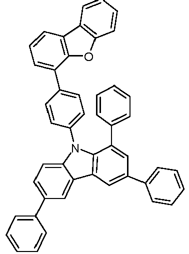 |
| (23) | (24)* |
| 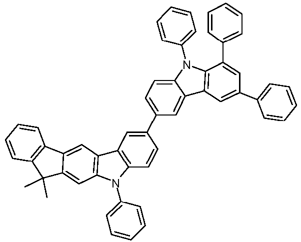 | 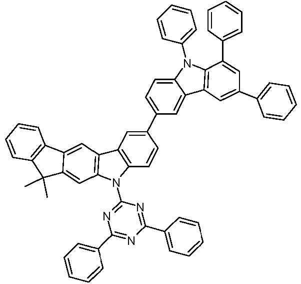 |
| (25)* | (26)* |
| 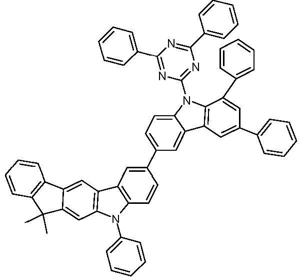 | 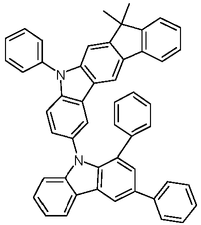 |
| (27)* | (28) |
| 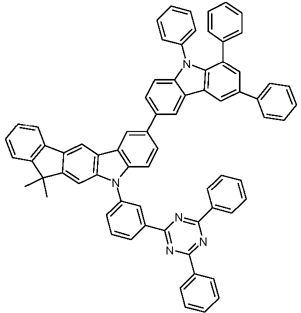 | 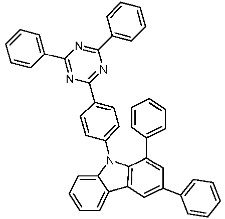 |
| (29)* | (30) |
| 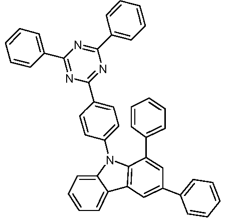 | 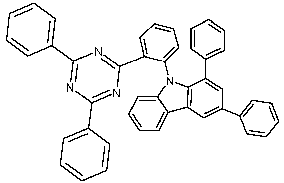 |
| (31) | (32) |
| 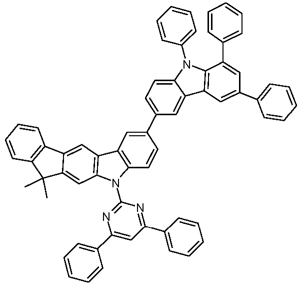 | 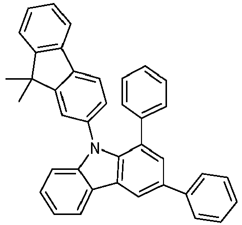 |
| (33)* | (34) |
| 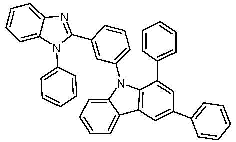 | 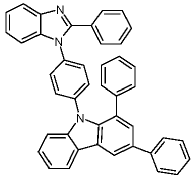 |
| (35)* | (36)* |
| 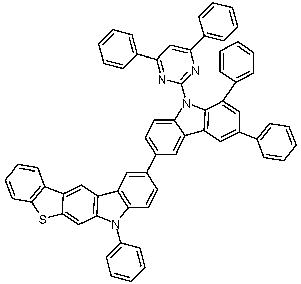 | 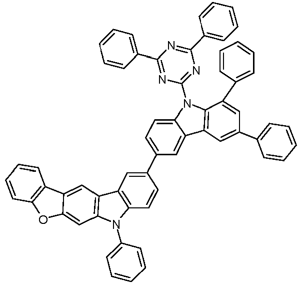 |
| (37)* | (38)* |
| 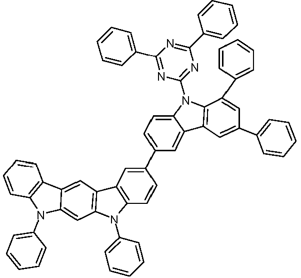 | 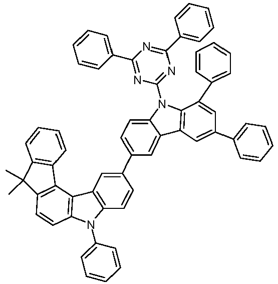 |
| (39)* | (40)* |
| 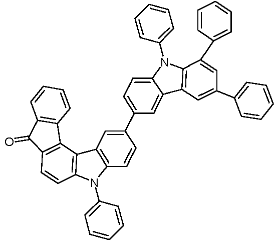 | 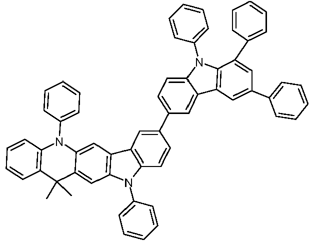 |
| (41)* | (42)* |
| 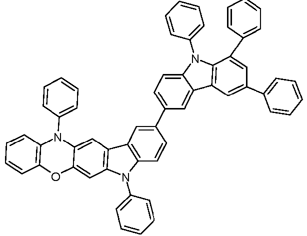 | 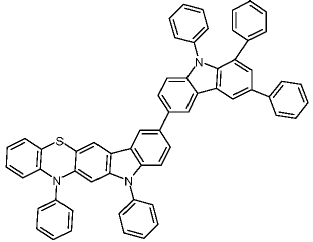 |
| (43)* | (44)* |
| 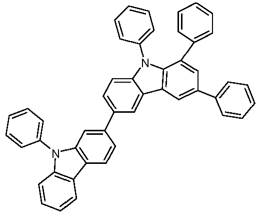 | 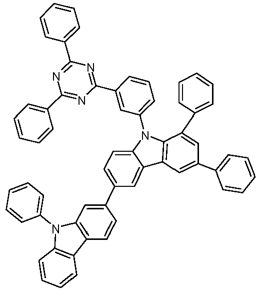 |
| (45)* | (46)* |
| 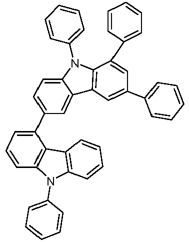 | 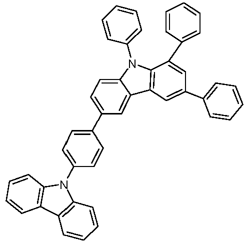 |
| (47)* | (48)* |
| 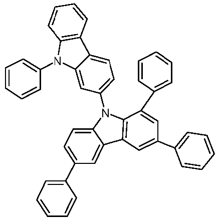 | 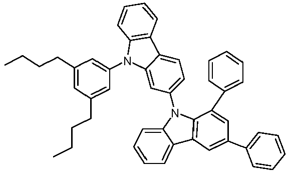 |
| (49)* | (50) |
| 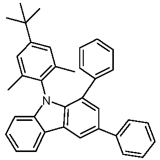 | 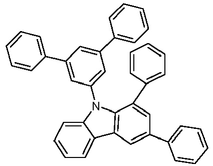 |
| (51) | (52) |
| 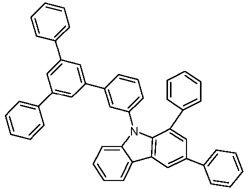 | 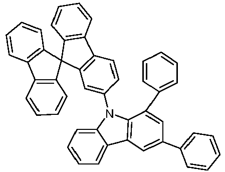 |
| (53) | (54) |
| 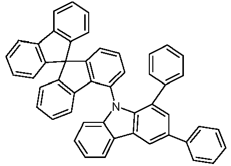 | 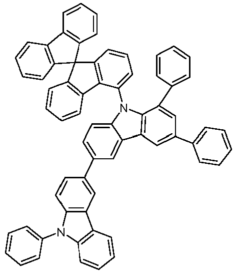 |
| (55) | (56)* |
| 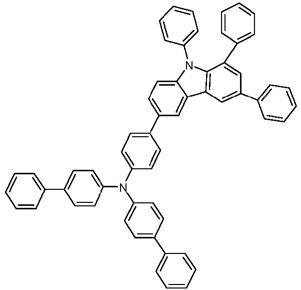 | 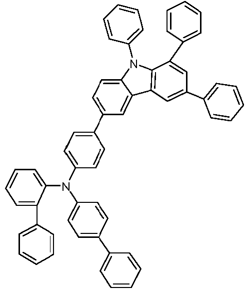 |
| (57)* | (58)* |
| 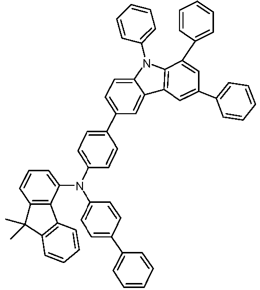 | 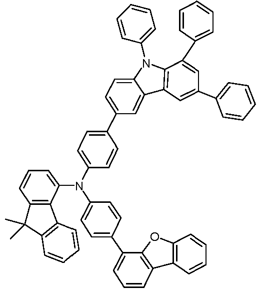 |
| (59)* | (60)* |
| 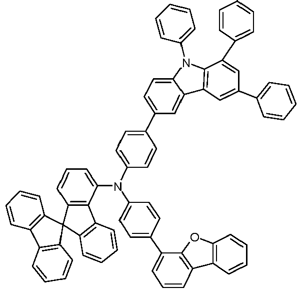 | 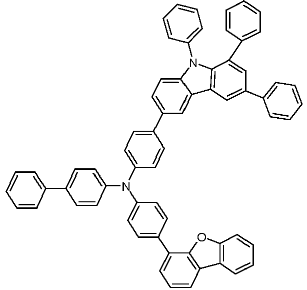 |
| (61)* | (62)* |
| 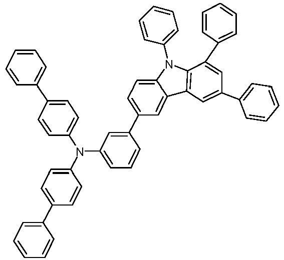 | 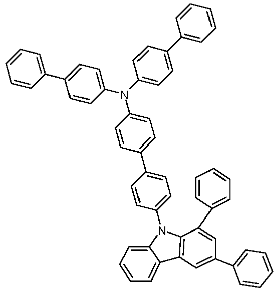 |
| (63)* | (64) |
| 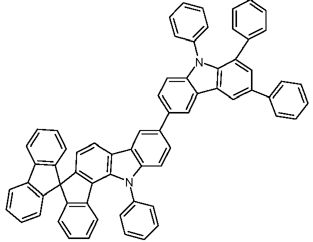 | 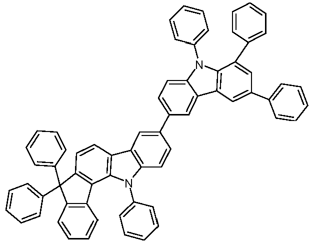 |
| (65)* | (66)* |
| 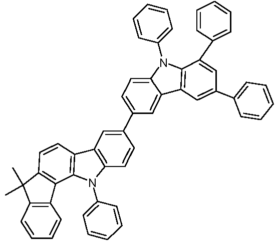 | 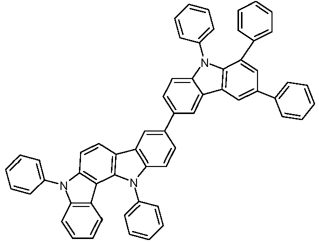 |
| (67)* | (68)* |
| 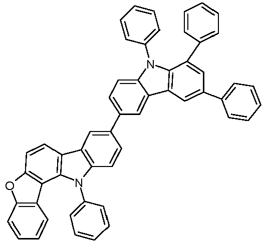 | 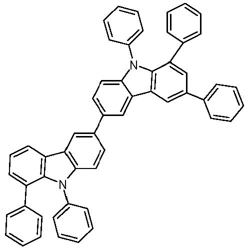 |
| (69)* | (70)* |
| 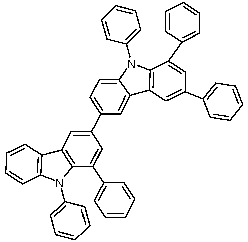 | 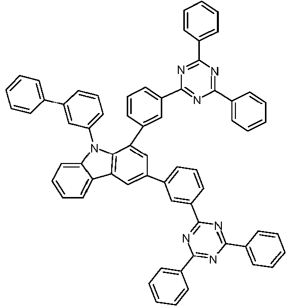 |
| (71)* | (72) |
| 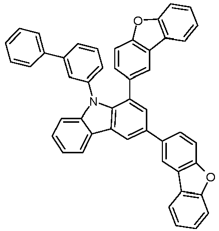 | 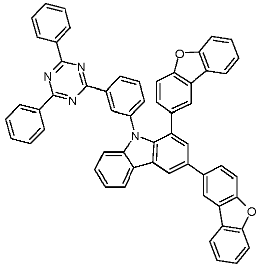 |
| (73)* | (74)* |
| 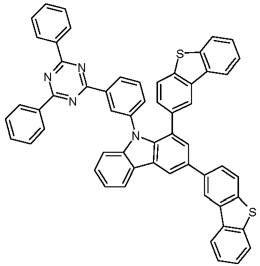 | 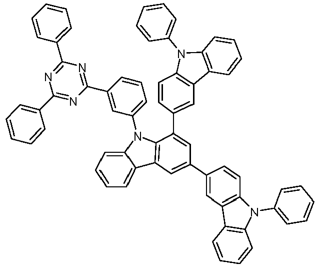 |
| (75)* | (76)* |
| 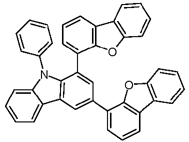 | 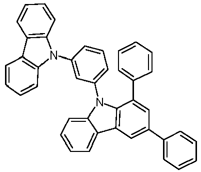 |
| (77)* | (78) |
| 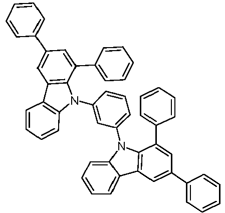 | 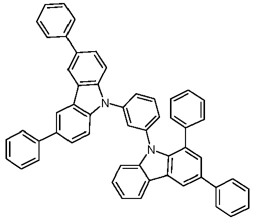 |
| (79) | (80) |
| 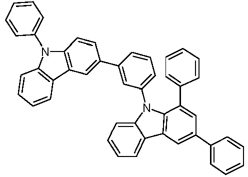 | 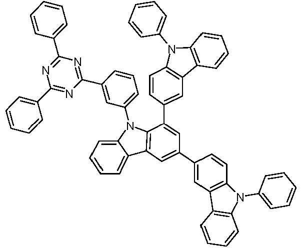 |
| (81) | (82)* |
| 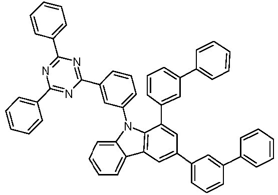 | 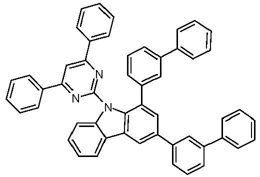 |
| (83) | (84) |
| 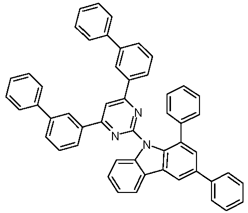 | 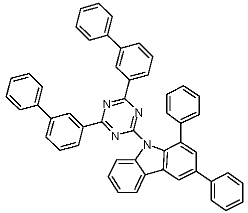 |
| (85) | (86) |
| 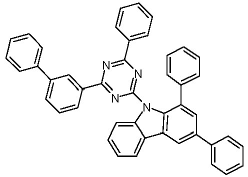 | 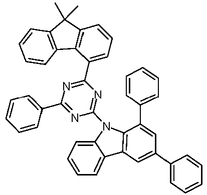 |
| (87) | (88) |
| 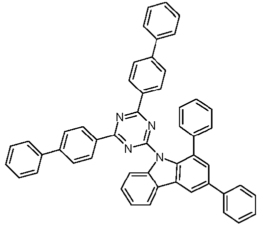 | 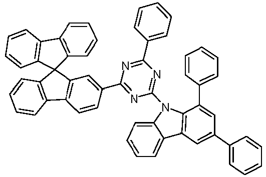 |
| (89) | (90) |
| 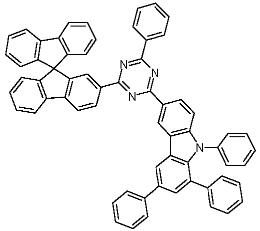 | 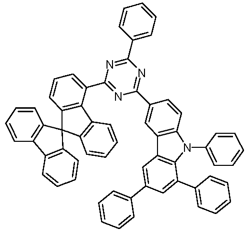 |
| (91)* | (92)* |
| 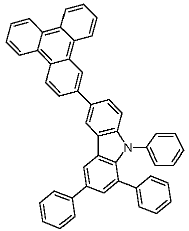 | 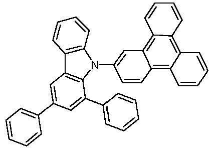 |
| (93)* | (94) |
| 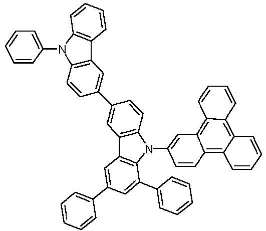 | 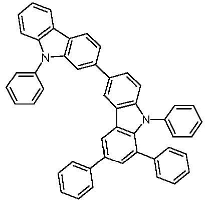 |
| (95)* | (96)* |
| 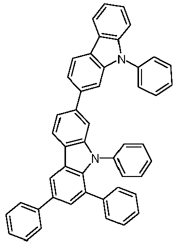 | 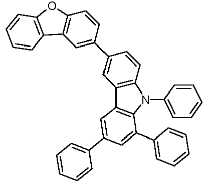 |
| (97)* | (98)* |
| 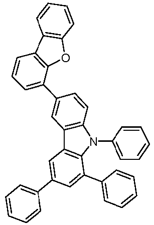 | 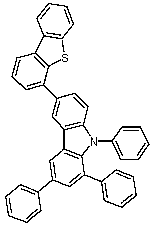 |
| (99)* | (100)* |
| 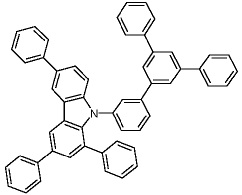 | 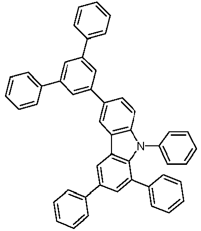 |
| (101)* | (102)* |
| 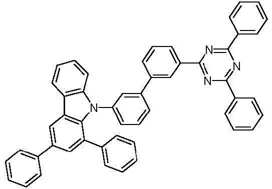 | 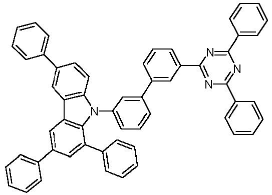 |
| (103) | (104)* |
| 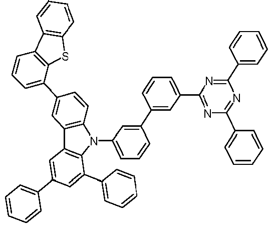 | 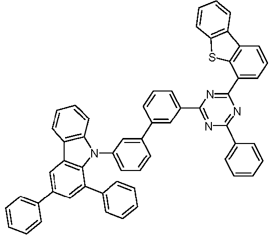 |
| (105)* | (106) |
| 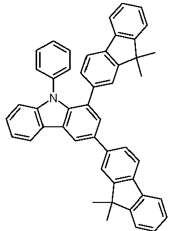 | 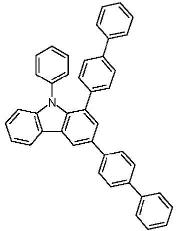 |
| (107) | (108) |
| 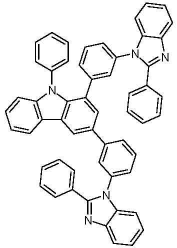 | 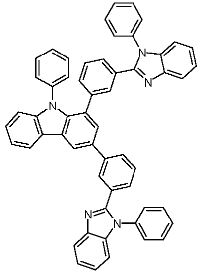 |
| (109) | (110) |
| 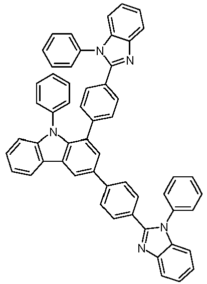 | 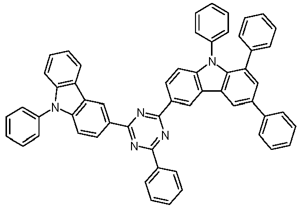 |
| (111) | (112)* |
| 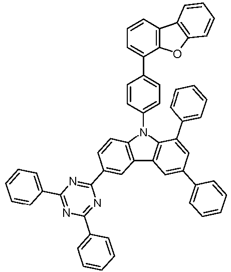 | 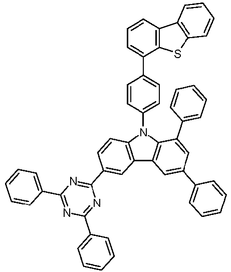 |
| (113)* | (114)* |
| 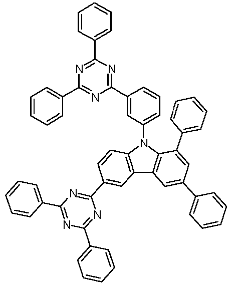 | 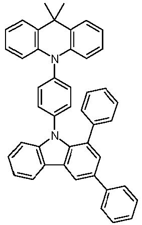 |
| (115)* | (116) |
| 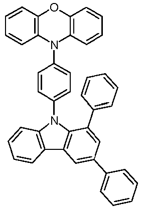 | 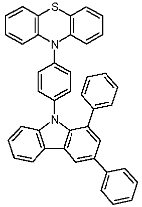 |
| (117) | (118) |
| 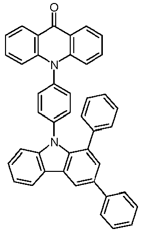 | 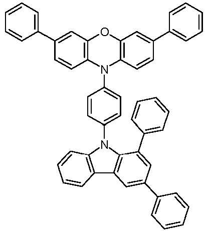 |
| (119) | (120) |
| 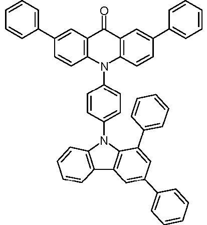 | 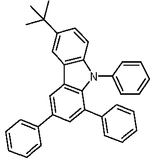 |
| (121) | (122) |
| 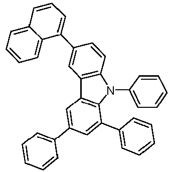 | 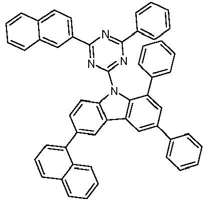 |
| (123)* | (124)* |
| 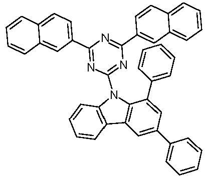 | 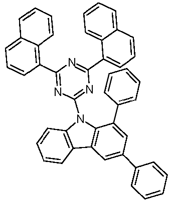 |
| (125) | (126) |
| 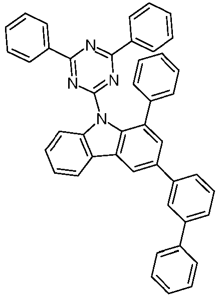 | 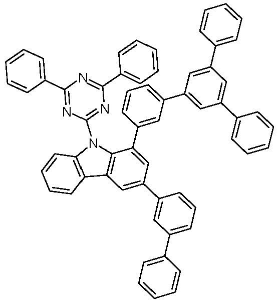 |
| (127) | (128) |
| 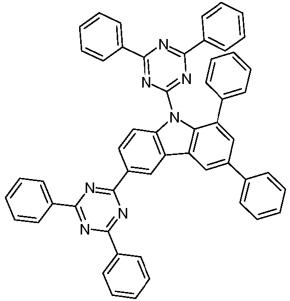 | 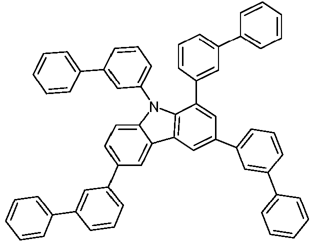 |
| (129)* | (130)* |
| 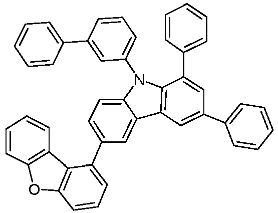 | 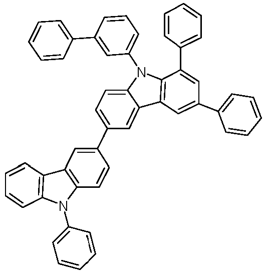 |
| (131)* | (132)* |
| 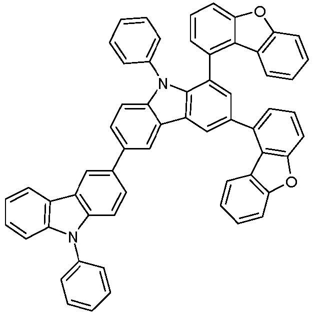 | 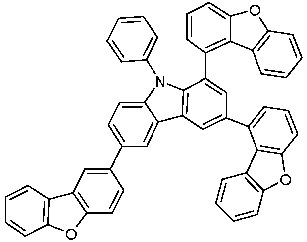 |
| (133)* | (134)* |
| 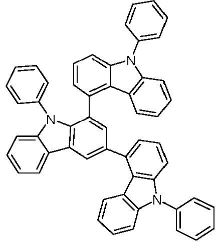 | 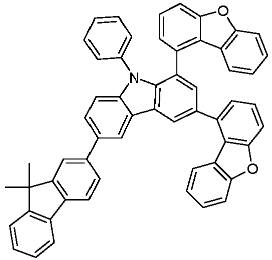 |
| (135)* | (136)* |
| 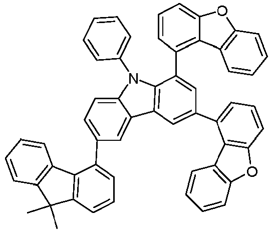 | 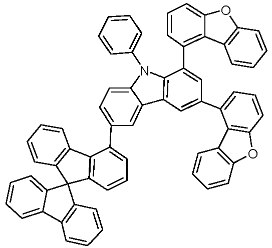 |
| (137)* | (138)* |
| 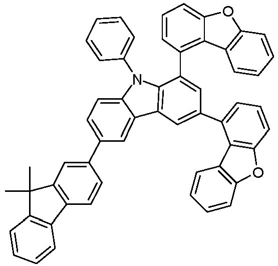 | 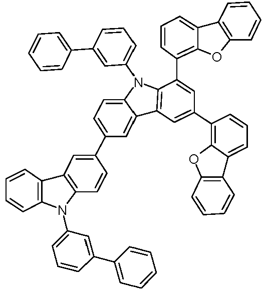 |
| (139)* | (140)* |
| 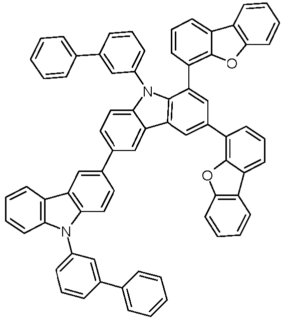 | 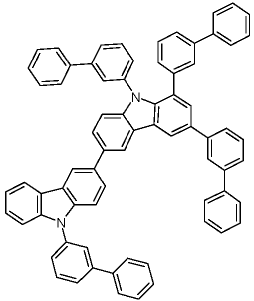 |
| (141)* | (142)* |
| 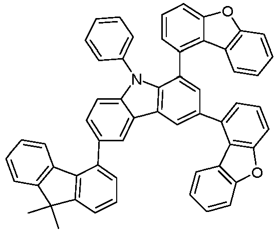 | 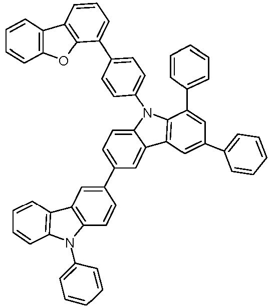 |
| (143)* | (144)* |
| 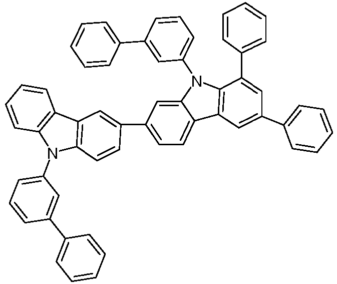 | 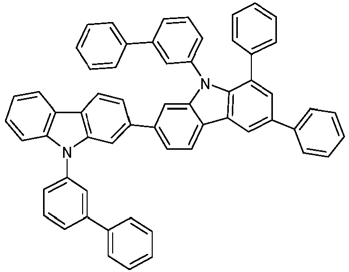 |
| (145)* | (146)* |
| 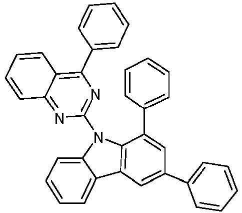 | 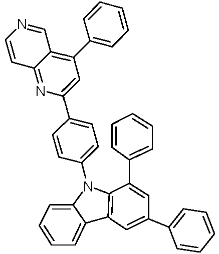 |
| (147) | (148) |
| 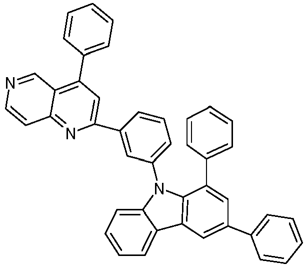 | 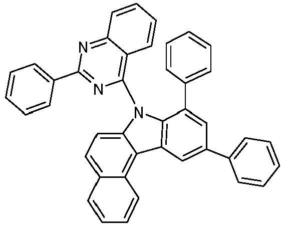 |
| (149) | (150)* |
| 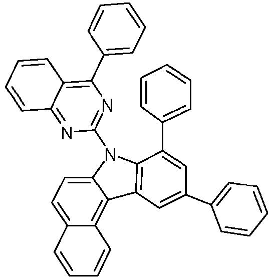 | 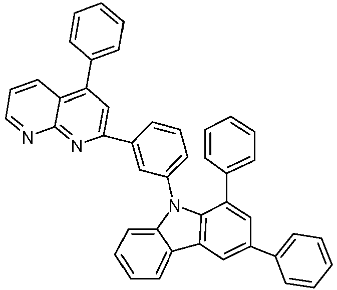 |
| (151)* | (152) |
| 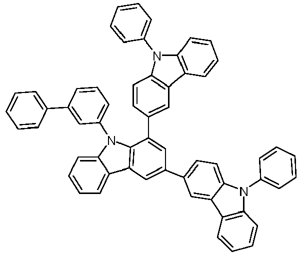 | 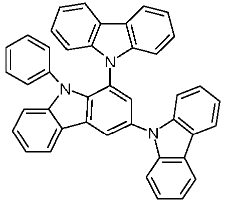 |
| (153)* | (154)* |
| 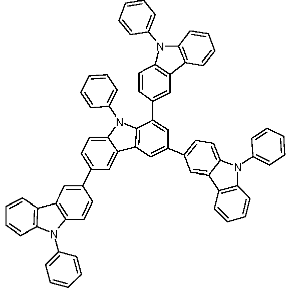 | 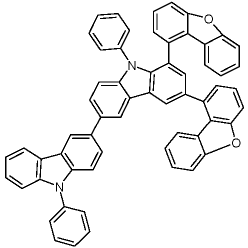 |
| (155)* | (156)* |
| 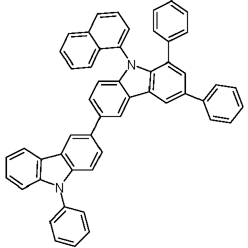 | 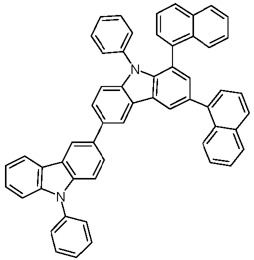 |
| (157)* | (158)* |
| 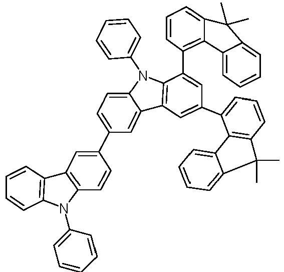 | 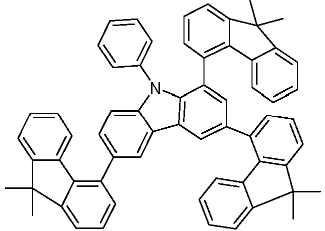 |
| (159)* | (160)* |
| 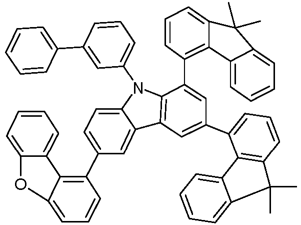 | 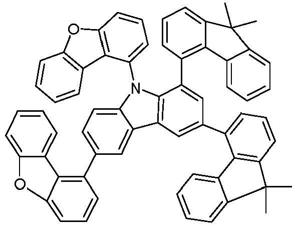 |
| (161)** | (162)* |

| | |
|---|---|
| * nicht erfindungsgemäße Verbindungen | |

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden.

Die Synthese der erfindungsgemäßen Verbindungen geht beispielsweise von einem gegebenenfalls weiter substituierten 1,3,5-Tribrombenzol aus. Statt Brom lassen sich auch andere reaktive Abgangsgruppen, wie zum Beispiel Iod, Chlor oder Triflat, einsetzen. Diese Verbindung wird dann an zwei Positionen mit den entsprechenden Gruppen (L¹)_{q}Ar¹ gekuppelt. Dafür eignen sich insbesondere übergangsmetallkatalysierte Kupplungsreaktionen (z.B. Suzuki-Kupplung oder Stille-Kupplung). Hierfür muss die zu kuppelnde Gruppe auch eine entsprechend geeignete Abgangsgruppe aufweisen, insbesondere Chlor, Brom, Iod, Triflat oder ein Boronsäurederivat, insbesondere Boronsäure bzw. einen Boronsäureester.

In einem nächsten Schritt kann dann das mindestens disubstituierte Benzolderivat durch eine weitere Kupplungsreaktion mit einem weiteren Aromaten umgesetzt werden, welcher in ortho-Position zur Kupplungsposition eine Nitro-Gruppe trägt. Das so erhaltene Nitro-Biphenylderivat kann zum Carbazol-Grundgerüst zyklisiert werden. In weiteren Schritten können durch weitere Kupplungsreaktionen die Gruppen am N-Atom des Carbazols (z.B. Buchwald-Kupplung oder Ullmann-Kupplung) eingeführt werden. Zusätzlich können noch weitere Gruppen durch selektive Bromierung und anschließender Kupplung eingeführt werden.

Ein weiterer Gegenstand der vorliegenden Erfindung ist daher ein Verfahren zur Herstellung einer Verbindung gemäß Formel (4), wobei die Verbindung der Formel (4) durch eine oder mehrere Kupplungsreaktionen und/oder Zyklisierungen aufgebaut wird.

Die oben gezeigten Syntheseverfahren haben exemplarischen Charakter und können vom Fachmann auf dem Gebiet der organischen Synthese in geeigneter Weise abgewandelt werden, wenn dies für die Synthese bestimmter Ausführungsformen von erfindungsgemäßen Verbindungen vorteilhaft ist.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. CC-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstände der Erfindung sind daher Oligomere, Polymere oder Dendrimere enthaltend eine oder mehrere Verbindungen gemäß Formel (4), wobei die Bindung(en) zum Polymer, Oligomer oder Dendrimer an beliebigen freien Positionen in Formel (4) lokalisiert sein können. Je nach Verknüpfung der erfindungsgemäßen Verbindung ist die Verbindung Bestandteil einer Seitenkette des Oligomers oder Polymers oder Bestandteil der Hauptkette.

Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist.

Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein.

In den linear verknüpften Strukturen können die Einheiten gemäß Formel (4) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein.

In verzweigten und dendritischen Strukturen können beispielsweise 3, 5 oder mehrere Einheiten gemäß Formel (4) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (4) in Oligomeren, Dendrimeren und Polymeren gelten dieselben Bevorzugungen wie oben für die erfindungsgemäßen Verbindungen beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder
WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (4) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation
(B) YAMAMOTO-Polymerisation
(C) STILLE-Polymerisation und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können durch dem Fachmann bekannte Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1 995), 26(1-3), 127-36; Janssen, H. M.; Meijer, E. W., "The synthesis and characterization of dendritic molecules", Materials Science and Technology (1 999), 20 (Synthesis of Polymers), 403-458; Tomalia, Donald A., "Dendrimer molecules", Scientific American (1995), 272(5), 62-6;
WO 2002/067343 A1 und WO 2005/026144 A1 .

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion enthaltend mindestens eine Verbindung gemäß Formel (4) oder mindestens ein Polymer, Oligomer oder Dendrimer enthaltend mindestens eine Einheit gemäß Formel (4) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Ein weiterer Gegenstand der vorliegenden Erfindung sind Mischungen enthaltend mindestens eine erfindungsgemäße Verbindung und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird, insbesondere ein phosphoreszierender Dotand. Geeignete Dotanden sind unten im Zusammenhang mit den organischen Elektrolumineszenzvorrichtungen aufgeführt und sind auch für die erfindungsgemäßen Mischungen bevorzugt.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, sodass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (4) oder gemäß den bevorzugten Ausführungsformen als Matrixmaterial für fluoreszierende oder phosphoreszierende Emitter, insbesondere für phosphoreszierende Emitter, und/oder in einer Elektronentransportschicht und/oder in einer elektronenblockierenden bzw. exzitonenblockierenden Schicht und/oder in einer Lochtransportschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (4) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Eine weitere bevorzugte Ausführungsform der vorliegenden Erfindung ist der Einsatz der Verbindung gemäß Formel (4) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (4) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710,
EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder gemäß der nicht offen gelegten Anmeldung EP 11003232.3, Triphenylenderivaten, z. B. gemäß WO 2012/048781, oder Lactame, z. B. gemäß WO 2011/116865 oder WO 2011/137951. Ebenso kann ein weiterer phosphoreszierender Emitter, welcher kürzerwellig als der eigentliche Emitter emittiert, als Co-Host in der Mischung vorhanden sein.

Die Mischung aus der Verbindung gemäß Formel (4) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (4) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Vom Begriff phosphoreszierende Dotanden (Emitter) sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

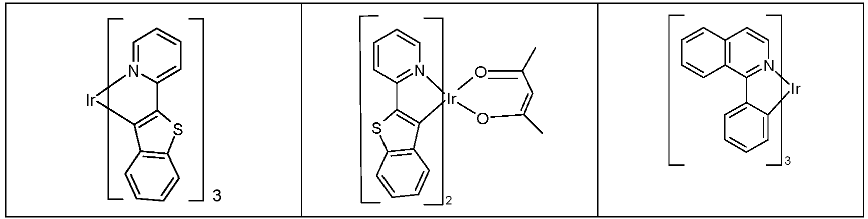

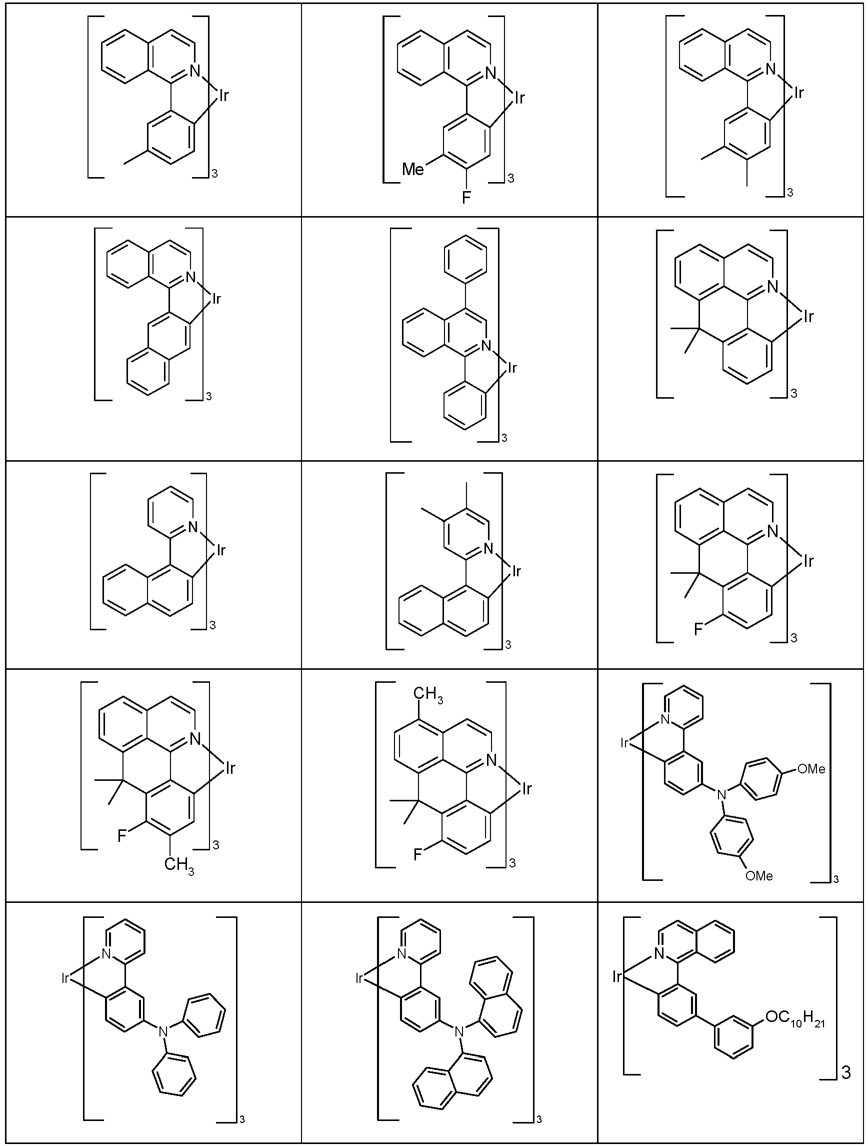

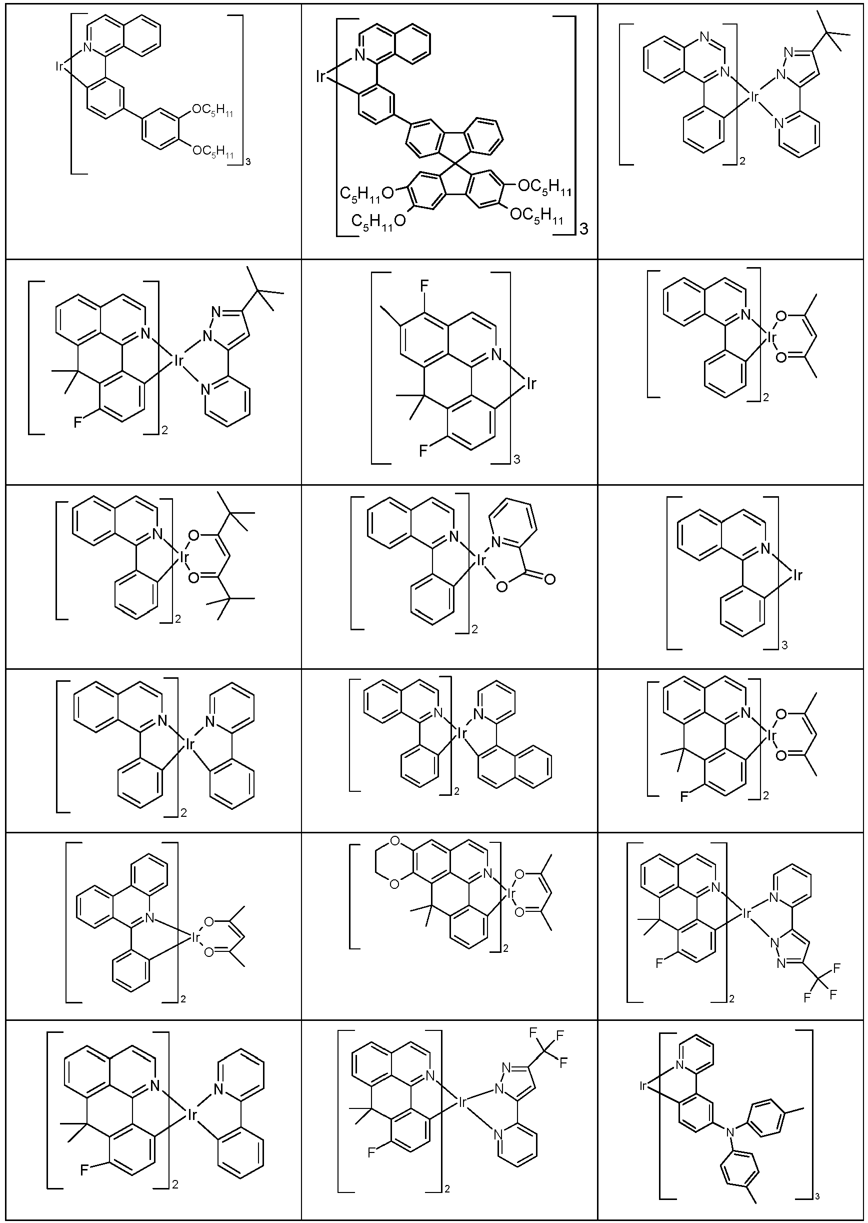

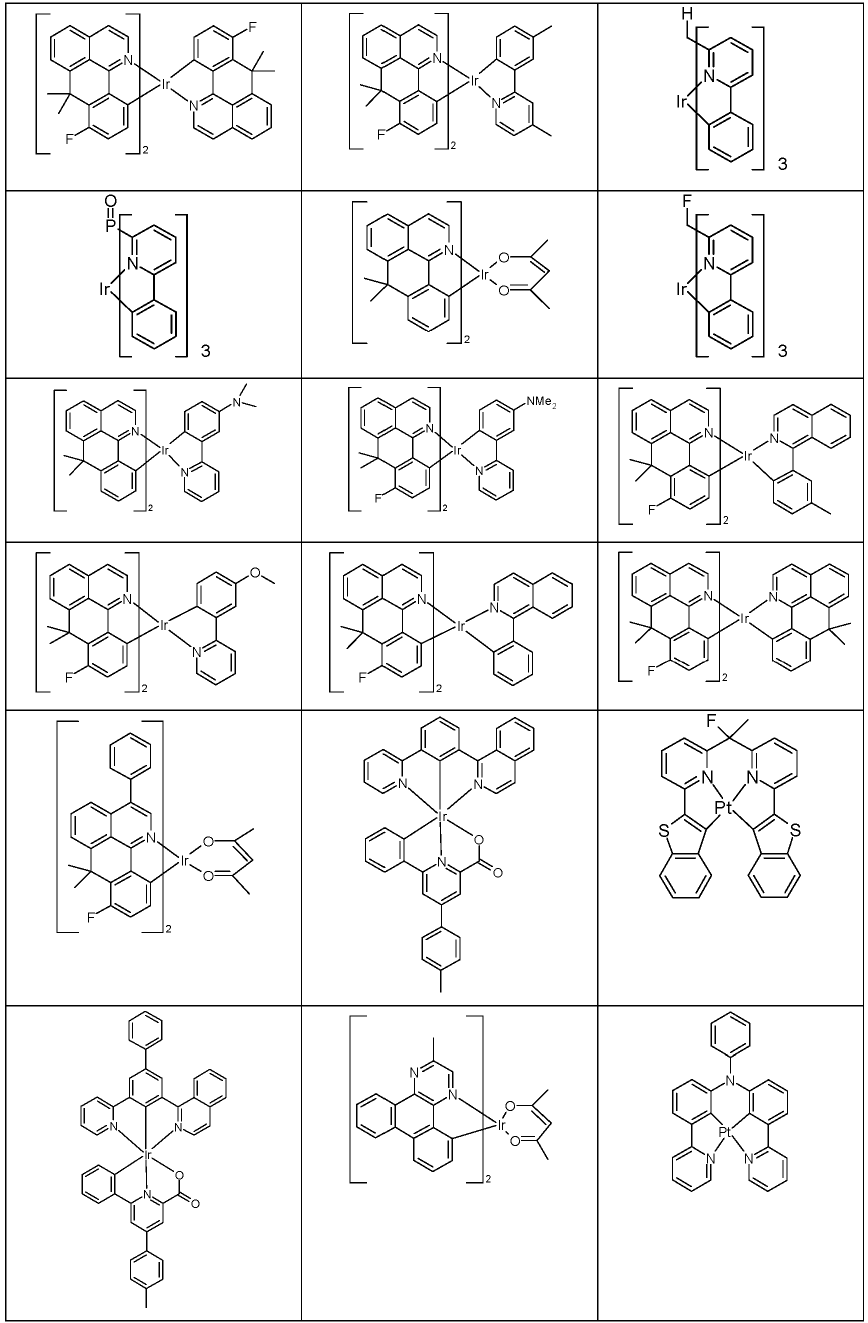

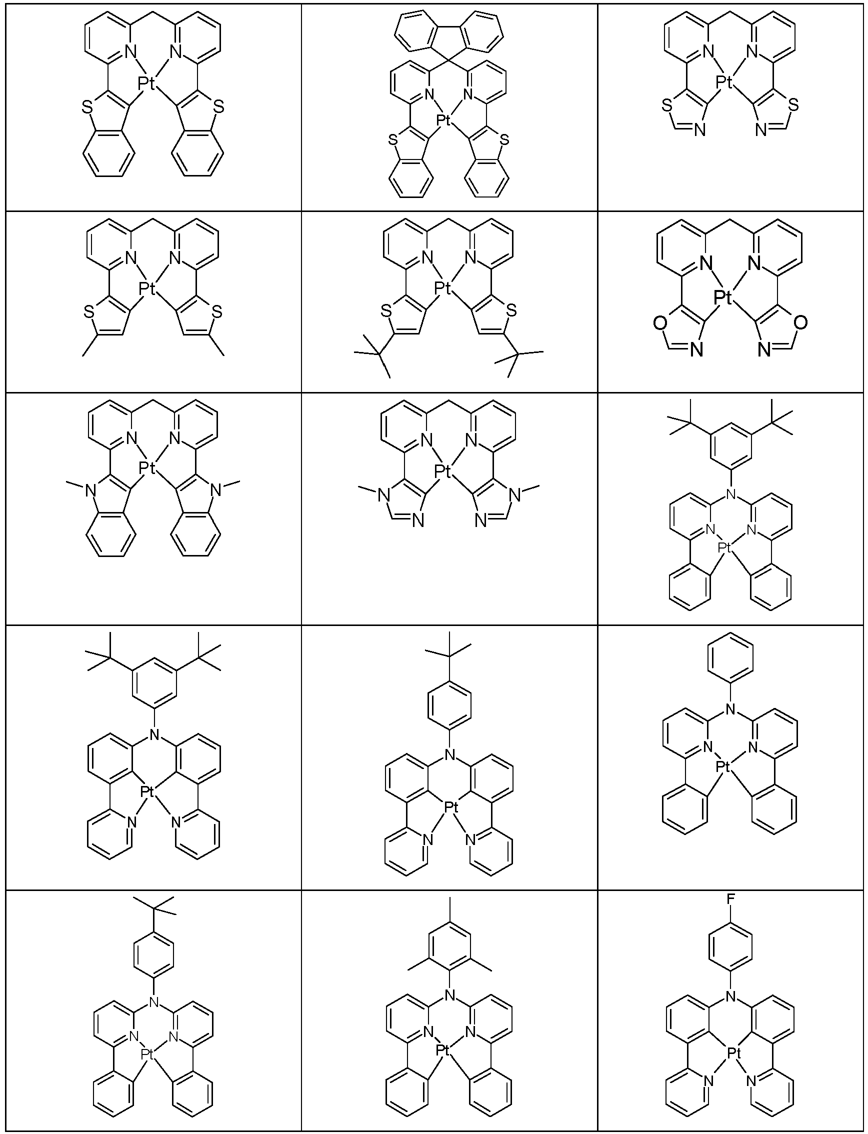

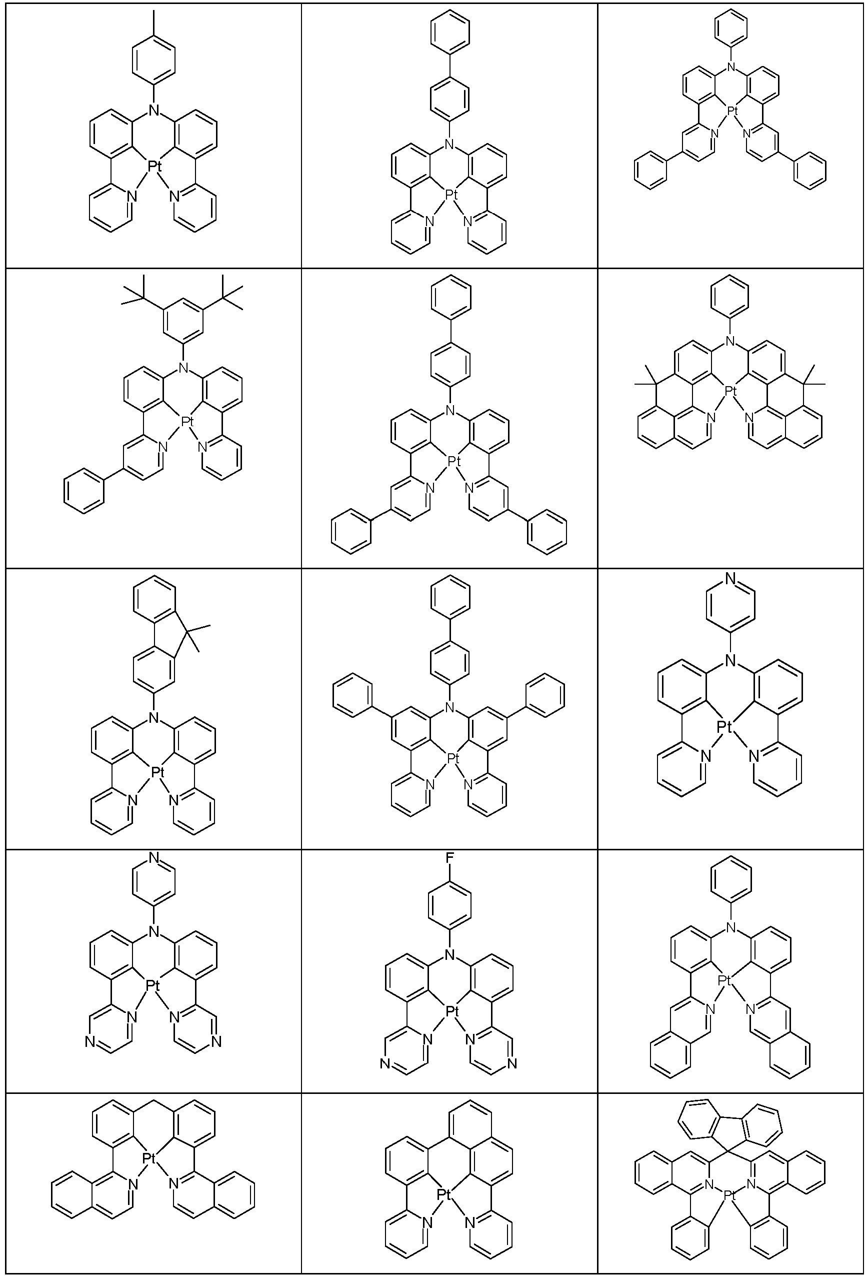

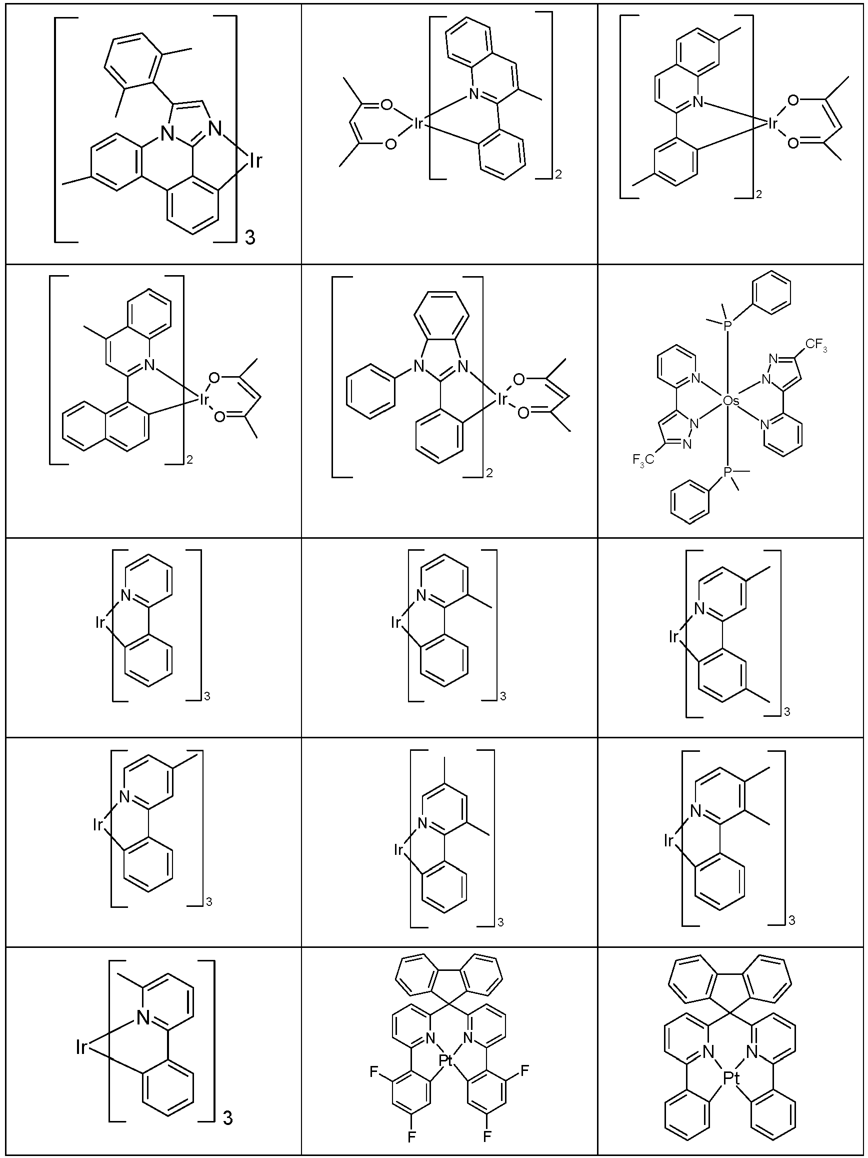

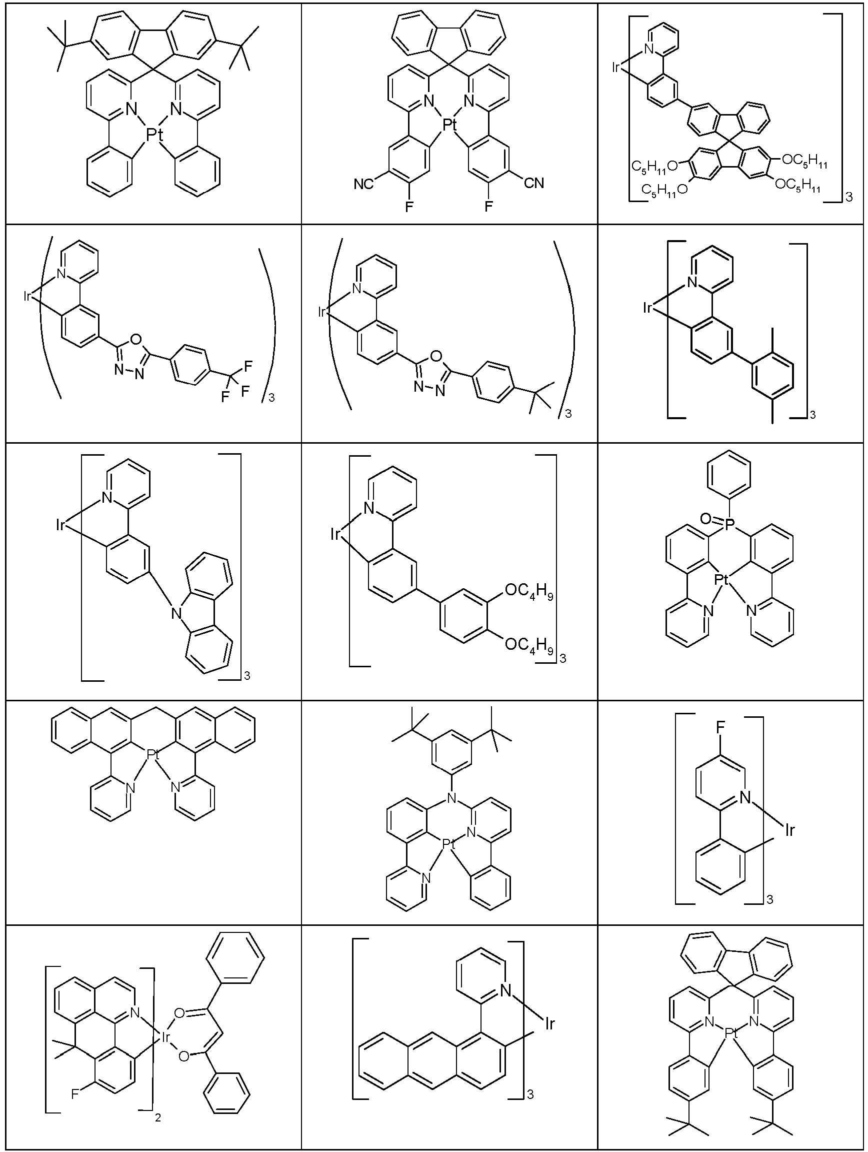

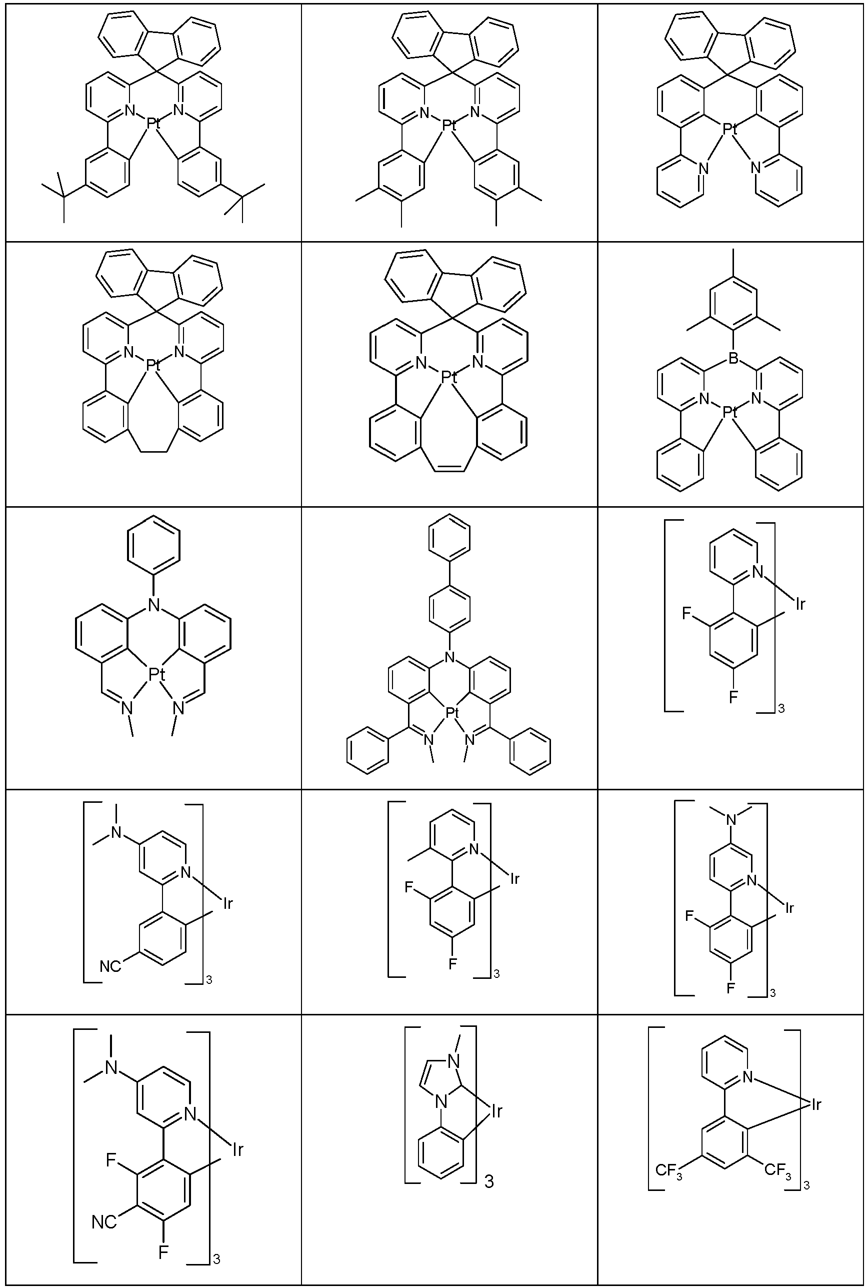

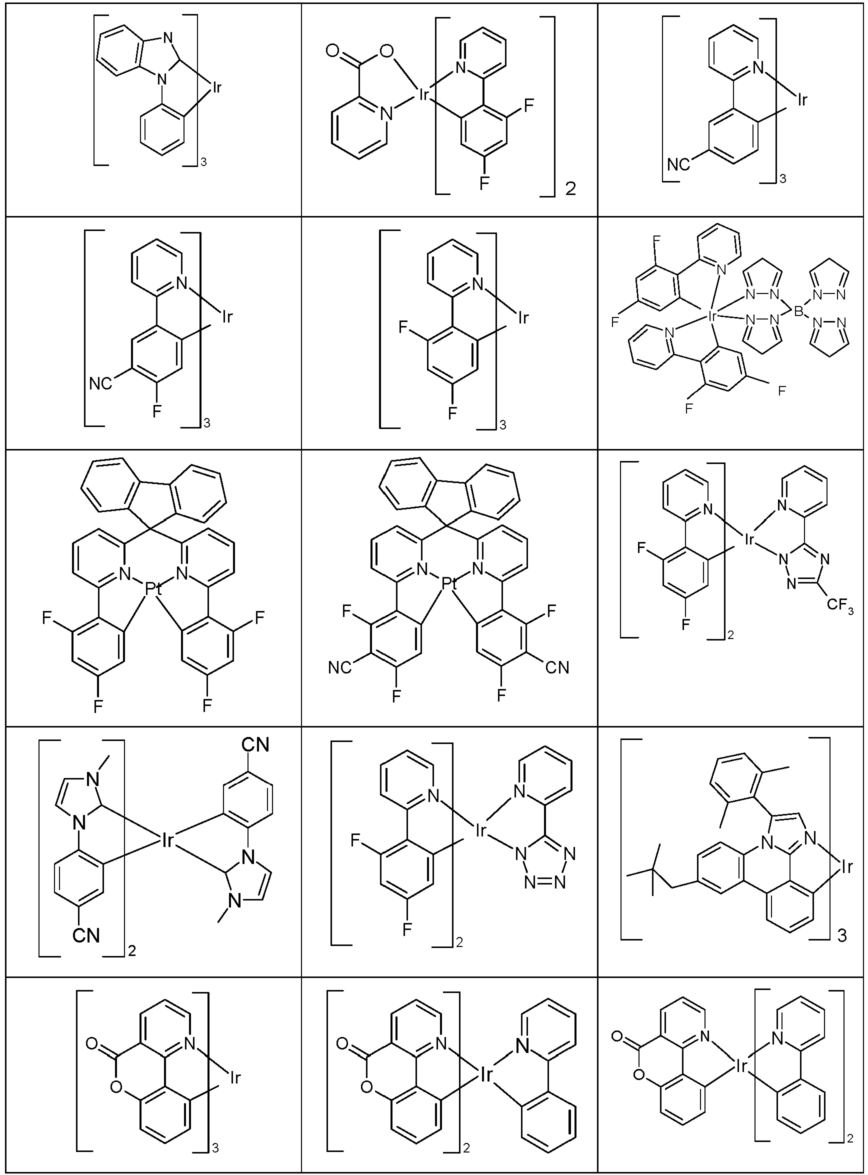

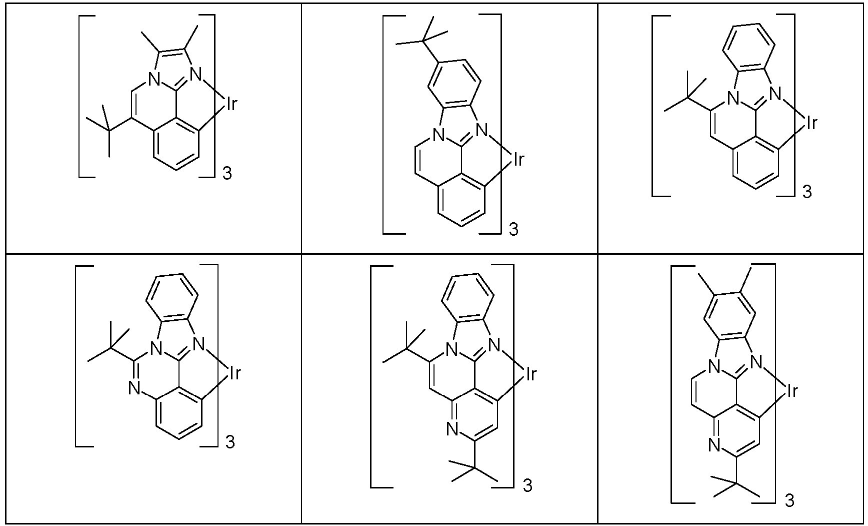

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochtransportschicht oder in einer Lochinjektionsschicht oder in einer Exzitonen- bzw. Elektronenblockierschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination

mit den erfindungsgemäßen Verbindungen gemäß Formel (4) bzw. gemäß den bevorzugten Ausführungsformen verwenden.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen einen oder mehrere der folgenden überraschenden Vorteile gegenüber dem Stand der Technik auf:
1. Die Leistungseffizienz entsprechender Vorrichtungen wird höher im Vergleich zu Systemen gemäß dem Stand der Technik.
2. Die Stabilität entsprechender Vorrichtungen wird höher im Vergleich zu Systemen gemäß dem Stand der Technik, was sich vor allem in einer deutlich höheren Lebensdauer zeigt.
3. Die erfindungsgemäßen organische Elektrolumineszenzvorrichtungen weisen eine verringerte Betriebsspannung auf.
4. Wenn die erfindungsgemäßen Verbindungen als Matrixmaterial für phosphoreszierende Emitter verwendet werden, ist es möglich, mit nur einer geringen Emitterkonzentration im Bereich von weniger als 10 Vol.% bereits sehr gute Ergebnisse zu erzielen.
5. Die erfindungsgemäßen Verbindungen weisen eine sehr gute thermische Stabilität auf.

Die Erfindung wird nun durch die nachfolgenden Beispiele näher erläutert, ohne sie dadurch einschränken zu wollen.

### Ausführungsbeispiele

### A) Synthesebeispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### Beispiel 1: 3,3-(5-chlor-1,3-phenylen)bis[9-phenyl-9H-carbazol]

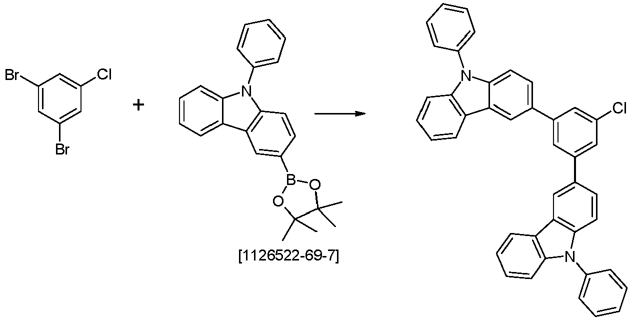

50 g (185 mmol) 1,3-Dibrom-5-chlor-benzen, 143 g (388 mmol) Phenylboronsäure und 78 g (369 mmol) Kaliumphosphat werden in 250 ml Wasser und 500 ml Dioxan suspendiert. Zu dieser Suspension werden 830 mg (3,6 mmol) Pd(OAc)₂ und 3,3 g (11 mmol) P(o-Tol)₃ gegeben, die Reaktionsmischung wird 16 h unter Rückfluss erhitzt. Das Gemisch wird im Anschluss zwischen Essigester und Wasser verteilt, die organische Phase dreimal mit Wasser gewaschen und über Na₂SO₄ getrocknet und einrotiert. Der verbleibende Rückstand wird aus Heptan/Toluol umkristallisiert. Die Ausbeute beträgt 104 g (175 mmol, 95%).

Analog können folgende Verbindungen erhalten werden:

| | **Edukt 1** | **Edukt 2** | **Produkt** | **Ausbeute** |
|---|---|---|---|---|
| 1a | 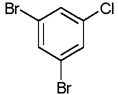 | 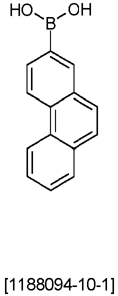 | 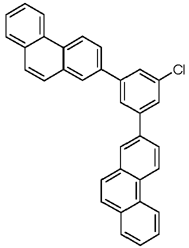 | 89% |
| 1b | 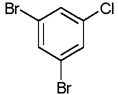 | 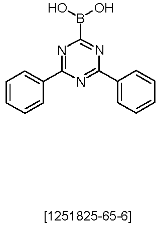 | 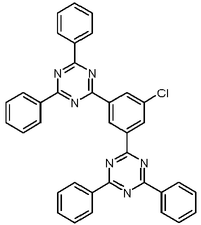 | 88% |
| 1c | 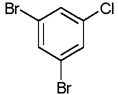 | 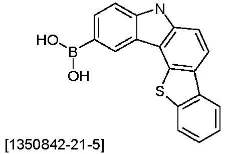 | 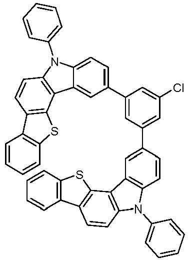 | 75% |
| 1e | 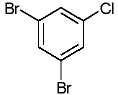 | 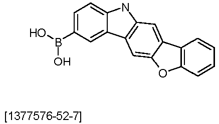 | 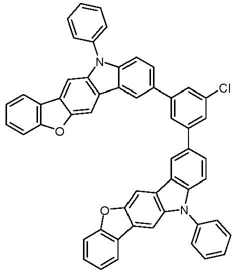 | 78% |
| 1f | 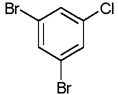 | 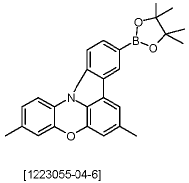 | 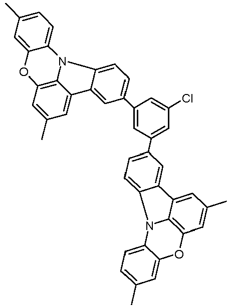 | 69% |
| 1g | 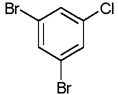 | 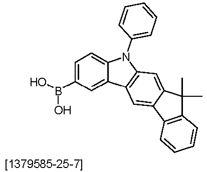 | 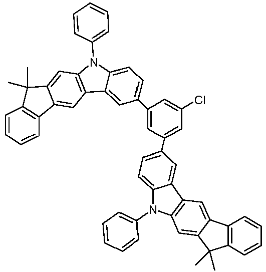 | 89% |
| 1h | 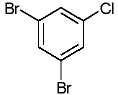 | 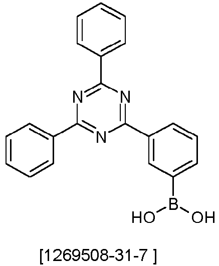 | 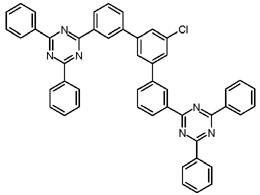 | 87% |
| 1i | 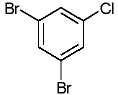 | 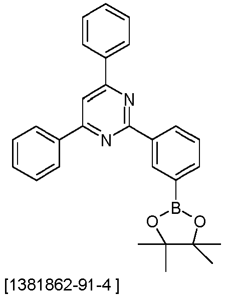 | 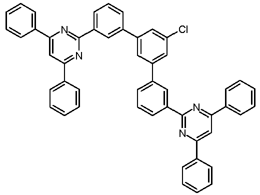 | 82% |
| 1j | 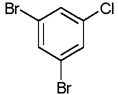 | 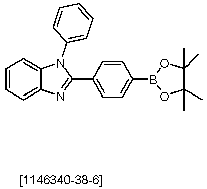 | 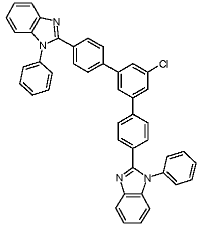 | 87% |
| 1k | 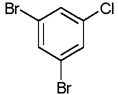 | 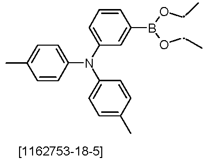 | 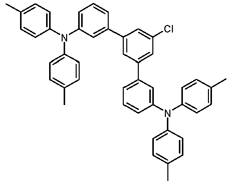 | 88% |
| 1l | 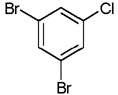 | 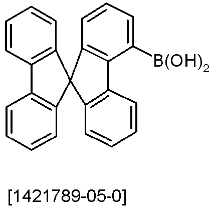 | 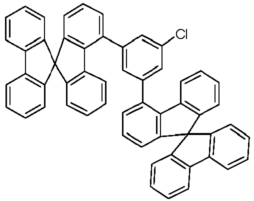 | 85% |
| 1i | 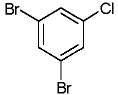 | 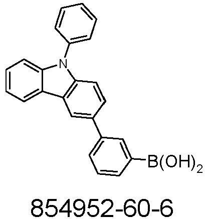 | 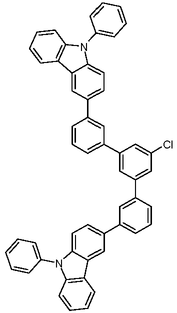 | 78% |
| 1j | 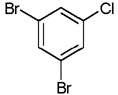 | 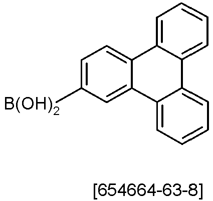 | 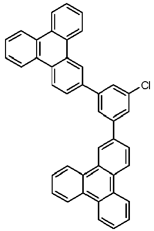 | 77% |

### Beispiel 2: 3,3-[5(4,4,5,5-tetramethyl-[1,3,2]dioxaborolanyl-2y1)-1,3-phenylen]bis-[9-phenyl-9H-carbazol]

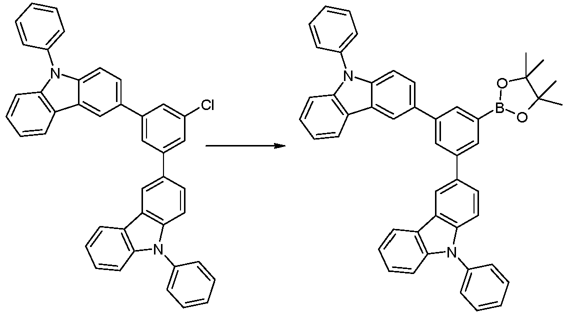

75 g (126 mmol) 3,3-(5-chlor-1,3-phenylen)bis[9-phenyl-9H-carbazol], 41 g (163 mmol) Bis-(pinacolato]-diboron und 18 g (25 mmol) Palladiumdichlorid-tricyclohexylphosphin und 21g (214 mmol) Kaliumacetat werden in 1200 ml Dioxan suspendiert. Die Reaktionsmischung wird 16 h unter Rückfluss bei 130 °C erhitzt. Das Gemisch wird im Anschluss zwischen Essigester und Wasser verteilt, die organische Phase dreimal mit Wasser gewaschen und über Na₂SO₄ getrocknet und einrotiert. Der verbleibende Rückstand wird aus Heptan/Toluol umkristallisiert. Die Ausbeute beträgt 76 g (110 mmol, 88%).

Analog können folgende Verbindungen erhalten werden:

| | **Edukt 1** | **Produkt** | **Ausbeute** |
|---|---|---|---|
| 2a | 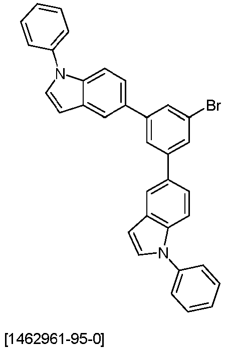 | 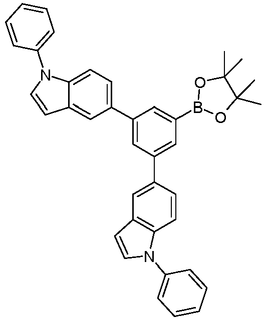 | 77% |
| 2b | 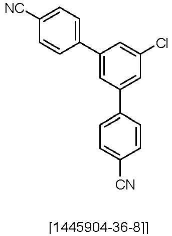 | 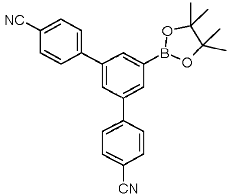 | 78% |
| 2c | 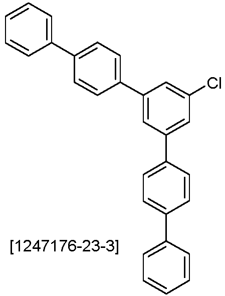 | 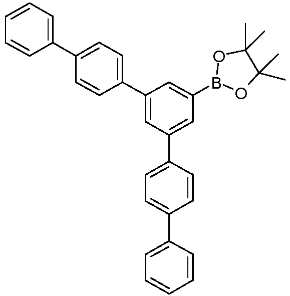 | 65% |
| 2d | 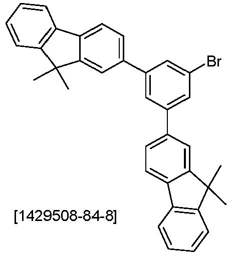 | 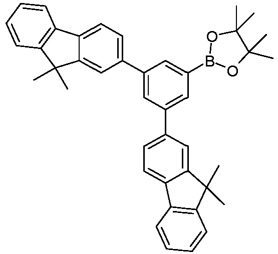 | 82% |
| 2e | 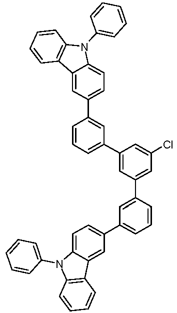 | 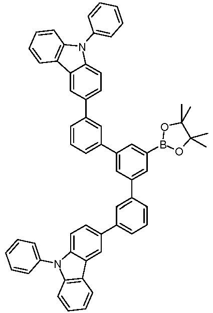 | 86% |
| 2f | 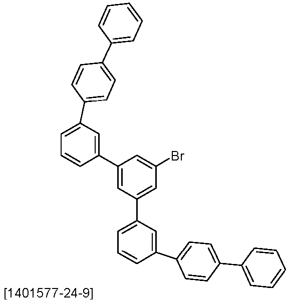 | 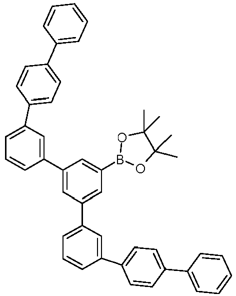 | 90% |
| 2h | 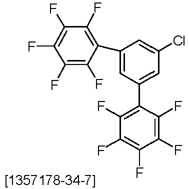 | 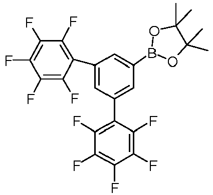 | 93% |
| 2i | 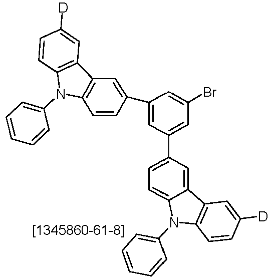 | 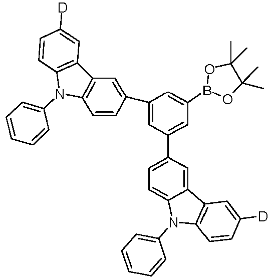 | 81% |
| 2j | 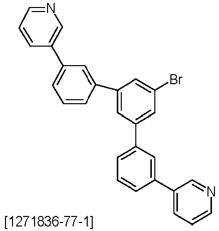 | 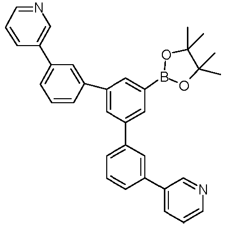 | 90% |
| 2k | 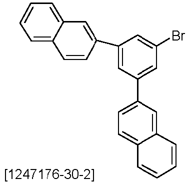 | 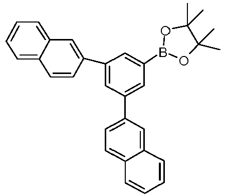 | 96% |
| 2l | 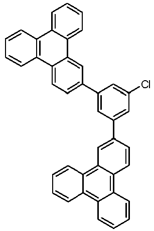 | 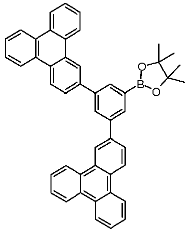 | 87% |
| 2m | 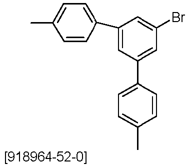 | 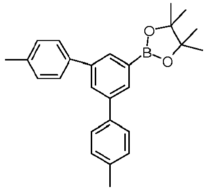 | 95% |
| 2n | 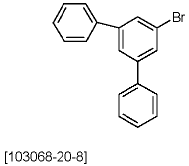 | 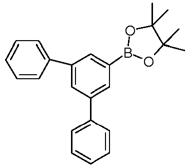 | 97% |
| 2o | 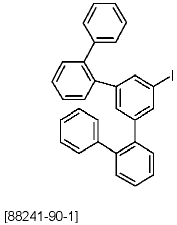 | 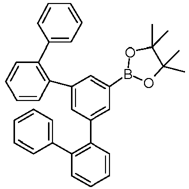 | 86% |
| 2p | 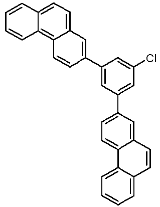 | 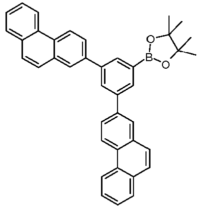 | 88% |
| 2q | 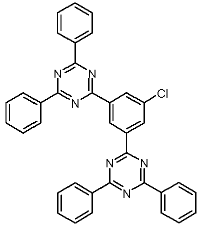 | 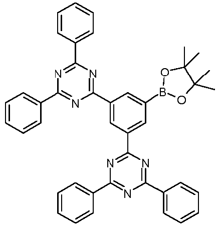 | 73% |
| 2r | 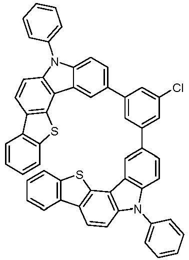 | 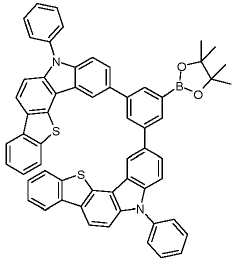 | 70% |
| 2s | 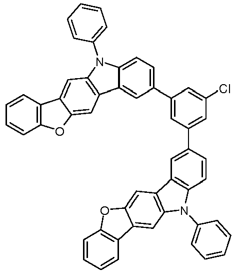 | 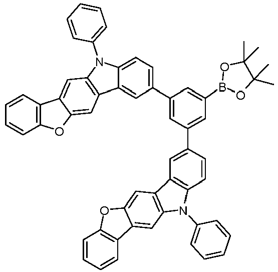 | 79% |
| 2t | 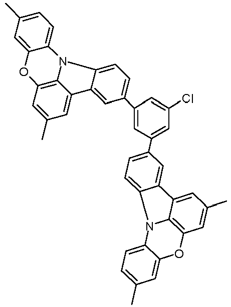 | 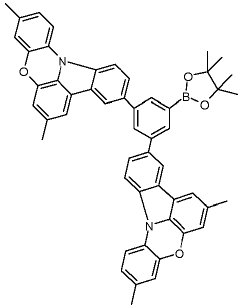 | 75% |
| 2u | 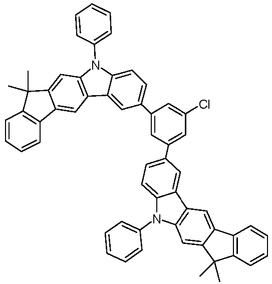 | 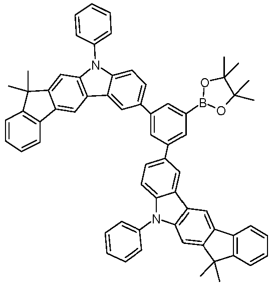 | 78% |
| 2v | 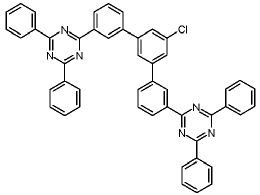 | 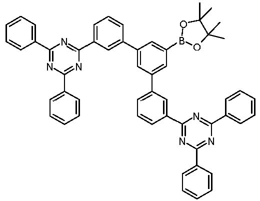 | 69% |
| 2w | 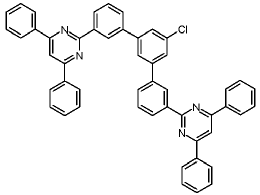 | 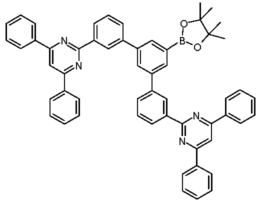 | 74% |
| 2x | 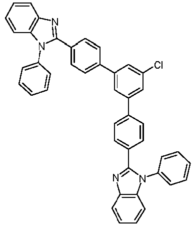 | 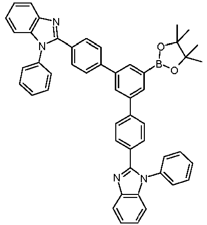 | 86% |
| 2z | 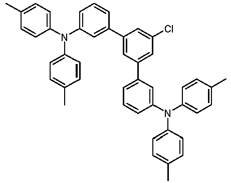 | 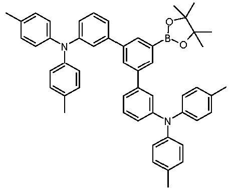 | 89% |
| 2aa | 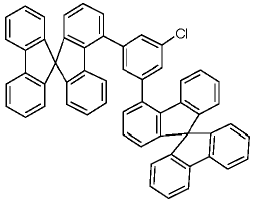 | 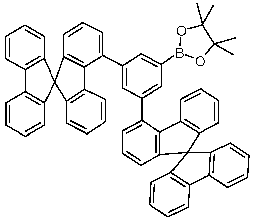 | 76% |

### Beispiel 3: Umsetzung mit 1-Brom-2-nitrobenzen

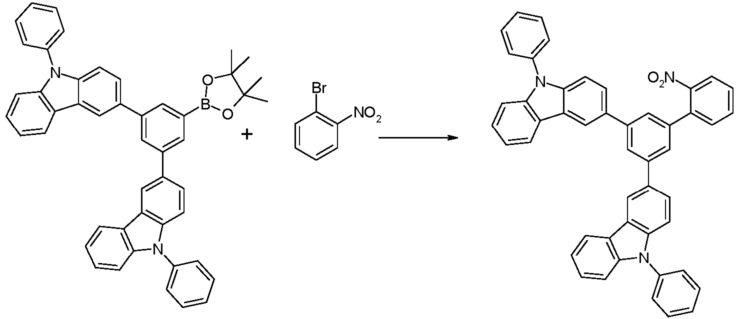

46 g (67 mmol) 3,3-[5(4,4,5,5-tetramethyl-[1,3,2]dioxaborolanyl-2y1)-1,3-phenylen]bis-[9-phenyl-9H-carbazol], 16 g (81 mmol) 1-Brom-2-nitrobenzen und 152 mg (0,67 mmol) Pd(OAc)₂ und 178 mg (0,67 mmol) P(o-Tol)₃ und 136 g (980 mmol) Kaliumcarbonat werden in 1000 ml THF und 300 ml Wasser suspendiert. Die Reaktionsmischung wird 16 h unter Rückfluss bei 130 °C erhitzt. Das Gemisch wird im Anschluss zwischen Essigester und Wasser verteilt, die organische Phase dreimal mit Wasser gewaschen und über Na₂SO₄ getrocknet und einrotiert. Der verbleibende Rückstand wird aus Heptan/Toluol umkristallisiert. Die Ausbeute beträgt 42 g (62 mmol, 91%).

Analog können folgende Verbindungen erhalten werden:

| | **Edukt 1** | **Edukt 2** | **Produkt** | **Ausbeute** |
|---|---|---|---|---|
| 3a | 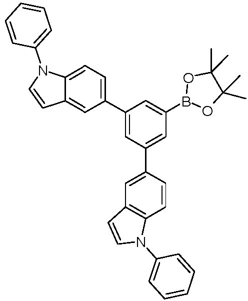 | 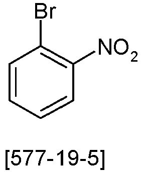 | 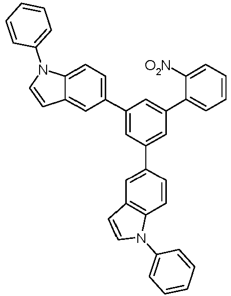 | 90% |
| 3b | 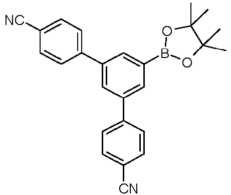 | 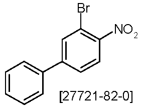 | 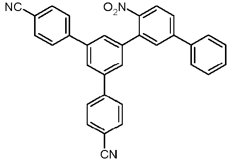 | 92% |
| 3c | 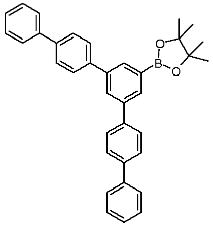 | 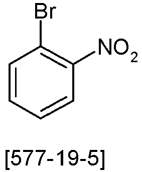 | 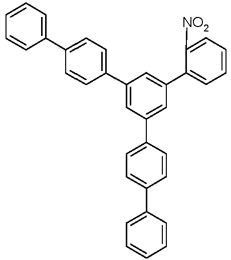 | 97% |
| 3d | 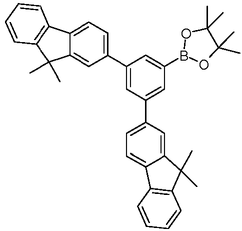 | 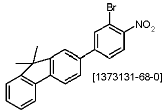 | 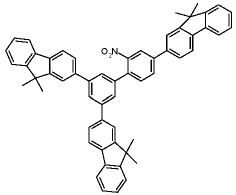 | 91% |
| 3e | 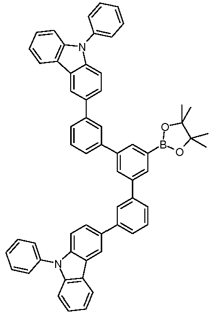 | 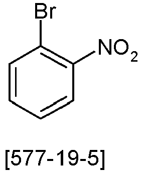 | 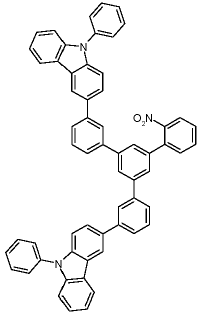 | 93% |
| 3f | 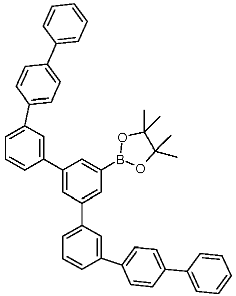 | 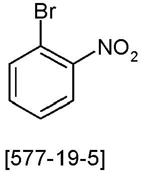 | 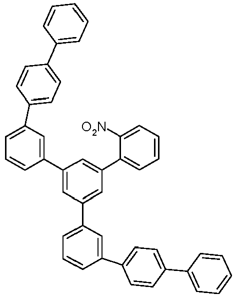 | 94% |
| 3h | 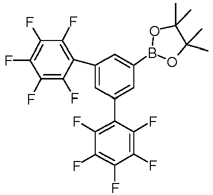 | 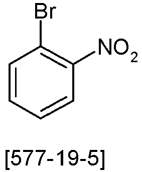 | 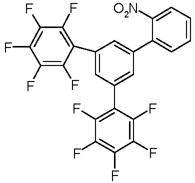 | 91% |
| 3i | 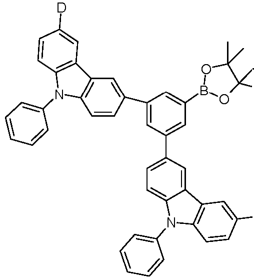 | 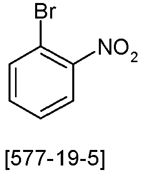 | 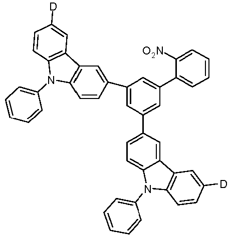 | 96% |
| 3j | 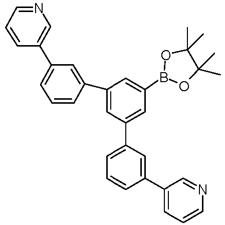 | 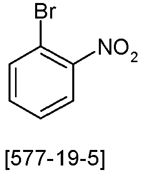 | 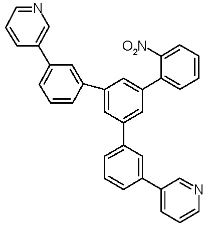 | 95% |
| 3k | 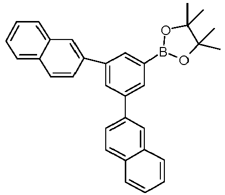 | 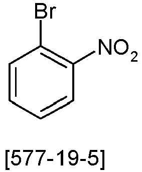 | 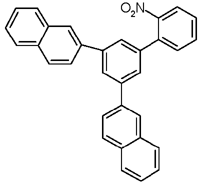 | 93% |
| 31 | 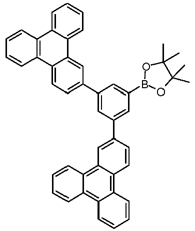 | 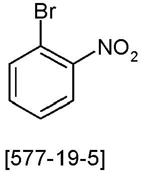 | 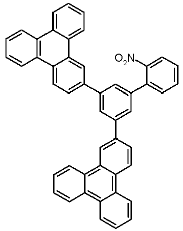 | 92% |
| 3m | 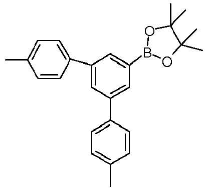 | 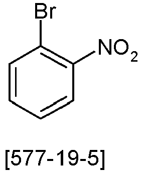 | 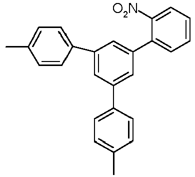 | 95% |
| 3n | 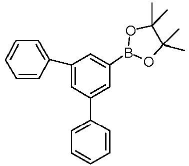 | 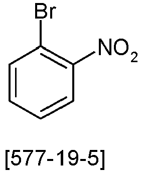 | 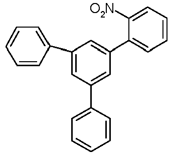 | 98% |
| 3o | 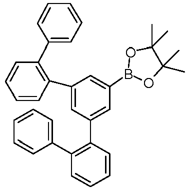 | 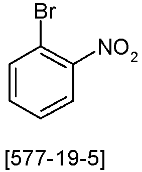 | 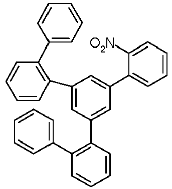 | 90% |
| 3p | 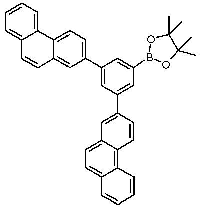 | 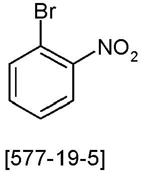 | 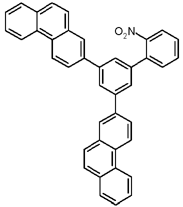 | 93% |
| 3q | 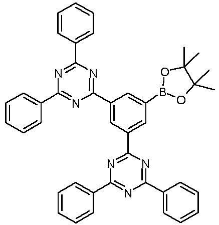 | 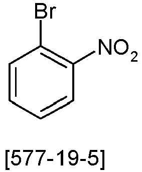 | 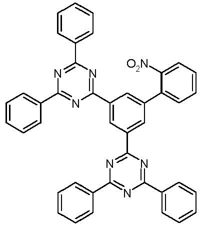 | 87% |
| 3r | 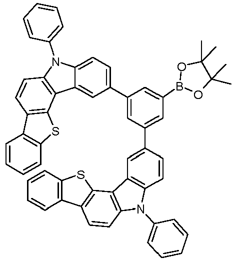 | 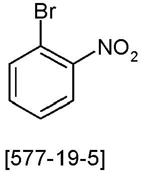 | 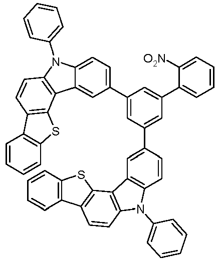 | 86% |
| 3s | 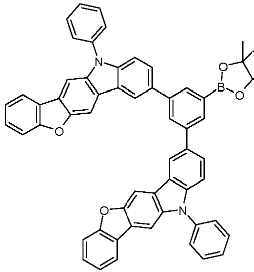 | 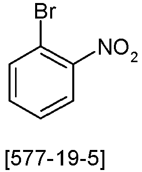 | 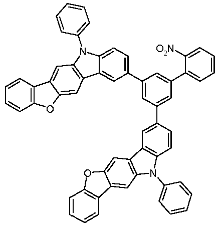 | 89% |
| 3t | 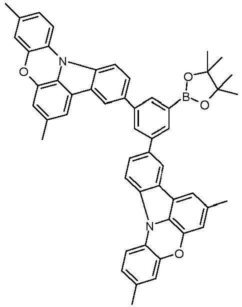 | 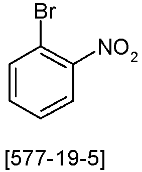 | 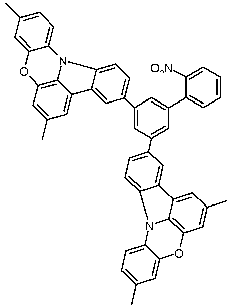 | 78% |
| 3u | 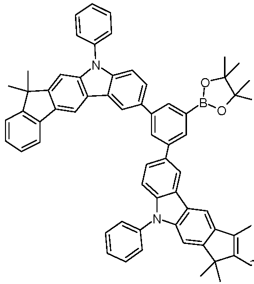 | 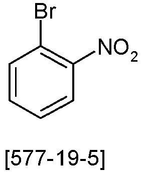 | 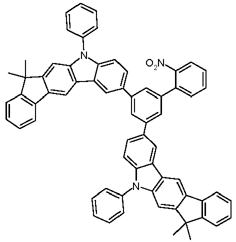 | 91% |
| 3v | 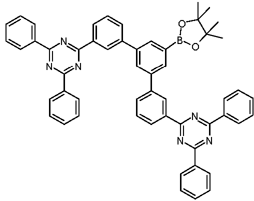 | 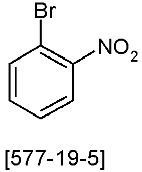 | 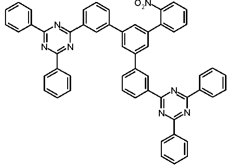 | 76% |
| 3w | 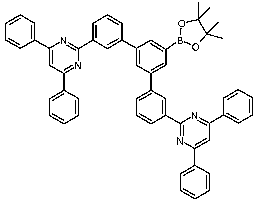 | 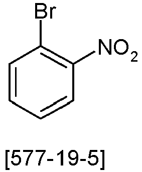 | 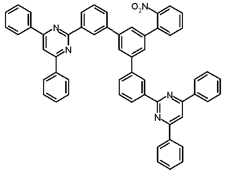 | 77% |
| 3x | 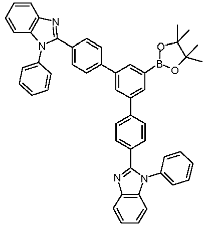 | 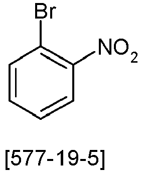 | 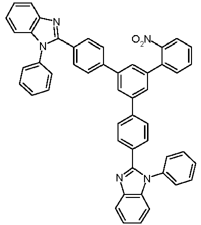 | 89% |
| 3z | 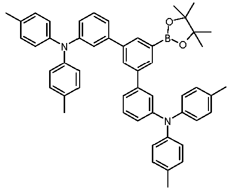 | 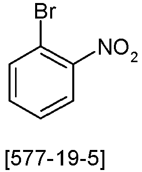 | 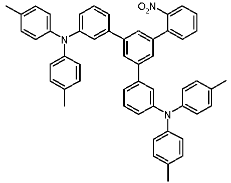 | 94% |
| 3aa | 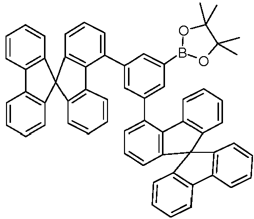 | 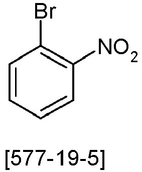 | 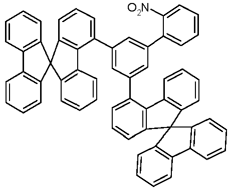 | 78% |
| 3ab | 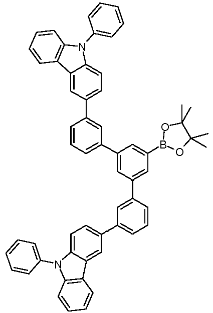 | 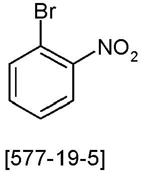 | 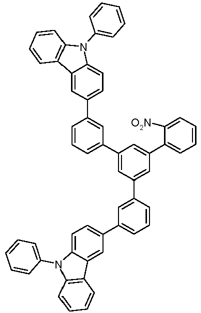 | 87% |

### Beispiel 4: 9,9"-Diphenyl-9H,9'H,9"H-[3,1';3',3"]tercarbazol

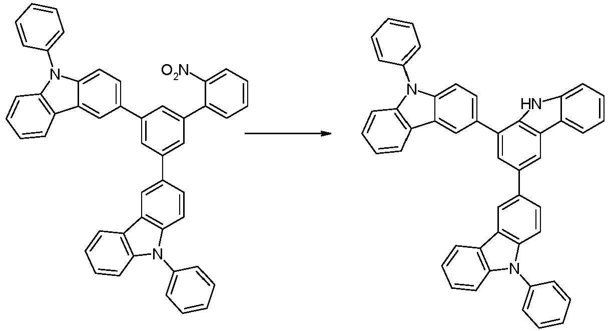

Eine Mischung aus 40 g (59 mmol) des entsprechenden Nitroaromaten und 239 ml (1400 mmol) Triethylphosphit wird 12 h unter Rückfluss auf 130°C erhitzt. Anschließend wird das restliche Triethylphosphit abdestilliert (72-76 °C / 1200 Pa (9 mm Hg)).

Der Rückstand wird mit Wasser/MeOH (1:1) versetzt, der Feststoff abfiltriert und umkristallisiert. Die Ausbeute beträgt 38 g (58 mol, 99%).

Analog können folgende nicht erfindungsgemäße Verbindungen erhalten werden:

| | **Edukt 1** | **Produkt** | **Ausbeute** |
|---|---|---|---|
| 4a | 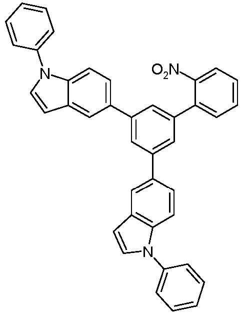 | 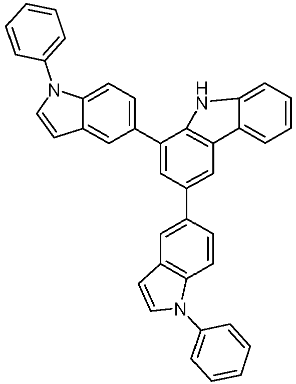 | 89% |
| 4b | 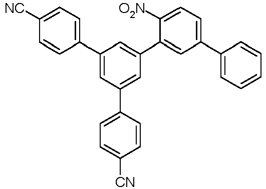 | 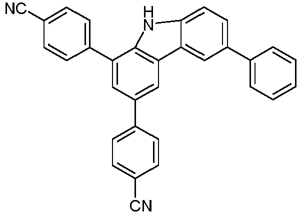 | 90% |
| 4c | 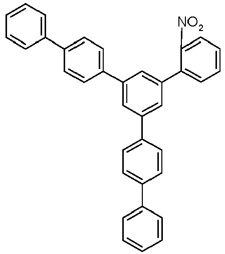 | 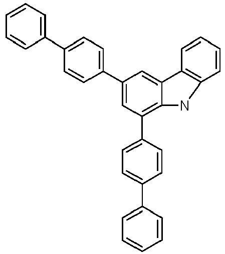 | 91% |
| 4d | 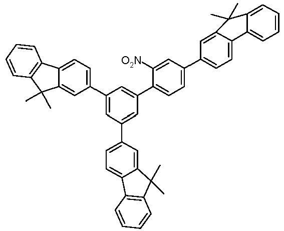 | 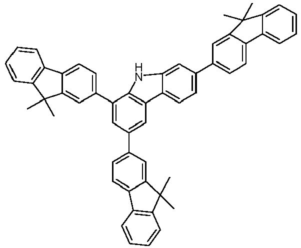 | 96% |
| 4e | 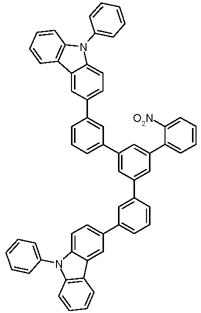 | 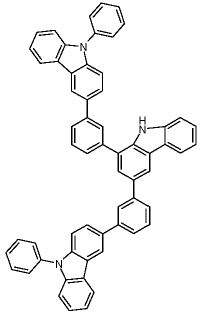 | 94% |
| 4f | 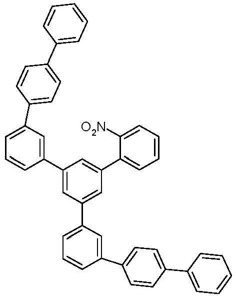 | 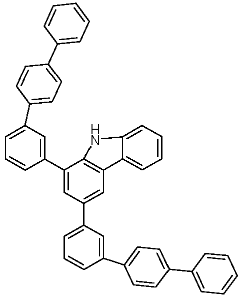 | 97% |
| 4h | 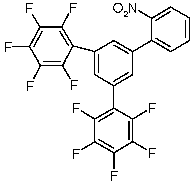 | 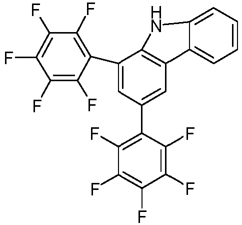 | 93% |
| 4i | 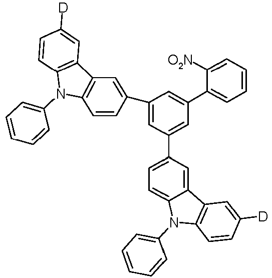 | 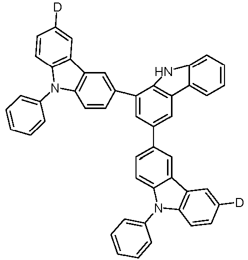 | 95% |
| 4j | 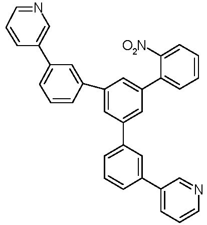 | 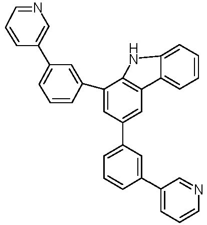 | 99% |
| 4k | 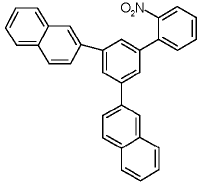 | 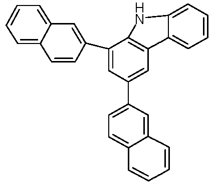 | 93% |
| 4l | 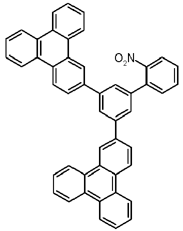 | 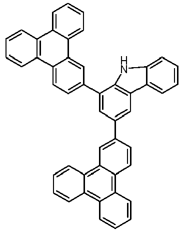 | 92% |
| 4m | 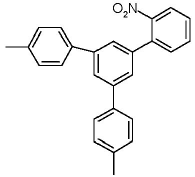 | 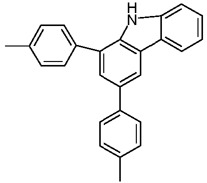 | 93% |
| 4n | 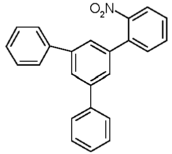 | 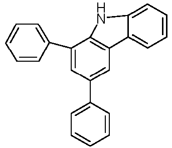 | 97% |
| 4o | 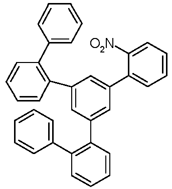 | 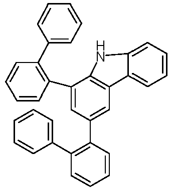 | 91% |
| 4p | 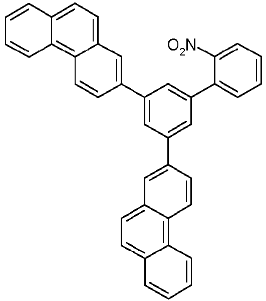 | 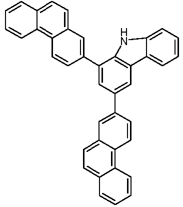 | 87% |
| 4q | 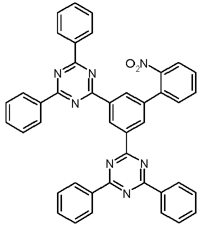 | 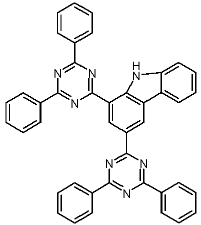 | 69% |
| 4r | 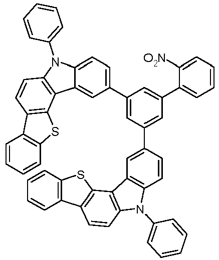 | 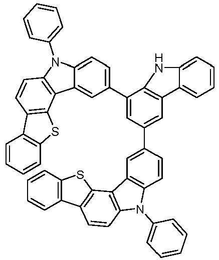 | 86% |
| 4s | 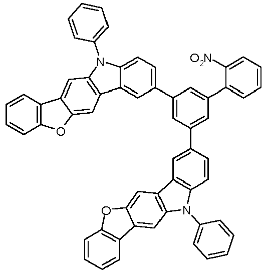 | 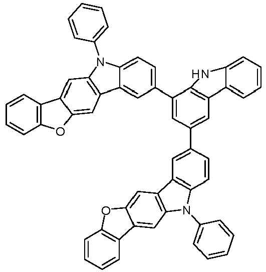 | 83% |
| 4t | 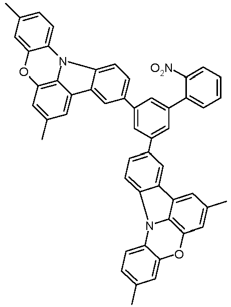 | 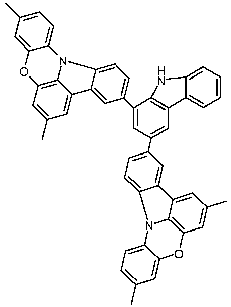 | 80% |
| 4u | 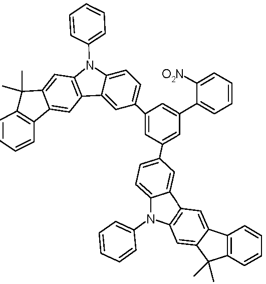 | 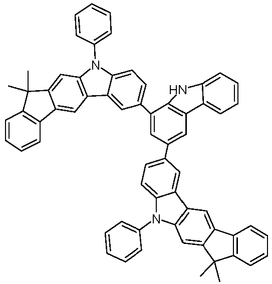 | 90% |
| 4v | 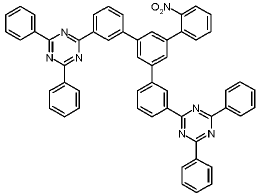 | 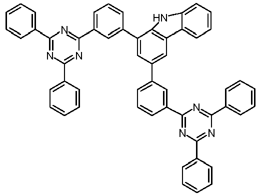 | 77% |
| 4w | 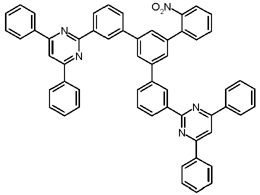 | 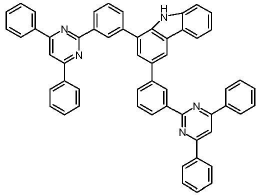 | 75% |
| 4x | 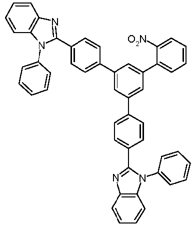 | 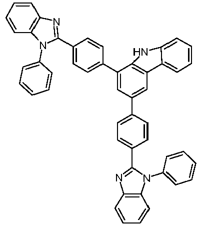 | 83% |
| 4z | 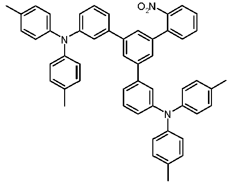 | 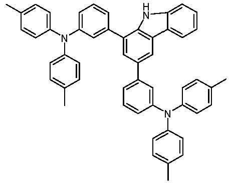 | 69% |
| 4aa | 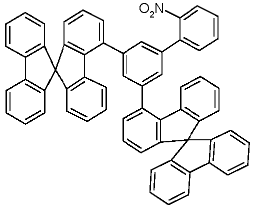 | 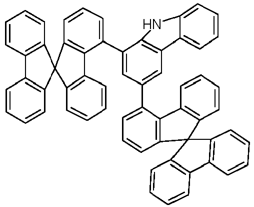 | 85% |

### Beispiel 5: 9,9',9"-Triphenyl-9H,9'H,9"H-[3,1';3',3"]tercarbazole (nicht erfindungsgemäß)

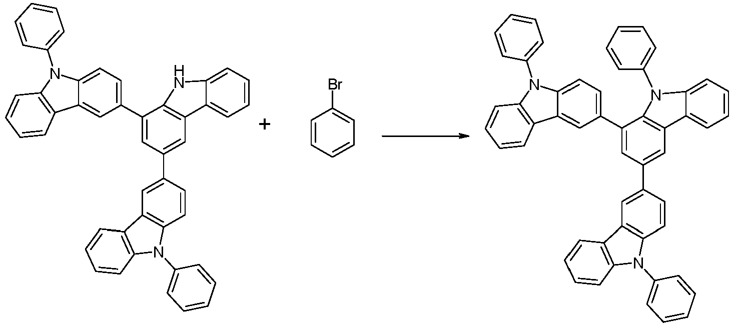

25 g (38 mmol ) 9,9"-Diphenyl-9H,9'H,9"H-[3,1';3',3"]tercarbazol und 7 g (46 mmol) Brombenzol werden in 450 ml Toluol gelöst und mittels Schutzgaseinleitung entgast. Anschließend wird mit 7 ml (7 mmol, 1 M Lösung in Toluol) Tri-tert-butylphosphin, 633,8 mg (2,82 mmol) Pd(OAc)₂ und 7 g (76 mmol) NaOtBu versetzt. Die Feststoffe werden zuvor entgast, die Reaktionsmischung wird nachentgast und anschließend unter Rückfluss für 3 h gerührt. Die warme Reaktionslösung wird über Alox B (Aktivitätsstufe 1) filtriert, mit Wasser gewaschen, getrocknet und eingeengt. Die Ausbeute beträgt 27 g (37 mol, 98%).

Analog können folgende Verbindungen erhalten werden:

| | **Edukt 1** | **Edukt 2** | **Produkt** | **Ausbeute** |
|---|---|---|---|---|
| 5a* | 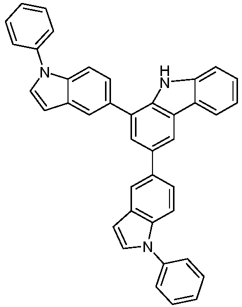 | 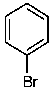 | 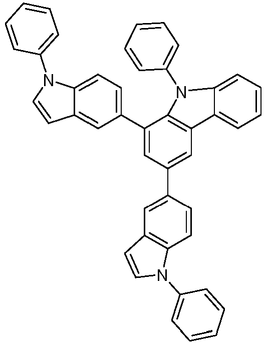 | 89% |
| 5b* | 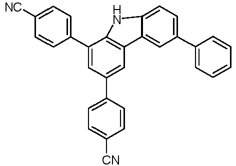 | 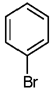 | 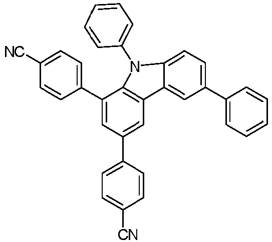 | 87% |
| 5c | 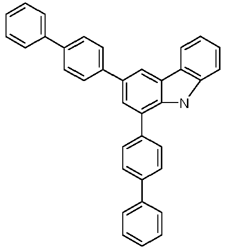 | 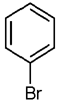 | 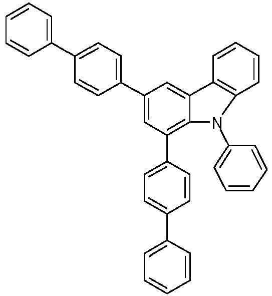 | 91% |
| 5d* | 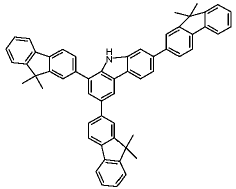 | 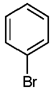 | 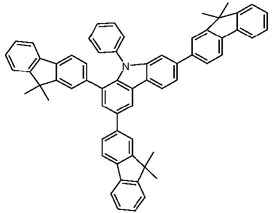 | 90% |
| 5e | 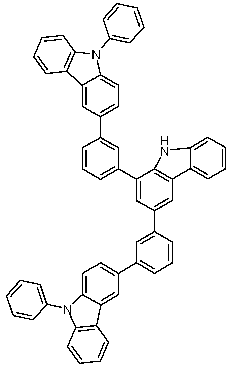 | 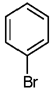 | 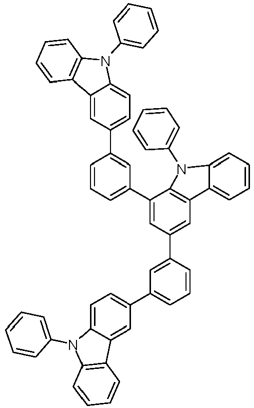 | 79% |
| 5f | 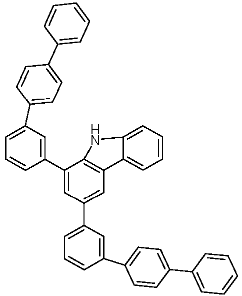 | 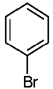 | 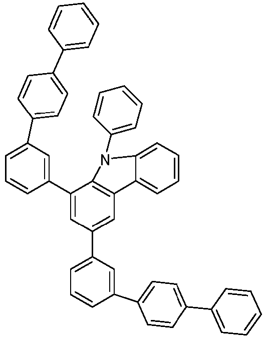 | 78% |
| 5h | 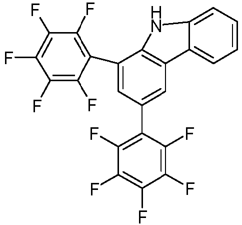 | 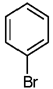 | 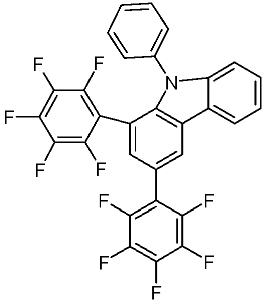 | 92% |
| 5i* | 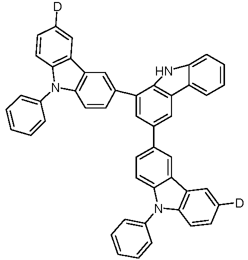 | 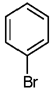 | 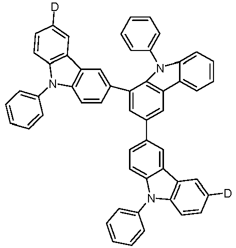 | 91% |
| 5j* | 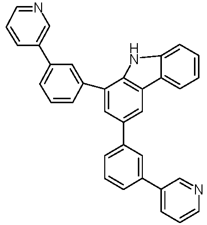 | 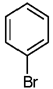 | 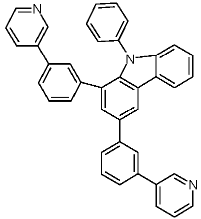 | 95% |
| 5k | 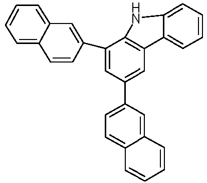 | 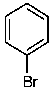 | 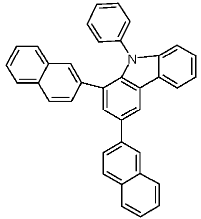 | 96% |
| 5l | 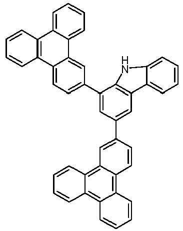 | 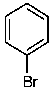 | 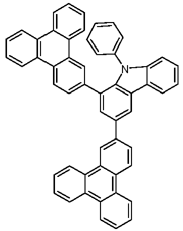 | 93% |
| 5m | 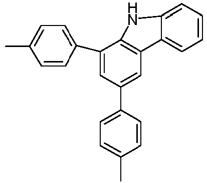 | 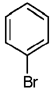 | 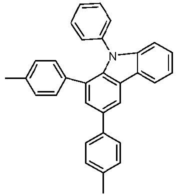 | 94% |
| 5n | 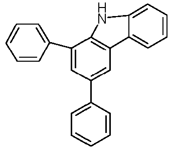 | 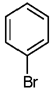 | 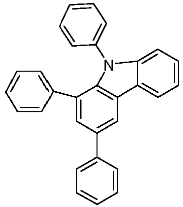 | 97% |
| 5o | 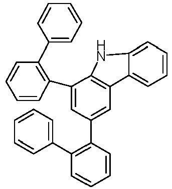 | 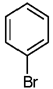 | 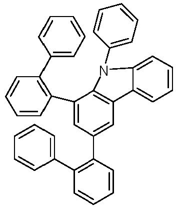 | 85% |
| 5p | 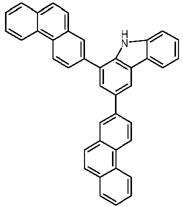 | 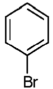 | 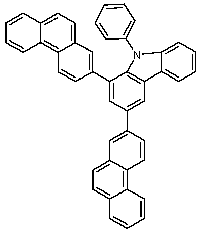 | 89% |
| 5q* | 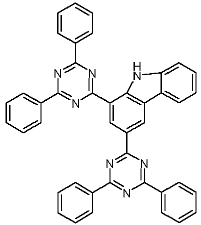 | 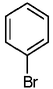 | 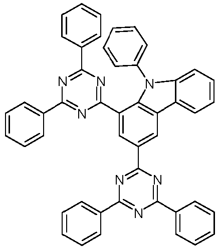 | 87% |
| 5r* | 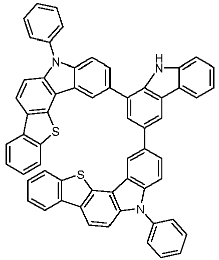 | 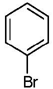 | 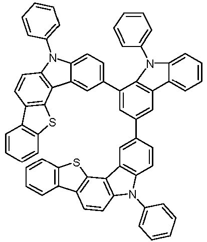 | 76% |
| 5s* | 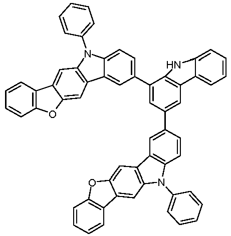 | 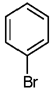 | 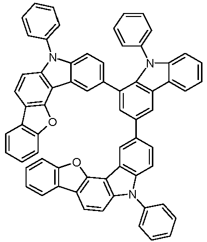 | 79% |
| 5t* | 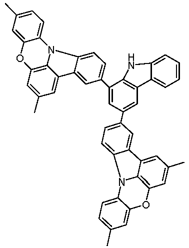 | 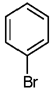 | 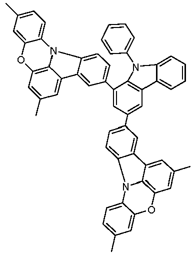 | 73% |
| 5u* | 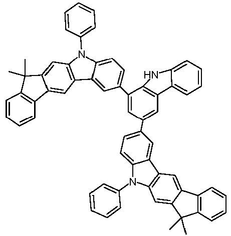 | 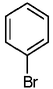 | 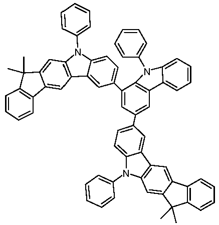 | 88% |
| 5v | 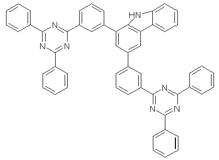 | 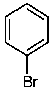 | 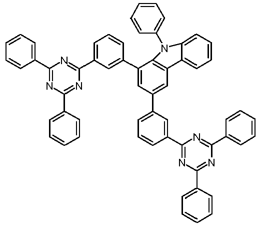 | 87% |
| 5w | 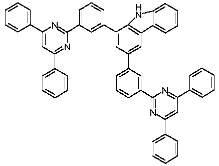 | 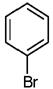 | 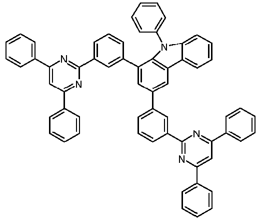 | 83% |
| 5x | 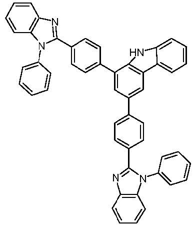 | 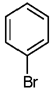 | 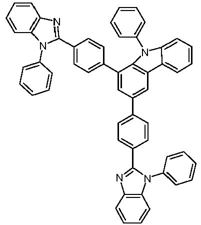 | 87% |
| 5z* | 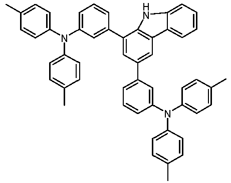 | 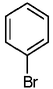 | 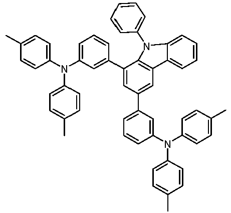 | 94% |
| 5aa | 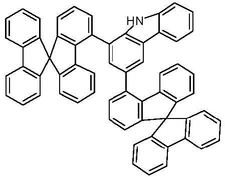 | 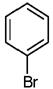 | 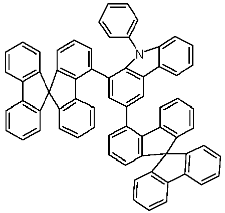 | 86% |
| 5ab * | 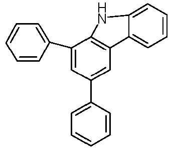 | 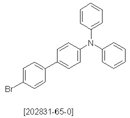 | 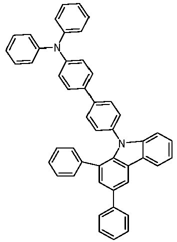 | 87% |
| 5ac | 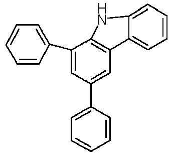 | 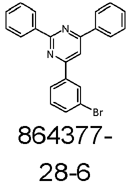 | 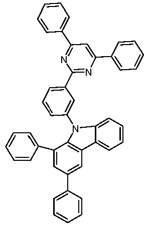 | 95% |
| 5ad | 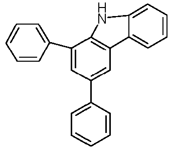 | 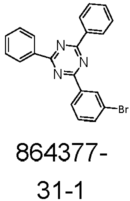 | 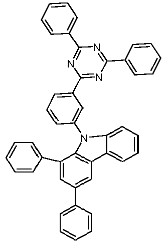 | 92% |
| 5ae | 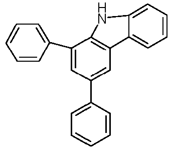 | 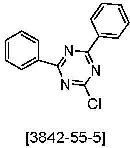 | 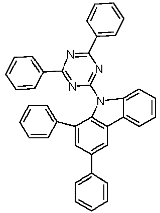 | 90% |
| 5af | 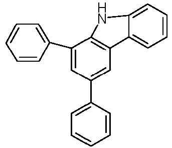 | 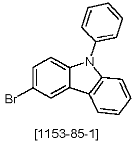 | 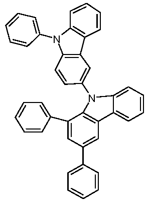 | 86% |

### Beispiel 6: 6-Bromo-1,3-diphenyl-9H-carbazole

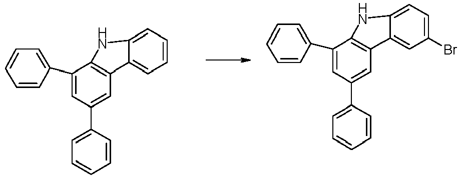

12,7 g (40 mmol) 1,3-Diphenyl-9H-carbazol werden in 450 mL Acetonitril suspendiert und bei -20 °C portionsweise mit 7,15 g (40 mmol) N-Bromsuccinimid versetzt, so dass die Reaktionstemperatur nicht über -20 °C steigt. Es wird für 18 h nachgerührt und die Reaktionsmischung dabei auf Raumtemperatur erwärmt. Die Reaktionsmischung wird anschließend einrotiert, in Dichlormethan gelöst und mit Wasser gewaschen. Es wird getrocknet, eingeengt und anschließend aus Toluol zweimal umkristallisiert. Die Ausbeute beträgt 12 g (30 mol, 76%).

Analog können folgende nicht erfindungsgemäße Verbindungen erhalten werden:

| | **Edukt 1** | **Produkt** | **Ausbeute** |
|---|---|---|---|
| 6a | 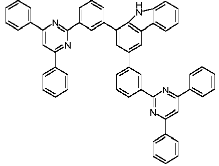 | 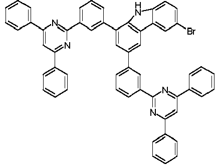 | 70% |
| 6b | 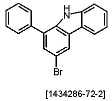 | 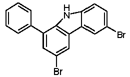 | 86% |
| 6c | 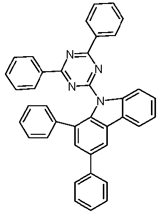 | 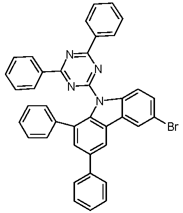 | 76% |
| 6d | 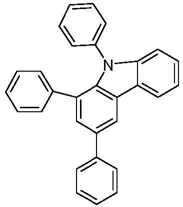 | 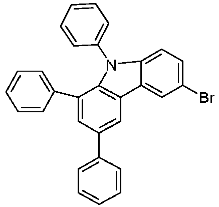 | 78% |

### Beispiel 7: 1,3-Diphenyl-6-(4,4,5,5-tetramethyl-[1,3,2]dioxaborolan-2-yl)-9H-carbazole

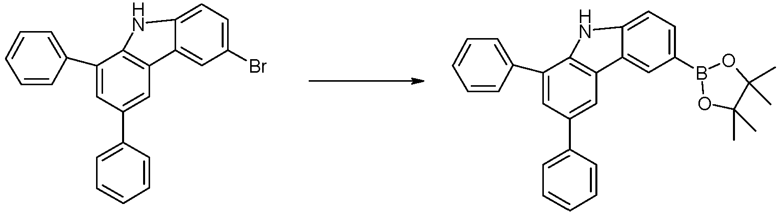

50 g (125 mmol) 6-Bromo-1,3-diphenyl-9H-carbazol, 40 g (161 mmol) Bis-(pinacolato]-diboron und 18 g (25 mmol) Palladiumdichlorid-tricyclohexylphosphin und 21g (214 mmol) Kaliumacetat werden in 1200 ml Dioxan suspendiert. Die Reaktionsmischung wird 16 h unter Rückfluss bei 130 °C erhitzt. Das Gemisch wird im Anschluss zwischen Essigester und Wasser verteilt, die organische Phase dreimal mit Wasser gewaschen und über Na₂SO₄ getrocknet und einrotiert. Der verbleibende Rückstand wird aus Heptan/Toluol umkristallisiert. Die Ausbeute beträgt 48 g (108 mmol, 86%).

Analog kann folgende nicht erfindungsgemäße Verbindung erhalten werden:

| | **Edukt 1** | **Produkt** | **Ausbeut e** |
|---|---|---|---|
| 7a | 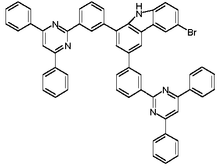 | 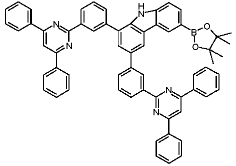 | 82% |

### Beispiel 8: 6-(4-Dibenzofuran-4-yl-phenyl)-1,3-diphenyl-9H-carbazole

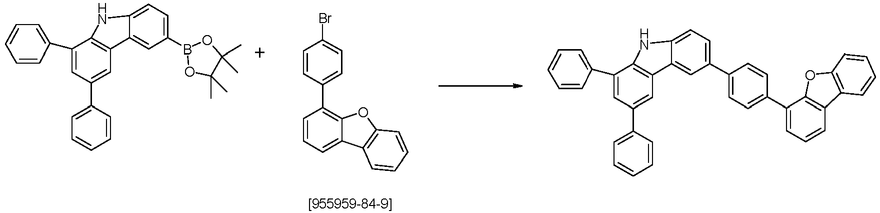

30g (68 mmol) 1,3-Diphenyl-6-(4,4,5,5-tetramethyl-[1,3,2]dioxaborolan-2-yl)-9H-carbazol, 26 g (81 mmol) 4-(4-Bromo-phenyl)-dibenzofura und 152 mg (0,67 mmol) Pd(OAc)₂ und 178 mg (0,67 mmol) P(o-Tol)₃ und 136 g (980 mmol) Kaliumcarbonat werden in 1000 ml THF und 300 ml Wasser suspendiert. Die Reaktionsmischung wird 16 h unter Rückfluss bei 130 °C erhitzt. Das Gemisch wird im Anschluss zwischen Essigester und Wasser verteilt, die organische Phase dreimal mit Wasser gewaschen und über Na₂SO₄ getrocknet und einrotiert. Der verbleibende Rückstand wird aus Heptan/Toluol umkristallisiert. Die Ausbeute beträgt 34 g (60 mmol, 90%). Analog können folgende nicht erfindungsgemäße Verbindungen erhalten werden:

| | **Edukt 1** | **Edukt 1** | **Produkt** | **Ausbeute** |
|---|---|---|---|---|
| 8a | 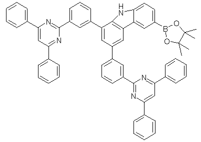 | 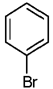 | 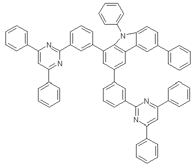 | 81% |
| 8b | 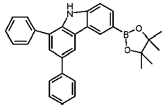 | 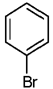 | 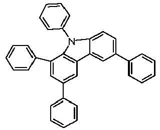 | 80% |
| 8c | 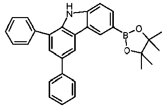 | 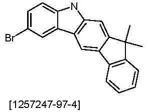 | 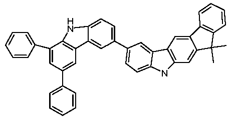 | 86% |
| 8d | 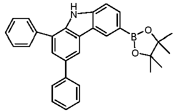 | 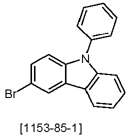 | 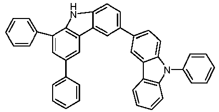 | 84% |
| 8e | 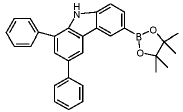 | 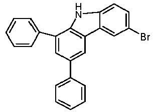 | 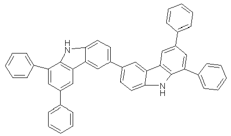 | 88% |
| 8f | 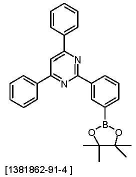 | 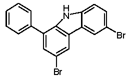 | 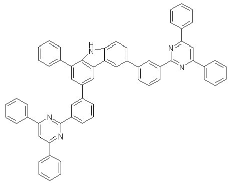 | 89% |
| 8g | 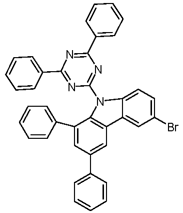 | 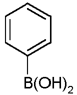 | 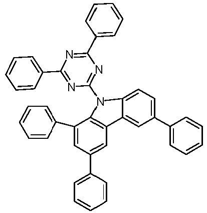 | 80% |
| 8h | 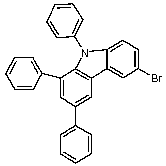 | 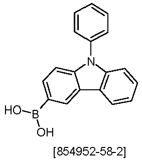 | 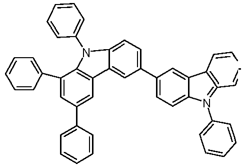 | 83% |

### Beispiel 9:

Analog zu Beispiel 5 können mit den entsprechenden Carbazol-Derivaten folgende Verbindungen hergestellt werden.

| | **Edukt 1** | **Edukt 1** | **Produkt** | **Ausbeute** |
|---|---|---|---|---|
| 9a* | 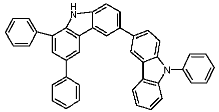 | 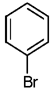 | 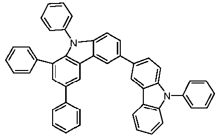 | 83% |
| 9b* | 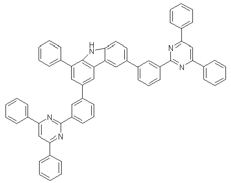 | 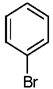 | 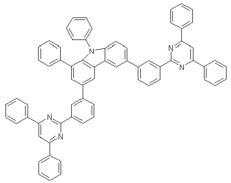 | 82% |
| 9c | 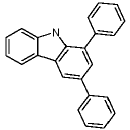 | 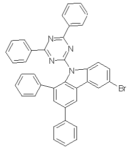 | 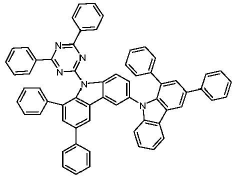 | 80% |
| 9d* | 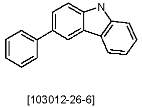 | 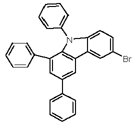 | 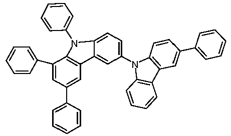 | 69% |
| 9e | 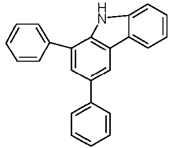 | 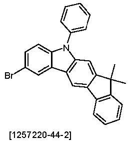 | 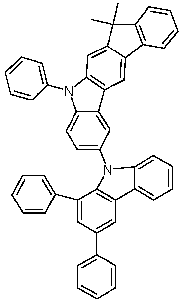 | 78% |

| | | | | |
|---|---|---|---|---|
| *nicht erfindungsgemäße Verbindungen | | | | |

Analog zu Beispiel 5 können mit 0,5 eq. des entsprechenden Carbazol-Derivats folgende nicht erfindungsgemäße Verbindungen hergestellt werden.

| | **Edukt 1** | **Edukt 1** | **Produkt** | **Ausbeute** |
|---|---|---|---|---|
| 9f | 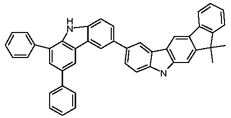 | 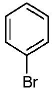 | 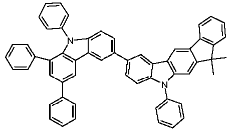 | 77% |
| 9g | 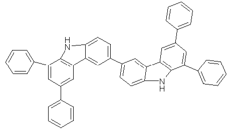 | 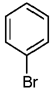 | 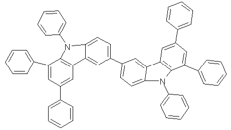 | 75% |
| 9h | 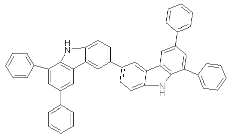 | 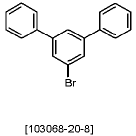 | 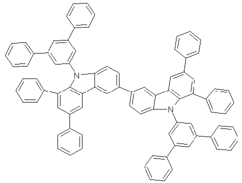 | 74% |
| 9i | 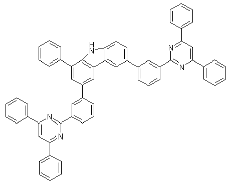 | 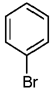 | 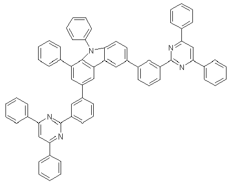 | 73% |
| 9j | 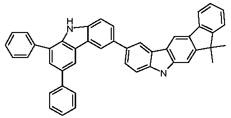 | 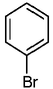 | 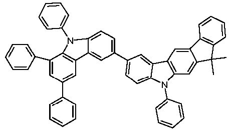 | 77% |

### Herstellung der OLEDs

In den folgenden Beispielen V1 bis E20 (siehe Tabellen 2 und 3) werden die Daten verschiedener OLEDs vorgestellt.

### Vorbehandlung für die Beispiele V1-E20:

Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen) poly(styrolsulfonat), bezogen als CLEVIOS^{™} P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 2 zu entnehmen. Die zur Herstellung der OLEDs benötigten Materialien sind in Tabelle 1 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie ST2:L1:TEG1 (55%:35%:10%) bedeutet hierbei, dass das Material ST2 in einem Volumenanteil von 55%, L1 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 3 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte auf einen gewissen Anteil L₁ absinkt. Eine Angabe von L₀;j₀ = 4000 cd/m² und L₁ = 70% in Tabelle 3 bedeutet, dass die in Spalte LD angegebene Lebensdauer der Zeit entspricht, nach der die Anfangsleuchtdichte von 4000 cd/m² auf 2800 cd/m² absinkt. Analog bedeutet L₀;j₀ = 20mA/cm², L₁= 80%, dass die Leuchtdichte bei Betrieb mit 20mA/cm² nach der Zeit LD auf 80% ihres Anfangswertes absinkt.

Die Daten der verschiedenen OLEDs sind in Tabelle 3 zusammengefasst. Die Beispiele V1 und V2 sind Vergleichsbeispiele gemäß dem Stand der Technik, die Beispiele E1-E20 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Mischungen in der Lochtransportschicht phosphoreszenter OLEDs

Die erfindungsgemäßen Materialien ergeben bei Einsatz als Elektronenblockierschicht (EBL) in phosphoreszierenden OLEDs wesentliche Verbesserungen gegenüber dem Stand der Technik in allen Parametern, vor allem bezüglich Spannung, externer Quanteneffizienz und Leistungseffizienz. Durch Einsatz der erfindungsgemäßen Verbindungen e4 und f4 lässt sich eine Verringerung der Spannung um ca. 20-40% und eine Verbesserung der externen Quanteneffizienz um ca. 25% gegenüber dem Stand der Technik SdT1 und SdT2 beobachten. Die Leistungseffizienz verbessert sich gegenüber dem Stand der Technik um ca. 40% (Beispiele V1, E1 und V2, E2).

**Tabelle 1: Strukturformeln der Materialien für die OLEDs**

| | |
|---|---|
| 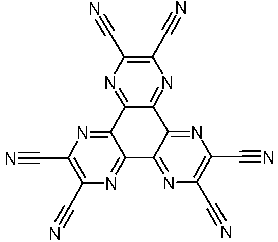 | 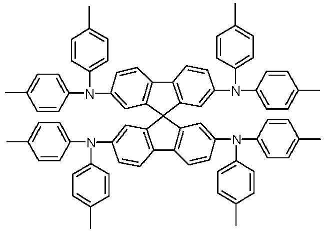 |
| HATCN | SpA1 |
| 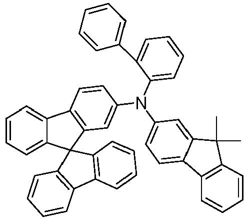 | 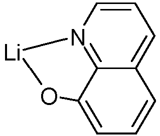 |
| SpMA1 | LiQ |
| 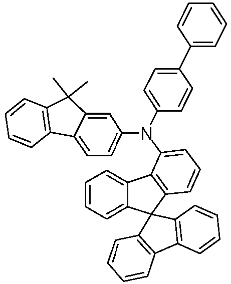 | 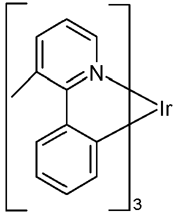 |
| SpMA2 | TEG1 |
| 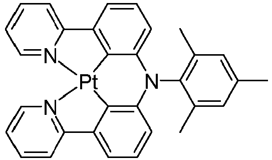 | 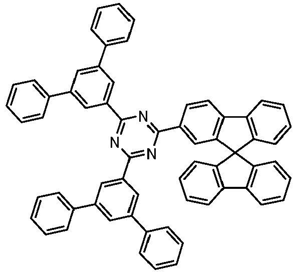 |
| TER1 | ST2 |
| 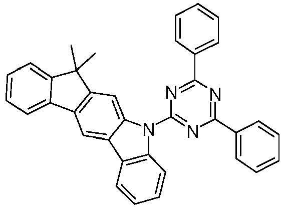 | 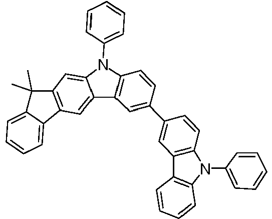 |
| IC1 | IC3 |
| 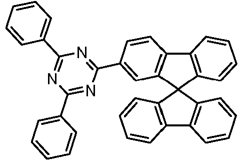 | |
| ST1 | |
| 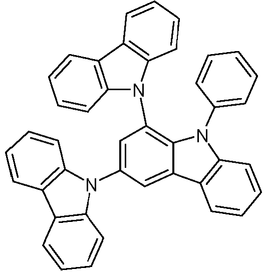 | 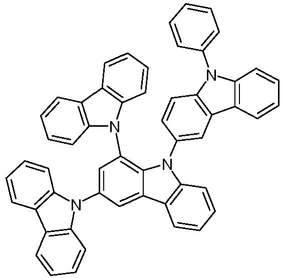 |
| SdT1 | SdT2 |
| 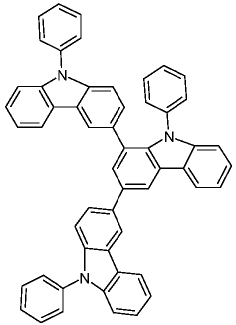 | 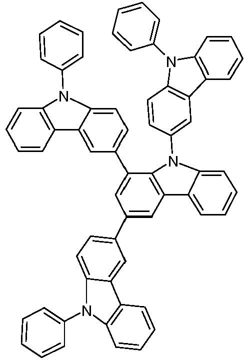 |
| e4 | f4 |
| 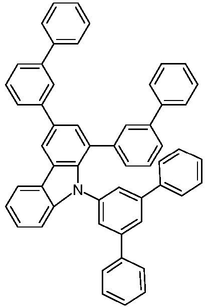 | 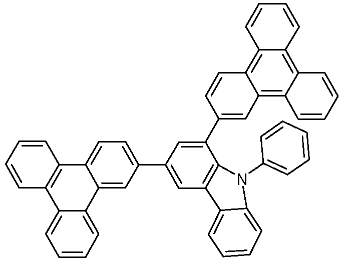 |
| e | e3 |
| 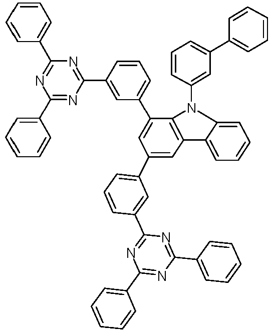 | 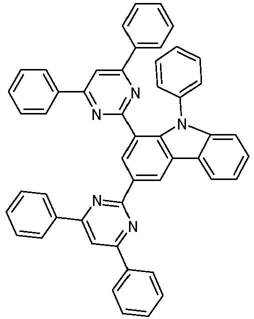 |
| e6* | e7* |
| 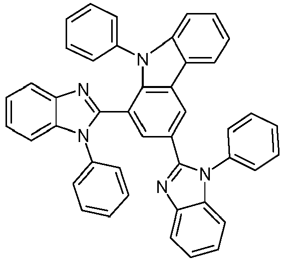 | 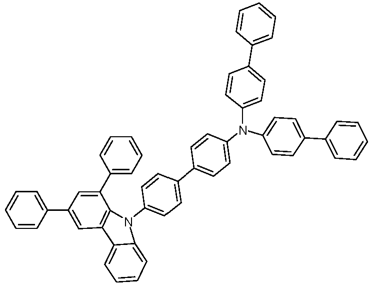 |
| e8* | f3* |
| 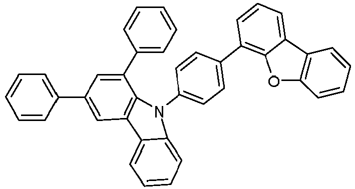 | 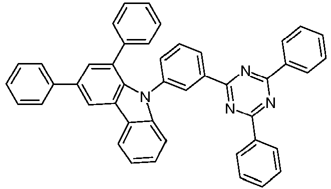 |
| f7 | f8 |
| 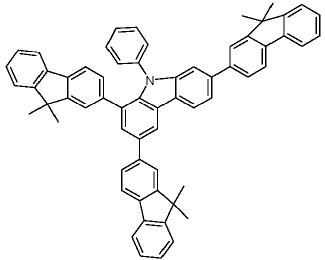 | 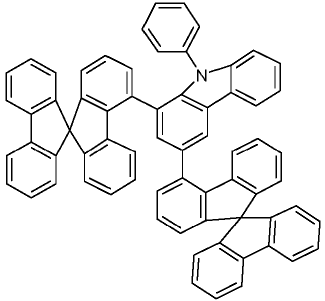 |
| f13* | f16 |
| 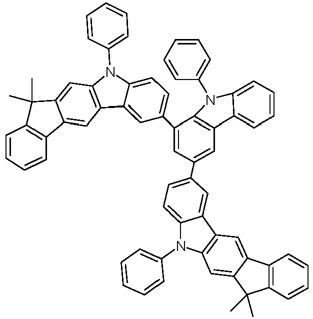 | 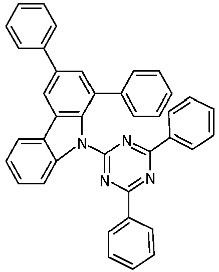 |
| f29* | g |
| 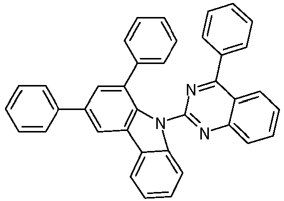 | 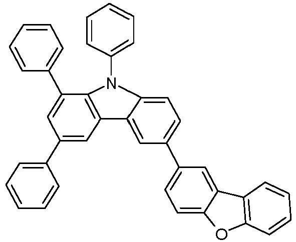 |
| q³ | i1* |
| 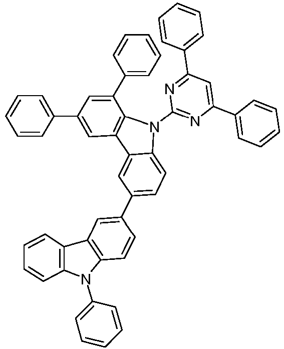 | 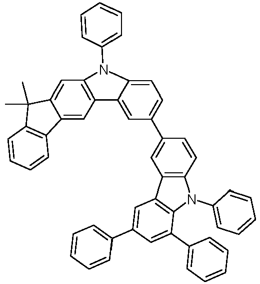 |
| i8* | i11 * |
| 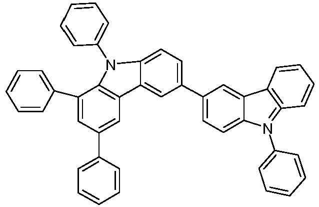 | 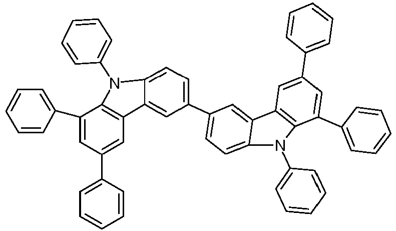 |
| k8* | 17* |

| | |
|---|---|
| *nicht erfindugnsgemäße Materialien | |

**Tabelle 2: Aufbau der OLEDs**

| Bsp. | HIL Dicke | IL Dicke | HTL Dicke | EBL Dicke | EML Dicke | HBL Dicke | ETL Dicke | EIL Dicke |
|---|---|---|---|---|---|---|---|---|
| V1 | SpA1 | HATCN | SpMA1 | SdT1 | IC1:TEG1 (90%:10%) | --- | ST2:LiQ (50%:50%) | --- |
| | 70nm | 5nm | 70nm | 20nm | | | | |
| | | | | | 30nm | | 40nm | |
| V2 | SpA1 | HATCN | SpMA1 | SdT2 | IC1:TEG1 (90%:10%) | --- | ST2:LiQ (50%:50%) | --- |
| | 70nm | 5nm | 70nm | 20nm | | | | |
| | | | | | 30nm | | 40nm | |
| E1* | SpA1 | HATCN | SpMA1 | e4 | IC1:TEG1 (90%:10%) | --- | ST2:LiQ (50%:50%) | --- |
| | 70nm | 5nm | 70nm | 20nm | | | | |
| | | | | | 30nm | | 40nm | |
| E2* | SpA1 | HATCN | SpMA1 | f4 | IC1:TEG1 (90%:10%) | --- | ST2:LiQ (50%:50%) | --- |
| | 70nm | 5nm | 70nm | 20nm | | | | |
| | | | | | 30nm | | 40nm | |
| E3 | SpA1 | HATCN | SpMA1 | --- | IC1:e:TEG1 | IC1 | ST2:LiQ | --- |
| | 70nm | 5nm | 90nm | | (65°,6:30°,6:5°,6) 30nm | 10nm | (50%:50%) 30nm | |
| E4 | SpA1 | HATCN | SpMA1 | --- | IC1 :e3:TEG1 (65% :30%:5%) | IC1 | ST2:LiQ (50%:50%) | --- |
| | 70nm | 5nm | 90nm | | | 10nm | | |
| | | | | | 30nm | | 30nm | |
| E5* | SpA1 | HATCN | SpMA1 | --- | IC1:TEG1 (90%:10%) | IC1 | e6:LiQ (50%:50%) | --- |
| | 70nm | 5nm | 90nm | | | 10nm | | |
| | | | | | 30nm | | 30nm | |
| E6* | SpA1 | HATCN | SpMA1 | --- | IC1:TEG1 (90%:10%) | --- | e7:ST2 (50%:50%) | LiQ |
| | 70nm | 5nm | 90nm | | | | | 3nm |
| | | | | | 30nm | | 40nm | |
| E7* | SpA1 | HATCN | SpMA1 | --- | IC1:TEG1 (90%:10%) | --- | e8:ST2 (50%:50%) | LiF |
| | 70nm | 5nm | 90nm | | | | | 1nm |
| | | | | | 30nm | | 40nm | |
| E8* | SpA1 | HATCN | SpMA1 | f3 | IC1:TEG1 (90%:10%) | --- | ST2:LiQ (50%:50%) | --- |
| | 70nm | 5nm | 70nm | 20nm | | | | |
| | | | | | 30nm | | 40nm | |
| E9* | SpA1 | HATCN | SpMA1 | --- | IC1:17:TEG1 (60%:30%:10%) | IC1 | ST2:LiQ (50%:50%) | --- |
| | 70nm | 5nm | 90nm | | | 10nm | | |
| | | | | | 30nm | | 30nm | |
| E10 | SpA1 | HATCN | SpMA1 | --- | f8:TEG1 (90%:10%) | IC1 | ST2:LiQ (50%:50%) | --- |
| | 70nm | 5nm | 90nm | | | 10nm | | |
| | | | | | 30nm | | 30nm | |
| E11* | SpA1 | HATCN | SpMA1 | --- | IC1:f13:TEG1 (45%:45%:10%) | IC1 | ST2:LiQ (50%:50%) | --- |
| | 70nm | 5nm | 90nm | | | 10nm | | |
| | | | | | 30nm | | 30nm | |
| E12 | SpA1 | HATCN | SpMA1 | --- | IC1:TEG1 (90%:10%) | f16 | ST2:LiQ (50%:50%) | --- |
| | 70nm | 5nm | 90nm | | | 10nm | | |
| | | | | | 30nm | | 30nm | |
| E13 | SpA1 | HATCN | SpMA1 | --- | IC1:129:TEG1 (45%:45%:10%) | IC1 | ST2:LiQ (50%:50%) | --- |
| | 70nm | 5nm | 90nm | | | 10nm | | |
| | | | | | 30nm | | 30nm | |
| E14 | SpA1 | HATCN | SpMA1 | --- | g:TEG1 (90%:10%) | IC1 | ST2:LiQ (50%:50%) | --- |
| | 70nm | 5nm | 90nm | | | 10nm | | |
| | | | | | 30nm | | 30nm | |
| E 15 | SpA1 | HATCN | SpMA1 | --- | g3:TER1 (92°,6:8°,6) 40nm | --- | ST2:LiQ | --- |
| | 90nm | 5nm | 130nm | | | | (50%:50%) | |
| | | | | | | | 40nm | |
| E16 | SpA1 | HATCN | SpMA1 | --- | IC1:i1:TEG1 | IC1 | ST2:LiQ (50%:50%) | --- |
| | 70nm | 5nm | 90nm | | (60%:30%:10%) | 10nm | | |
| | | | | | 30nm | | 30nm | |
| E17* | SpA1 | HATCN | SpMA1 | --- | i8:TEG1 (90%:10%) | IC1 | ST2:LiQ | --- |
| | 70nm | 5nm | 90nm | | | 10nm | (50%:50%) | |
| | | | | | 30nm | | 30nm | |
| E18* | SpA1 | HATCN | SpMA1 | --- | IC1:i11:TEG1 (50% :40%:10%) | IC1 | ST2:LiQ (50%:50%) | --- |
| | 70nm | 5nm | 90nm | | | 10nm | | |
| | | | | | 30nm | | 30nm | |
| E19* | SpA1 | HATCN | SpMA1 | --- | IC1:k8:TEG1 | IC1 | ST1:LiQ | --- |
| | 70nm | 5nm | 90nm | | (30%:60%:10%) 30nm | 10nm | (50%:50%) 30nm | |
| E20* | SpA1 | HATCN | SpMA1 | --- | IC1:17:TEG1 (30% :60%:10%) | IC1 | ST1:LiQ (50%:50%) | --- |
| | 70nm | 5nm | 90nm | | | 10nm | | |
| | | | | | 30nm | | 30nm | |

**Tabelle 3: Daten der OLEDs**

| Bsp. | U1000 (V) | SE1000 (cd/A) | LE1000 (Im/W) | EQE 1000 | CIE x/y bei 1000 cd/m² | L₀; j₀ | L₁ % | LD (h) |
|---|---|---|---|---|---|---|---|---|
| V1 | 4.4 | 52 | 37 | 14.4% | 0.35/0.61 | 20 mA/cm² | 80 | 100 |
| V2 | 4.1 | 54 | 41 | 14.7% | 0.34/0.63 | 20 mA/cm² | 80 | 110 |
| E1* | 3.3 | 67 | 64 | 18.0% | 0.33/0.63 | 20 mA/cm² | 80 | 140 |
| E2* | 3.2 | 70 | 69 | 18.1% | 0.34/0.63 | 20 mA/cm² | 80 | 150 |
| E3 | 3.4 | 59 | 55 | 16.6% | 0.33/0.62 | 20 mA/cm² | 80 | 140 |
| E4 | 3.5 | 64 | 57 | 17.2% | 0.32/0.64 | 20 mA/cm² | 80 | 160 |
| E5* | 4.2 | 66 | 49 | 17.6% | 0.33/0.63 | 20 mA/cm² | 80 | 340 |
| E6* | 3.3 | 69 | 66 | 18.4% | 0.33/0.62 | 20 mA/cm² | 80 | 170 |
| E7* | 3.4 | 66 | 60 | 17.8% | 0.33/0.64 | 20 mA/cm² | 80 | 140 |
| E8* | 3.1 | 70 | 71 | 18.1% | 0.34/0.63 | 20 mA/cm² | 80 | 160 |
| E9* | 3.2 | 62 | 61 | 16.9% | 0.33/0.63 | 20 mA/cm² | 80 | 180 |
| E10 | 3.1 | 67 | 68 | 18.2% | 0.34/0.63 | 20 mA/cm² | 80 | 150 |
| E11* | 3.6 | 63 | 55 | 17.1% | 0.34/0.63 | 20 mA/cm² | 80 | 160 |
| E12 | 3.4 | 63 | 58 | 17.3% | 0.32/0.63 | 20 mA/cm² | 80 | 190 |
| E13 | 3.3 | 59 | 56 | 16.4% | 0.33/0.62 | 20 mA/cm² | 80 | 200 |
| E14 | 3.2 | 61 | 60 | 16.5% | 0.32/0.64 | 20 mA/cm² | 80 | 140 |
| E15 | 4.6 | 11 | 8 | 11.4% | 0.67/0.33 | 4000 cd/m² | 80 | 310 |
| E16 | 3.3 | 62 | 59 | 16.8% | 0.34/0.62 | 20 mA/cm² | 80 | 190 |
| E17* | 3.2 | 60 | 59 | 16.2% | 0.33/0.63 | 20 mA/cm² | 80 | 155 |
| E18* | 3.2 | 62 | 61 | 16.8% | 0.35/0.62 | 20 mA/cm² | 80 | 210 |
| E19 | 3.4 | 59 | 55 | 15.9% | 0.33/0.64 | 10000 cd/m² | 70 | 220 |
| E20 | 3.5 | 60 | 54 | 16.3% | 0.32/0.63 | 10000 cd/m² | 70 | 180 |

| | | | | | | | | |
|---|---|---|---|---|---|---|---|---|
| *nicht erfindungsgemäß | | | | | | | | |

## Patentansprüche

1. Verbindung gemäß Formel (4) 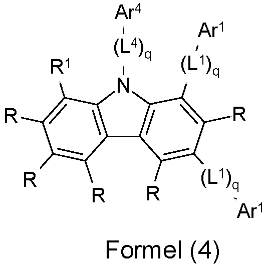 wobei für die verwendeten Symbole und Indizes gilt:.
L¹, L⁴ sind bei jedem Auftreten gleich oder verschieden ausgewählt aus den Formeln (Ar2-1) bis (Ar2-12): 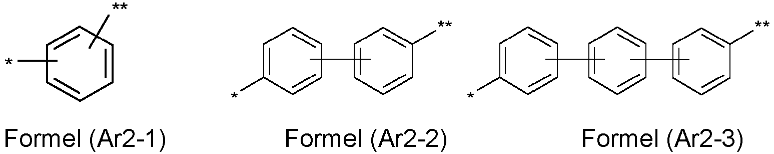 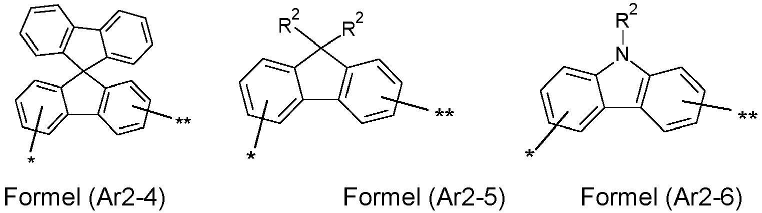 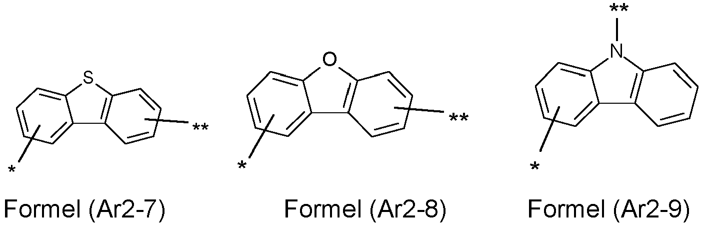 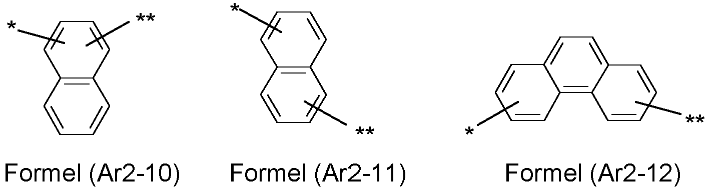
wobei die mit * gekennzeichnete Bindung, die Bindung zum Grundgerüst und die mit ** gekennzeichnete Bindung, die Bindung zum zugehörigen Rest Ar¹ oder Ar⁴ anzeigt; und wobei die Gruppen (Ar2-1) bis (Ar2-12) an den freien Positionen
mit R² substituiert sein können;
Ar¹ ist bei jedem Auftreten ein aromatisches Ringsystem mit 6 bis 60 aromatischen Ringatomen, das jeweils auch mit einem oder mehreren Reste R² substituiert sein kann;
Ar⁴ ist aus einer der Formeln (Ar-1), (Ar-5), (Ar-6), (Ar-8) oder (Ar-10) ausgewählt, 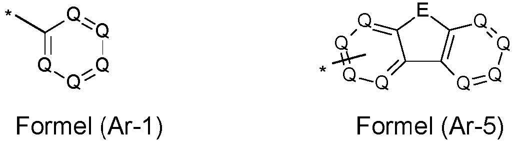 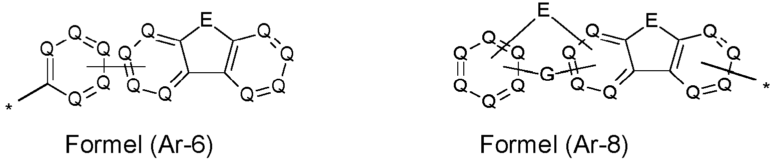 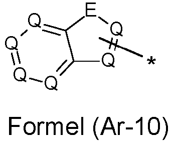 wobei:
Q ist bei jedem Auftreten gleich oder verschieden CR² oder N, wobei maximal 3 Symbole Q pro Cyclus für N stehen;
E ist bei jedem Auftreten gleich oder verschieden (CR²)₂, NR², O, S oder C=O;
G ist bei jedem Auftreten eine Einfachbindung, (CR²)₂, NR², O, S oder C=O;
* die Bindung zu dem Grundgerüst darstellt;
R ist bei jedem Auftreten gleich oder verschieden H, D, F oder ein aliphatischer Kohlenwasserstoffrest mit 1 bis 20 C-Atomen, wobei ein oder mehrere H-Atome durch D oder F ersetzt sein können;
R¹ ist H, D, F oder ein aliphatischer Kohlenwasserstoffrest mit 1 bis 20 C-Atomen, wobei ein oder mehrere H-Atome durch D oder F ersetzt sein können;
R² ist bei jedem Auftreten gleich oder verschieden H, D, F, CN, eine geradkettige Alkyl gruppe mit 1 bis 10 C-Atomen oder eine verzweigte oder cyclische Alkylgruppe mit 3 bis 10 C-Atomen, die jeweils mit einem oder mehreren Resten R³ substituiert sein kann, wobei ein oder mehrere H-Atome durch D oder F, ersetzt sein können, oder ein aromatisches Ringsystem mit 5 bis 60 aromatischen Ringatomen, das durch einen oder mehrere Substituenten R³ substituiert sein kann; dabei können zwei oder mehrere Substituenten R² zusammen mit den Atomen, an die sie gebunden sind und auch miteinander ein mono- oder polycyclisches, aliphatisches oder aromatisches Ringsystem bilden;
R³ ist bei jedem Auftreten gleich oder verschieden H, D, F, eine geradkettige Alkylgruppe mit 1 bis 40 C-Atomen oder eine verzweigte oder cyclische Alkylgruppe mit 3 bis 40 C-Atomen, wobei ein oder mehrere H-Atome durch D oder F ersetzt sein können,
q ist bei jedem Auftreten gleich oder verschieden 0 oder 1;
mit der Maßgabe, dass die folgenden Verbindungen von der Erfindung ausgenommen sind:

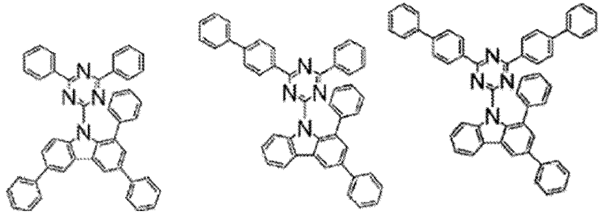

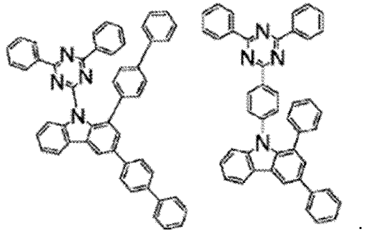

2. Mischung enthaltend mindestens eine Verbindung nach Anspruch 1 und mindestens einen fluoreszierenden oder phosphoreszierenden Dotanden.

3. Formulierung, insbesondere eine Lösung, eine Suspension oder eine Miniemulsion, enthaltend mindestens eine Verbindung nach Anspruch 1 oder eine Mischung nach Anspruch 2 und ein oder mehrere Lösemittel.

4. Verwendung einer Verbindung nach Anspruch 1 oder einer Mischung nach Anspruch 2 in einer elektronischen Vorrichtung.

5. Elektronische Vorrichtung, insbesondere ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoffsensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices", enthaltend mindestens eine Verbindung nach Anspruch 1 oder eine Mischung nach Anspruch 2.

6. Elektronische Vorrichtung nach Anspruch 5, dadurch gekenn-zeichnet, dass es sich um eine organische Elektrolumineszenzvor-richtung handelt und die Verbindung nach Anspruch 1 als Matrixmaterial für eine fluoreszierende oder phosphoreszierende Verbindung in einer emittierenden Schicht, und/oder in einer Lochtransportschicht und/oder in einer Elektronenblockierschicht eingesetzt wird.

## Claims

1. Compound of the formula (4) 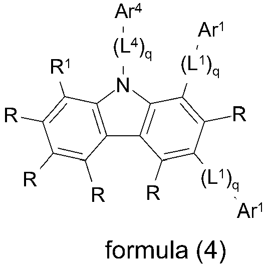 where the following applies to the symbols and indices used:
L¹, L⁴ are selected on each occurrence, identically or differently, from the formulae (Ar2-1) to (Ar2-12): 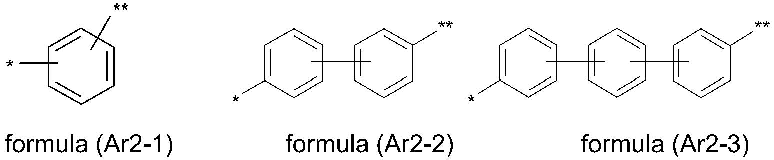 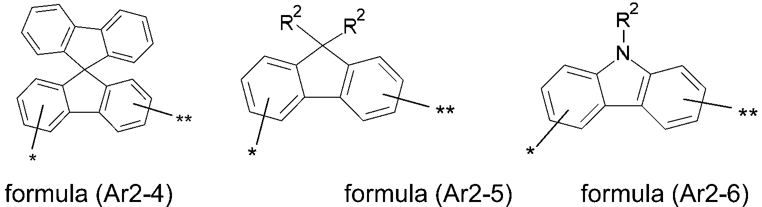 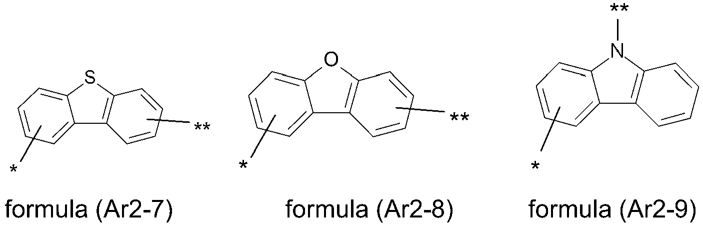 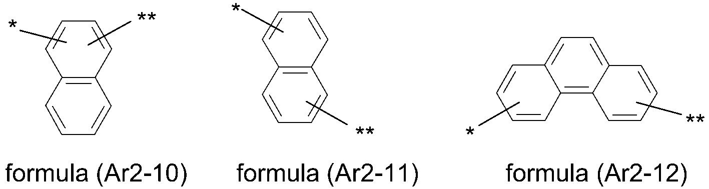
where the bond denoted by * indicates the bond to the basic structure and the bond denoted by ** indicates the bond to the associated radical Ar¹ or Ar⁴; and where the groups (Ar2-1) to (Ar2-12) may be substituted by R² at the free positions;
Ar¹ is on each occurrence an aromatic ring system having 6 to 60 aromatic ring atoms, which may in each case also be substituted by one or more radicals R²;
Ar⁴ is selected from one of the formulae (Ar-1), (Ar-5), (Ar-6), (Ar-8) or (Ar-10), 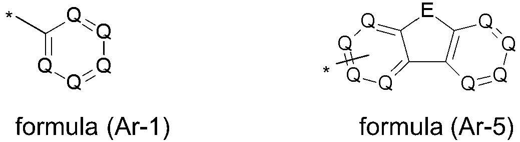 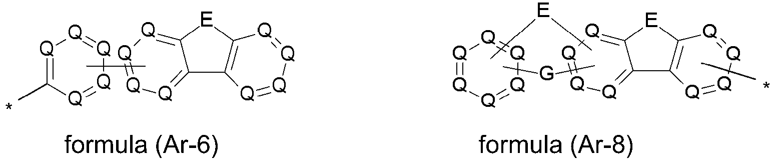 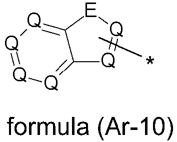 where:
Q is on each occurrence, identically or differently, CR² or N, where a maximum of 3 symbols Q per ring stand for N;
E is on each occurrence, identically or differently, (CR²)₂, NR², O, S or C=O;
G is on each occurrence a single bond, (CR²)₂, NR², O, S or C=O;
* represents the bond to the basic structure;
R is on each occurrence, identically or differently, H, D, F or an aliphatic hydrocarbon radical having 1 to 20 C atoms, where one or more H atoms may be replaced by D or F;
R¹ is H, D, F or an aliphatic hydrocarbon radical having 1 to 20 C atoms, where one or more H atoms may be replaced by D or F;
R² is on each occurrence, identically or differently, H, D, F, CN, a straight-chain alkyl group having 1 to 10 C atoms or a branched or cyclic alkyl group having 3 to 10 C atoms, which may in each case be substituted by one or more radicals R³, where one or more H atoms may be replaced by D or F, or an aromatic ring system having 5 to 60 aromatic ring atoms, which may be substituted by one or more substituents R³; two or more substituents R², together with the atoms to which they are bonded and also with one another, may form a mono- or polycyclic, aliphatic or aromatic ring system;
R³ is on each occurrence, identically or differently, H, D, F, a straight-chain alkyl group having 1 to 40 C atoms or a branched or cyclic alkyl group having 3 to 40 C atoms, where one or more H atoms may be replaced by D or F,
q is on each occurrence, identically or differently, 0 or 1;
with the proviso that the following compounds are excluded from the invention:

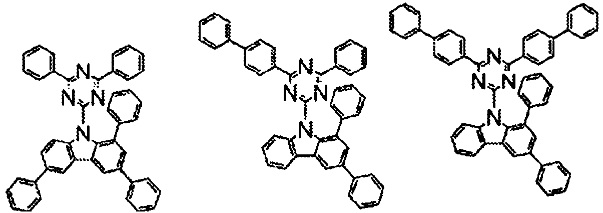

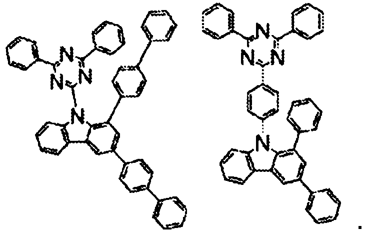

2. Mixture comprising at least one compound according to Claim 1 and at least one fluorescent or phosphorescent dopant.

3. Formulation, in particular a solution, a suspension or a miniemulsion, comprising at least one compound according to Claim 1 or a mixture according to Claim 2 and one or more solvents.

4. Use of a compound according to Claim 1 or a mixture according to Claim 2 in an electronic device.

5. Electronic device, in particular selected from the group consisting of organic electroluminescent devices, organic integrated circuits, organic field-effect transistors, organic thin-film transistors, organic light-emitting transistors, organic solar cells, organic dye-sensitised solar cells, organic optical detectors, organic photoreceptors, organic field-quench devices, light-emitting electrochemical cells, organic laser diodes and organic plasmon emitting devices, containing at least one compound according to Claim 1 or a mixture according to Claim 2.

6. Electronic device according to Claim 5, **characterised in that** it is an organic electroluminescent device and the compound according to Claim 1 is employed as matrix material for a fluorescent or phosphorescent compound in an emitting layer, and/or in a hole-transport layer and/or in an electron-blocking layer.

## Revendications

1. Composé de formule (4) 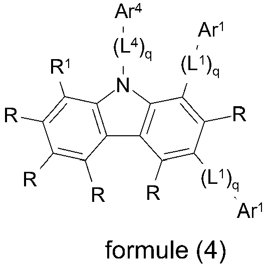 dans laquelle ce qui suit s'applique aux symboles et indices utilisés :
L¹, L⁴ sont choisis à chaque occurrence, de manière identique ou différente, parmi les formules (Ar2-1) à (Ar2-12) : 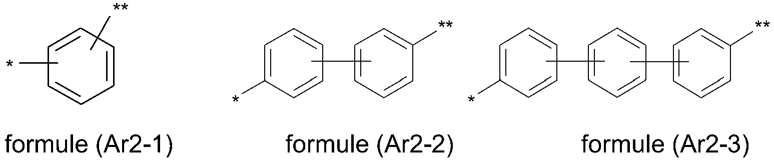 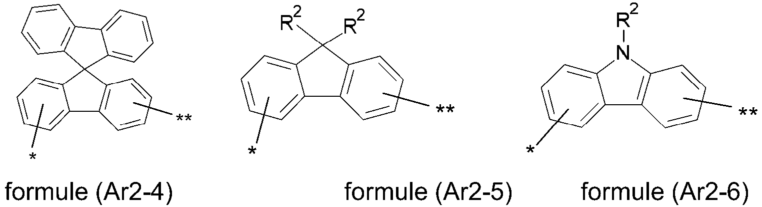 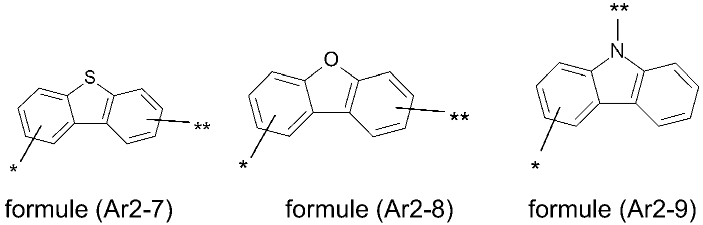 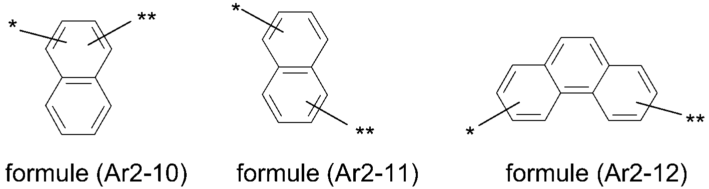
dans lesquelles la liaison désignée par * indique la liaison à la structure basique et la liaison désignée par ** indique la liaison au radical Ar¹ ou Ar⁴ associé ; et où les groupements (Ar2-1) à (Ar2-12) peuvent être substitués par R² au niveau des positions libres ;
Ar¹ est à chaque occurrence un noyau aromatique ayant de 6 à 60 atomes de cycle aromatique, qui peut dans chaque cas être également substitué par un ou plusieurs radicaux R² ;
Ar⁴ est choisi parmi l'une des formules (Ar-1), (Ar-5), (Ar-6), (Ar-8) ou (Ar-10), 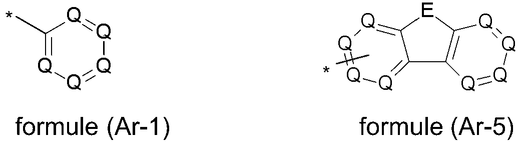 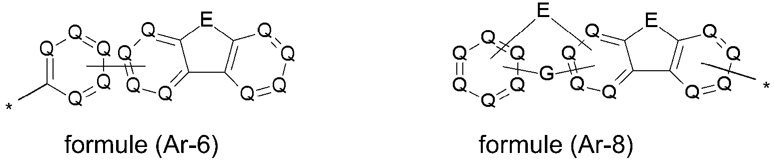 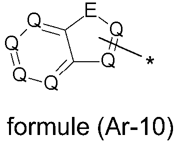 dans lesquelles :
Q est à chaque occurrence, de manière identique ou différente, CR² ou N, où un maximum de 3 symboles Q par cycle représentent N ;
E est à chaque occurrence, de manière identique ou différente, (CR²)₂, NR², O, S ou C=O ;
G est à chaque occurrence une liaison simple, (CR²)₂, NR², O, S ou C=O ;
* représente la liaison à la structure basique ;
R est à chaque occurrence, de manière identique ou différente, H, D, F ou un radical hydrocarboné aliphatique ayant de 1 à 20 atomes de C, où un ou plusieurs atomes de H peuvent être remplacés par D ou F ;
R¹ est H, D, F ou un radical hydrocarboné aliphatique ayant de 1 à 20 atomes de C, où un ou plusieurs atomes de H peuvent être remplacés par D ou F ;
R² est à chaque occurrence, de manière identique ou différente, H, D, F, CN, un groupement alkyle à chaîne linéaire ayant de 1 à 10 atomes de C ou un groupement alkyle ramifié ou cyclique ayant de 3 à 10 atomes de C, qui peut dans chaque cas être substitué par un ou plusieurs radicaux R³, où un ou plusieurs atomes de H peuvent être remplacés par D ou F, ou un noyau aromatique ayant de 5 à 60 atomes de cycle aromatique, qui peut être substitué par un ou plusieurs substituants R³ ; deux, ou plus, substituants R², conjointement avec les atomes auxquels ils sont liés et également les uns avec les autres, peuvent former un noyau aliphatique ou aromatique mono- ou polycyclique ;
R³ est à chaque occurrence, de manière identique ou différente, H, D, F, un groupement alkyle à chaîne linéaire ayant de 1 à 40 atomes de C ou un groupement alkyle ramifié ou cyclique ayant de 3 à 40 atomes de C, où un ou plusieurs atomes de H peuvent être remplacés par D ou F,
q est à chaque occurrence, de manière identique ou différente, 0 ou 1 ;
à condition que les composés suivants soient exclus de l'invention :

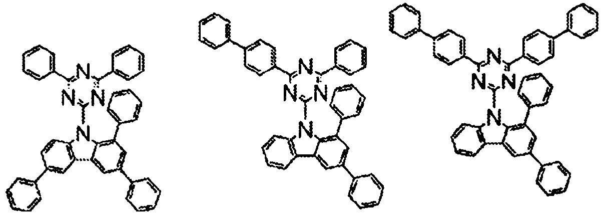

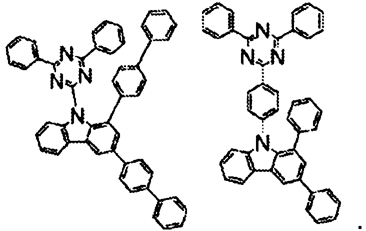

2. Mélange comprenant au moins un composé selon la revendication 1 et au moins un dopant fluorescent ou phosphorescent.

3. Formulation, en particulier une solution, une suspension ou une mini-émulsion, comprenant au moins un composé selon la revendication 1 ou un mélange selon la revendication 2 et un ou plusieurs solvants.

4. Utilisation d'un composé selon la revendication 1 ou d'un mélange selon la revendication 2, dans un dispositif électronique.

5. Dispositif électronique, choisi en particulier dans le groupe constitué par les dispositifs électroluminescents organiques, les circuits intégrés organiques, les transistors organiques à effet de champ, les transistors organiques en couche mince, les transistors organiques émetteurs de lumière, les cellules solaires organiques, les cellules solaires organiques à colorant, les détecteurs optiques organiques, les photorécepteurs organiques, les dispositifs organiques à extinction de champ, les cellules électrochimiques émettrices de lumière, les diodes laser organiques et les dispositifs organiques émetteurs de plasmons, contenant au moins un composé selon la revendication 1 ou un mélange selon la revendication 2.

6. Dispositif électronique selon la revendication 5, **caractérisé en ce qu'**il s'agit d'un dispositif électroluminescent organique et le composé selon la revendication 1 est employé comme matériau de matrice pour un composé fluorescent ou phosphorescent dans une couche émettrice, et/ou dans une couche de transport de trous et/ou dans une couche de blocage d'électrons.
